# EUROPEAN PATENT SPECIFICATION

(11) **EP 2 558 118 B1**
(45) Date of publication and mention of the grant of the patent: **05.06.2019**
(21) Application number: 11714556.5
(22) Date of filing: 14.04.2011
(51) Int. Cl.: A61K 39/12, C07K 14/08, A61P 31/14

(54) **CROSS-PROTECTING VACCINE FOR PORCINE REPRODUCTIVE AND RESPIRATORY SYNDROME VIRUS**
KREUZSCHUTZ-IMPFSTOFF FÜR DAS REPRODUKTIONS- UND ATEMWEGSSYNDROMVIRUS BEI SCHWEINEN
VACCIN À PROTECTION CROISÉE POUR LE VIRUS DU SYNDROME RESPIRATOIRE ET DE LA REPRODUCTION DU PORC

(30) Priority: 16.04.2010 EP 10160139
(43) Date of publication of application: 20.02.2013
(73) Proprietor: Universiteit Gent, 9000 Gent (BE)
(72) Inventor: DELPUTTE, Peter, 8500 Kortrijk (BE); NAUWYNCK, Hans, B-9930 Zomergem (BE); GELDHOF, Marc, B-9690 Berchem (Kluisbergen) (BE)
(74) Representative: LC Patents
(86) International application number: PCT/EP2011/055941
(87) International publication number: WO 2011/128415

(56) References cited:
- EP-A2- 0 676 467
- WO-A2-2006/074986
- FR-A1- 2 709 966
- MARTELLI P ET AL: "Efficacy of a modified live porcine reproductive and respiratory syndrome virus (PRRSV) vaccine in pigs naturally exposed to a heterologous European (Italian cluster) field strain: Clinical protection and cell-mediated immunity", VACCINE, ELSEVIER LTD, GB, vol. 27, no. 28, 8 June 2009 (2009-06-08), pages 3788-3799, XP026134056, ISSN: 0264-410X, DOI: DOI:10.1016/J.VACCINE.2009.03.028 [retrieved on 2009-04-03]
- ZUCKERMANN ET AL: "Assessment of the efficacy of commercial porcine reproductive and respiratory syndrome virus (PRRSV) vaccines based on measurement of serologic response, frequency of gamma-IFN-producing cells and virological parameters of protection upon challenge", VETERINARY MICROBIOLOGY, ELSEVIER BV, NL, vol. 123, no. 1-3, 13 June 2007 (2007-06-13), pages 69-85, XP022114779, ISSN: 0378-1135, DOI: DOI:10.1016/J.VETMIC.2007.02.009
- KIMMAN T G ET AL: "Challenges for porcine reproductive and respiratory syndrome virus (PRRSV) vaccinology", VACCINE, ELSEVIER LTD, GB, vol. 27, no. 28, 8 June 2009 (2009-06-08), pages 3704-3718, XP026134046, ISSN: 0264-410X, DOI: DOI:10.1016/J.VACCINE.2009.04.022 [retrieved on 2009-05-03]
- PLANA-DURAN ET AL: "Efficacy of an inactivated vaccine for prevention of reproductive failure induced by porcine reproductive and respiratory syndrome virus.", VETERINARY MICROBIOLOGY, vol. 55, no. 1-4, 1 April 1997 (1997-04-01), pages 361-370, XP55003577, ISSN: 0378-1135
- MISINZO GERALD ET AL: "Efficacy of an inactivated PRRSV vaccine: induction of virus-neutralizing antibodies and partial virological protection upon challenge", ADVANCES IN EXPERIMENTAL MEDICINE AND BIOLOGY, SPRINGER, US, vol. 581, 1 January 2006 (2006-01-01), pages 449-454, XP009150481, ISSN: 0065-2598
- LABARQUE G ET AL: "Impact of genetic diversity of European-type porcine reproductive and respiratory syndrome virus strains on vaccine efficacy", VACCINE, ELSEVIER LTD, GB LNKD- DOI:10.1016/J.VACCINE.2004.05.008, vol. 22, no. 31-32, 22 October 2004 (2004-10-22), pages 4183-4190, XP004594111, ISSN: 0264-410X
- MENG X J: "Heterogeneity of porcine reproductive and respiratory syndrome virus implications for current vaccine efficacy and future vaccine development", VETERINARY MICROBIOLOGY, ELSEVIER BV, NL LNKD- DOI:10.1016/S0378-1135(00)00196-6, vol. 74, no. 4, 12 June 2000 (2000-06-12), pages 309-329, XP002997174, ISSN: 0378-1135
- ROPP S L ET AL: "CHARACTERIZATION OF EMERGING EUROPEAN-LIKE PORCINE REPRODUCTIVE AND RESPIRATORY SYNDROME VIRUS ISOLATES IN THE UNITED STATES", JOURNAL OF VIROLOGY, THE AMERICAN SOCIETY FOR MICROBIOLOGY, US LNKD- DOI:10.1128/JVI.78.7.3684-3703.2004, vol. 78, no. 7, 1 April 2004 (2004-04-01), pages 3684-3703, XP009029649, ISSN: 0022-538X
- DIAZ I ET AL: "Different European-type vaccines against porcine reproductive and respiratory syndrome virus have different immunological properties and confer different protection to pigs", VIROLOGY, ACADEMIC PRESS,ORLANDO, US LNKD- DOI:10.1016/J.VIROL.2006.03.046, vol. 351, no. 2, 1 August 2006 (2006-08-01), pages 249-259, XP5581363, ISSN: 0042-6822 [retrieved on 2006-08-01]
- PAPATSIROS V G ET AL: "Long-term administration of a commercial porcine reproductive and respiratory syndrome virus (PRRSV)-inactivated vaccine in PRRSV-endemically infected sows", JOURNAL OF VETERINARY MEDICINE. SERIES B - ZENTRALBLATT FUER VETERINAERMEDIZIN. REINE B, PAUL PAREY, BERLIN, DE, vol. 53, no. 6, 1 August 2006 (2006-08-01) , pages 266-272, XP009138564, ISSN: 0931-1793, DOI: DOI:10.1111/J.1439-0450.2006.00965.X [retrieved on 2006-07-26]
- VANHEE MERIJN ET AL: "Development of an experimental inactivated PRRSV vaccine that induces virus-neutralizing antibodies", VETERINARY RESEARCH, ELSEVIER, PARIS, NL, vol. 40, no. 6, 1 November 2009 (2009-11-01), page 15PP, XP009138558, ISSN: 0928-4249, DOI: DOI:10.1051/VETRES/2009046
- PRIETO ET AL: "Similarity of European porcine reproductive and respiratory syndrome virus strains to vaccine strain is not necessarily predictive of the degree of protective immunity conferred", VETERINARY JOURNAL, BAILLIERE TINDALL, LONDON, GB, vol. 175, no. 3, 1 March 2008 (2008-03-01) , pages 356-363, XP022516400, ISSN: 1090-0233, DOI: DOI:10.1016/J.TVJL.2007.01.021

### SEQUENCE LISTING

<110> UNIVERSITEIT GENT
<120> CROSS-PROTECTING VACCINE FOR PORCINE REPRODUCTIVE AND RESPIRATORY SYNDROME VIRUS
<130> P09/036
<150> EP 10160139.1
   <151> 2010-04-16
<160> 13
<170> PatentIn version 3.5
<210> 1
   <211> 15014
   <212> DNA
   <213> Porcine reproductive and respiratory syndrome virus
<220>
   <221> misc_feature
   <222> (222)..(224)
   <223> Start codon open reading frame 1a
<220>
   <221> misc_feature
   <222> (7326)..(7328)
   <223> Stop codon open reading frame 1a
<220>
   <221> misc_feature
   <222> (7329)..(7331)
   <223> Start codon open reading frame 1b
<220>
   <221> misc_feature
   <222> (11699)..(11701)
   <223> Stop codon open reading frame 1b
<220>
   <221> misc_feature
   <222> (11712)..(11714)
   <223> Start codon open reading frame 2a
<220>
   <221> misc_feature
   <222> (11717)..(11719)
   <223> Start codon open reading frame 2b
<220>
   <221> misc_feature
   <222> (11927)..(11929)
   <223> Stop codon open reading frame 2b
<220>
   <221> misc_feature
   <222> (12320)..(12322)
   <223> Start codon open reading frame 3
<220>
   <221> misc_feature
   <222> (12459)..(12461)
   <223> Stop codon open reading frame 2a
<220>
   <221> misc_feature
   <222> (12862)..(12864)
   <223> Start codon open reading frame 4
<220>
   <221> misc_feature
   <222> (13115)..(13117)
   <223> Stop codon open reading frame 3
<220>
   <221> misc_feature
   <222> (13410)..(13412)
   <223> Start codon open reading frame 5
<220>
   <221> misc_feature
   <222> (13411)..(13413)
   <223> Stop codon open reading frame 4
<220>
   <221> misc_feature
   <222> (14003)..(14005)
   <223> Start codon open reading frame 6
<220>
   <221> misc_feature
   <222> (14013)..(14015)
   <223> Stop codon open reading frame 5
<220>
   <221> misc_feature
   <222> (14514)..(14516)
   <223> Start codon open reading frame 7
<220>
   <221> misc_feature
   <222> (14522)..(14524)
   <223> Stop codon open reading frame 6
<220>
   <221> misc_feature
   <222> (14898)..(14900)
   <223> Stop codon open reading frame 7
<400> 1
   atgatgtgta gggtattccc cctacacaca caacactgtt agtgtttgtg tgctttggag      60
   gtgtgggtac agccccgccc cacctcttgg cccctgttcc agcccaacag gtacccttct     120
   ccctcggggc gagtgcgccg cctgctgctc tctcacagca ggaaggacct cccgagtatt     180
   tccggagcgc acctgcttta caggatctcc accctttaac catgtctggg acgttctccc     240
   ggtgcatgtg caccccggct gcccgggtgt tttggaacgc cggccaagtc tactgcacac     300
   ggtgtctcag tgcgcggtcc cttctccctc ttgagcttca agacactgat cttggggcaa     360
   ttggcttgtt tcacaaacct aaagacaaac ttcgctggaa agtccctatc ggcatccccc     420
   aggtggagtg ttccccatcc gggtgctgct ggctttctgt cattttccct ttggcgcgta     480
   tgacctccgg caatcacaac ttcctccaac ggcttgtgaa agttgctgaa gtattgtacc     540
   gtgatggttg cttgaccccc cgacatcttc gtgaactcca agtttatgag cgtggctgca 600
   actggtaccc gatcacgggg ccagtgcccg gaatgggtgt gttcgcaaac tccatgcacg 660
   tatctgacca accgtttcct ggagccaccc atgtgttgac gaactcacct ctgcctcaac 720
   aggcttgccg gcagccgttc tgcccatttg aggaggctca ctctgacgta tacaaatgga 780
   aggaattcgt gatttttgtg gactctcttt ccgacggcca acctcgcatg atgtggacac 840
   cggggtctag cgattcggct gccttagaag cactgccgcc tggactagaa tgtcaagtcg 900
   aaattctcat tcggagtttc cctgcccacc accctgtcaa tctcgctgac tgggagctca 960
   ccgaatcccc tgagcacgga ttttccttta gtacgtctca gtccagtggt tatctggccc 1020
   agggccctga cgggtttgac ggcaagtgtt ggctttcctg cttcttgagc ctgcctgcca 1080
   aagtgtggcg tcatgaggag catttggcta gtgcctacgg ttatcaaacc aagtggggcg 1140
   tgcacggcaa ataccttcag cgcaggcttc aaatcaatgg tattcgtgct gtggtcgacc 1200
   ctgacggtcc cattcacgtt gaagcgctgt cttgccccca gtcttggatt aggcacctga 1260
   ccatggagga tgatgtgacc ccagggttcg tccgtttggc atctctccgc attgtgccga 1320
   acacagaacc caccgccctc ccgatctttc ggtttggagc gcataagtgg tatggtgctg 1380
   ccggcaaacg agctcgcgcc aagcgtgccg ccaaaagtga gaaggatccg gcccttaccc 1440
   ccaaggttgc ccagtcggcc cccactaacg gaaccaccat ctactctcca ccggcggacg 1500
   ggtcctgtgg ttggcatgtc ctcgccgcca taatgaaccg gatggtgaat aatgacttca 1560
   catcccctct ggcccagtac aacagaccgg aggatgattg ggcttctgat tatgatcttg 1620
   ctcaggcgat tcaatgccta caactgcccg ctgccatagt tcggaatcgt gcctgcccca 1680
   acgccaaata cctcgtgaaa cttaacgggg ttcattggga ggtagaggtg agatctggaa 1740
   tggccccccg ctccctttct cgtgagtgtg tggttggcgt ctgctccgaa ggctgcattg 1800
   cactacctta cccaaaagac gggctaccta aacgtgcgcc cgaggccttg gcgtctgctt 1860
   acagactacc ctccgactgt gtcagtgctg gtatcgctga ttttcttgct aatccaaccc 1920
   ctcaagaatt ttggaccctc gacaaaatgt tgacctcccc gtcaccagag cggtccggct 1980
   tctccagctt gtataaattg ctattggagg tagttccgca aaaatgtggt gccacagaag 2040
   gagctttcgt ctatgctgtt gagaggatgt tgaaggattg tccaagttcc aaacaggcca 2100
   tggccctcct gtcaaaaatt aaagttccat cttcaaaggc cccgtctgtg tctttggacg 2160
   agtgtttccc tacggatgtt tcggccgact tcgagccaac atttcatgaa aggtcccaag 2220
   gttccggcac tgcaggtgtc tcgtgttcac cggatgcaaa aaggttcgag aaggcgaccc 2280
   cagaagaagt ccaggaggat ggccacgagg ctgtccgctc tacgcccttc accgacgacc 2340
   ctactctggc ttcggctggt tcaaccaacc cagtggatgg gaatctgtcc tcttcagact 2400
   cagcggaaga aagcacgctc aacagccggg aagacgaacc actggaccta tctcaacctg 2460
   caccggttgt aacaacaggc ctcgtaaggg aacaaacacc ttgcgaccta ggtttcgatg 2520
   ccggtgcccc cttcgtcact gtccgaaaat ttgtcccaat agggtctacg tttcgccacg 2580
   ttgagcactg tggcacagaa tccgacaaca tcagctcgcc tctgactttg cctgatgcgc 2640
   aggccttaga ccagcccttg gatctgtctc tggccgcctg gccggtaagg gcaaccgcgt 2700
   ctgaccccgg ctgggttcac ggcaggcgtg aacctgtttt cgtaaagccc aggggcactc 2760
   tttccgatgg cgactcagtc ctccagtttg gggagctttc tgaggccagt tccgtcgttg 2820
   agtttgaccg gttaagagag attccggcgg tcgacgcccc tgtcgatttg acaccttcga 2880
   atgaggccct ctctgcagat gaccctttca gatctgccga gttcaagcgc ccacgtttct 2940
   ctgcacaggc cctaattgac cgaggcggtc ctctcgccga tgtccatgca aaaataaaga 3000
   gccgggtata cgaacagtgc cttcaggctt gtgaacccgg tagtcgtgcg accccagcca 3060
   ccaaggcgtg gctcgacaaa atgtgggata gggtggacat gaaaacttgg cgctgcacgt 3120
   cgcagttcca agctggtcgc attcttgcgt cccttaaatt tctccctgat atgattcagg 3180
   atacaccgcc tcctgttccc aggaagagcc gaaccagcgg tagtgccggt ctgaagcaac 3240
   tggtagcaca gtgggatagg aagttgagtg cgacctccca aaaaaaaccg gttgagccaa 3300
   tgcgcaacca ggccacccct ccacctacgg atatccagca agaagaagtc accccccccg 3360
   atgggtcacc ccatgcgcca gacttcccta gtcgagtgag catgagctgg aattggaaag 3420
   gcttcatgct ttccggcacc cgtttcgcgg ggtccattag ccagcgcctc atggcatggg 3480
   tttttgaaat tgtctcccat ctcccagctt ttatgctcac acttttctcg ccgcggggct 3540
   ctatggctcc aggtgattgg ttgtttgcag gtgttgtttt acttgctctc ttgctctgtc 3600
   gttcttaccc aatactcggg tgtctaccct tattgggtgt cttttctgga tctttgcggc 3660
   gtgttcgtct gggtgttttt ggttcttgga tggcttttgc tgtattttta ttctcgactc 3720
   catccaaccc agtcggttct tcttgtgacc acgattcgcc ggagtgtcat gctgagcttc 3780
   tggctcttga gcagcgccaa ctttgggaac ctgtgcgcgg ccttgttgtt ggcccctcag 3840
   gtctcttatg tgtcattctt ggcaagttac tcggtgggtc acgttatttc tggcatgttt 3900
   tcctacgttt atgcatgctt gcagatttgg ccctttctct tgtttatgtg gtgtcccagg 3960
   ggcgttgtca caagtgttgg ggaaagtgta taaggacggc tcctgcggag gtggctctca 4020
   atgtgttccc tttctcgcgt gctacccgca actctcttgt gtctttgtgc gatcgatttc 4080
   aagcgcctaa aggggttgac cctgtgcatt tggcaacagg ttggcgcggg tgttggcacg 4140
   gcgagagccc cattcatcaa cagcaccaaa agcctatagc ctatgccaat ttggatgaaa 4200
   agaagatatc tgcccaaacg gtggtcgctg tcccatatga tcccagccaa gctatcaaat 4260
   gcctgaaagt tttgcaagcg ggaggagcta tcgtggacca gcccatacct gaggtcgttc 4320
   gtgtgtccga gatccctttc tcagccccat tctttccgaa ggtaccagtc aacccggatt 4380
   gtaggattgt agtggactcg gacacttttg tggctgcggt tcgctgcggt tactcaacga 4440
   cacaactagt cctaggccga ggcaacttcg ccaagttaaa tcagaccccc cttaggggct 4500
   ccgtctccac caaaacgatt ggtggggcct cctacaccat ggctgttgcc caagtgtctg 4560
   tgtggactct tgttcatttc gtcctcggtc tttggctcac gtcacctcag gtgtgtggtc 4620
   gaggaacctc tgacccatgg tgttcgaatc ctttctcata tcctacttat ggccccgggg 4680
   ttgtgtgctc ctctcgactt tgcgtgtctg ccgacggagt caccctacca ttgttttcag 4740
   cagtggcaca actctccggt agagaagtgg gggtttttat cttggtactc gtttccttgt 4800
   gtgctttggc ccaccgcatg gctcttaagg cagacatgct gatggtcttc ttggcttttt 4860
   gtgcttacgc ctggcccatg agctcctggt taatttgctt ctttcctata ctcttgaagt 4920
   gggtcaccct tcaccccctc accttgtttt gggtacactc attcttggtg ttctgtttgc 4980
   cagcagctgg tgtcctctca ctaggaataa ccggccttct ctgggcagtt ggccgcttca 5040
   cccagattgc aggaattatc accccttatg acatccacca gtacacctcc gggccgcgcg 5100
   gtgcagctgc cgtagcaaca gccccagaag gcacttacat ggctgctgtt cggagggctg 5160
   ctttaaccgg gcgaactctg attttcacac catctgcagt cggatccctt cttgaaggcg 5220
   cctttagaac ccataaaccc tgccttaaca ccgtaaatgt tgtgggctct tcccttggtt 5280
   ccgggggggt tttcaccatt gacggcagaa aaactatcgt caccgccacc catgtgttga 5340
   acggcgacac agctagggtc acaggcgact cctataatcg tatgcacact tttaagacaa 5400
   atggtgatta tgcctggtcc cacgctgatg attggcgagg cgttgccccg gcggtcaatg 5460
   tcgcaaaggg atactgcggt cgtgcttatt ggcaaacatc aactggcgtc gaacccggta 5520
   tcattgggaa tggatttgcc ttctgtttta ctaactgtgg cgattcgggg tcacccgtta 5580
   tctcagaaac tggtgatctc attggaatcc acaccggttc aaacaaactt ggctctggtc 5640
   ttgtgacaac tcctgcaggg gagacctgcg ccatcaaaga aaccaagctc tctgaccttt 5700
   ccaagcactt cgcaggtccg agcgtccctc ttggggacat taagttgagc ccggctatca 5760
   ttcctgatgt aacatccgtt ccgagtgact tggcatcgct cctagcctct gttcctgtga 5820
   tggaaggtgg cctctcgacc gtccaacttt tgtgtgtctt tttccttctc tggcgcatga 5880
   tgggccatgc ctggacaccc attgttgccg tgggcttctt tttgctgaat gaaatccttc 5940
   cagctgtttt agtccgagcc gtgttttctt tcgcactctt tgtgcttgca tgggttaccc 6000
   cctggtctgc acaggtgttg atgattagac tcctcacggc atctctcaac cgcaacaagc 6060
   tttctctggc gttttacgcg cttggtggtg tcgccggctt ggctgccgaa atcgggactt 6120
   ttgctggcag attctctgaa ctgtctcaag ctctttcgac atactgcttc ttacccagag 6180
   tccttgctgt gaccagttgt gtccctatca tcatcattgg tggacttcac gccctcggcg 6240
   taattttgtg gttattcaaa taccggtgcc tccacaatat gctggttggt gacgggagtt 6300
   tttcgagcgc cttcttcctg cggtattttg cagagggtaa tctcagaaaa ggtgtttcgc 6360
   agtcctgtgg catgaataac gagtccctaa cggctgcttt agcttgcaaa ttgtcacagg 6420
   ctgaccttga ttttctgtcc agcttaacga acttcaagtg ctttgtgtcc gcctcaaaca 6480
   tgaaaaatgc agctggccag tatattgaag cagcgtacgc caaggccttg cgccaagagc 6540
   tggcctcttt agttcagatt gacaaaatga agggagtctt gtctaaactc gaggcctttg 6600
   ctgaaacagc caccccgtcc cttgacacag gtgacgtgat tgtcctgctt gggcaacatc 6660
   ctcacggatc cattctcgac atcaacgtgg ggactgaaag gaaaactgtg tctgtgcaag 6720
   agacccggag cctaggcggc accaaattca gtgtttgcac tgtcgtgtcc aacacacccg 6780
   tggacgcatt aaccagcatc cctctccaga caccaactcc tctttttgag aatggtccgc 6840
   gtcatcgtag tgaggaggac gatcttaaag tcgagaggat gaaaaaacac tgtgtatccc 6900
   tcggctacca caacataaat ggcaaagttt actgcaaaat ttgggataag tctaccggtg 6960
   acacctttta tacggacgat tctcggtaca cccaagatta tgctttccag gacaggtcag 7020
   ccgactacag agatgggggc tatgagggtg tgcaaaccgc ccctcaacat gggttcgacc 7080
   caaagtctga gacccctatt ggcactgttg tgatcggcgg tattacttat aacaggtacc 7140
   tgattaaagg taaggaggtt ctggtcccca agcctgacaa ctgccttgaa gccgccaagc 7200
   tgtcccttga acaagctctc gctgggatgg gccaaacttg tgacctcaca gccgccgagg 7260
   tggaaaagtt aaaacgcatc attagtcaac ttcaaggttt gactactgag caggctttaa 7320
   actgttagcc gccagcggtt tgacccgctg tggccgcggc ggcctagttg tgactgacac 7380
   ggcggtaaaa atcataaaat accacagcag aaccttcacc ttgggcccta tagacctaaa 7440
   agtcacttca gaggtagagg tgaagaaatc aactgagcaa ggtcacgctg ttgtggcaaa 7500
   cttatgttcc ggtgtcgtct taatgagacc tcacccgcca tcccttgttg acgttcttct 7560
   gaaacccgga cttgacatag cacccggcat acaacccgga catggagccg gaaatatggg 7620
   tgtggacggt tccgtttggg attttgaaac cgcacccaca aaagcagagc ttgagctgtc 7680
   caagcaaata attcaggcat gtgaggtcag gcgcggagac gccccgaacc tccaactccc 7740
   ctacaagctt taccctgtta gaggggaccc tgagcggcat gaaggttacc tcatcaacac 7800
   caggtttgga gatttgcctt acaagactcc tcaagacacc aagtctgcta tccatgcggc 7860
   ttgttgtcta caccccaatg gagtccttgt gtccgatggc aagtccgcac taggcactac 7920
   tcttcaacat gggttcgagc tttatgttcc cacagtgccc tacagcgtca tggagtacct 7980
   tgattcacgt cctgacactc ctcccatgtt cactaaacat ggcacttcta aggctgctgc 8040
   agaagacctc caaaaatatg acctatccac tcaaggattc gtcttgcctg gggttctacg 8100
   cctaatgcgc agatttatct ttggccatat tggtaaggca ccgccattat ttcttccatc 8160
   aacatatccc gccaagaact ccatggcagg tattaatggc cagagattcc caacaaaaga 8220
   cgtccaaagc atacctgaaa ttgatgaagt gtgcgcccgc gccgtcaagg agaactggca 8280
   aactgtgaca ccttgtactc tcaaaaaaca gtactgttcc aagcccaaga ccaggactat 8340
   cctgggcacc aacaacttca tcgccttggc tcacagatcg gcgctcagtg gtgtcaccca 8400
   ggcattcatg aaaaaggcct ggaagtcccc aattgcctta gggaaaaaca aattcaagga 8460
   gttgcattgc acagtcgccg gcaggtgtct tgaggccgat ttagcttcct gtgaccgcag 8520
   cacccccgcc attgtaagat ggtttgttgc caacctcctg tatgaacttg caggatgtga 8580
   ggagtacttg cccagctacg tgctcaactg ttgccacgac ctagtggcaa cgcaagatgg 8640
   cgcctttaca aaacgtggtg gcctatcatc tggggacccc gtcaccagtg tgtctaacac 8700
   cgtatattca ctgataattt atgctcagca tatggtgctg tctgctctaa aaatgggtca 8760
   tgaaatcggc cttaagttcc tcgaggaaca gctcaagttt gaggacctcc ttgaaatcca 8820
   gcccatgctg gtatactctg atgatcttgt gctgtatgct gaaaaaccca ctttccccaa 8880
   ttaccattgg tgggttgagc accttgacct aatgttgggt tttaaaacgg accccaaaaa 8940
   gactgtcatc actgacaaac ccagctttct tggctgtaaa atcgaagccg ggcgacagtt 9000
   ggtccccaat cgcgaccgca ttctggccgc tcttgcatac cacatgaaag cgcagaacgc 9060
   ttcagagtac tacgcatctg ctgccgcgat cctgatggat tcgtgtgctt gtatcgacca 9120
   tgaccctgag tggtatgaag acctcatttg cggtattgcc cggtgcgccc gtcaggacgg 9180
   ctacagtttt ccaggcccac cgtttttctt gtccatgtgg gagaaactga aaagccacaa 9240
   tgaaggcaag aaattccgtc actgcggcat ctgcgacgct aaggccgacc atgcgtccgc 9300
   ctgtgggctt gacttgtgtt tgtttcactc acactttcat caacactgcc ctgtcactct 9360
   tagctgcggc caccacgccg gctcaagaga atgcccacag tgtcagtcac ccgttggggc 9420
   tggtaagtcc ccccttgacg ctgtgttgaa acagattccg tacaaacccc cccgtactgt 9480
   cattatgaag gtggataata aaacaacaac ccttgacccg ggaaggtatc aatcccgtcg 9540
   aggacttgtt gcagtcaaga ggggcattgc aggcaatgag gttgatctca ctgatggaga 9600
   ctaccaggtg gtgcccttac tgccgacctg caaggacata aatatggtaa aggtggctgt 9660
   taatgtgcta ctcagtaagt tcatagttgg gccgccaggt tctggaaaga ccacctggct 9720
   actgagccaa gtccaggatg atgatgttat ctatacaccc acccatcaga ccatgtttga 9780
   catagtcaac gctcttaaag tttgcaggta ttccatcccg ggggcttcag ggctcccttt 9840
   cccaccacct gctagatccg gaccgtgggt cagacttgtt gccagcgggc acatccctgg 9900
   ccgaatatca tacctcgacg aggctggata ttgcaaccac ctggatattc tcagattgct 9960
   ttctaaaaca cctctggtgt gtttgggtga ccttcagcag cttcaccctg ttggctttga 10020
   ctcttactgt tatgtgtttg atcaaatgcc tcagaaacag ctgactacca tctacagatt 10080
   tggccctaac atctgtgctg ccatccaatc ttgttacagg gagaaacttg aatctaaggc 10140
   caggaacacc agggtggtct ttaccactcg gcctgtagat tttggtcagg tgctgacgcc 10200
   gtaccacaaa gatcgcatcg gctcagctat aaccatagac tcatcccagg gggccacctt 10260
   tgatgttgtg acgttgcact taccatcgcc aatgtcccta aataaatccc gtgcactggt 10320
   agccatcact cgggcgagat atgggttatt catttatgac cctcacgacc aacttcggga 10380
   gtttttcaac ttgacccctg agcgtactga ttgcaatctc gtgttcaacc gaggggaaga 10440
   gttggtagtt ttgaacgctg acaatgcagt cacgactgtg gcgaaggccc ttgaggcagg 10500
   tccctctaga tttcaagtgt cagacccgag gtgcaagtct cttctggccg cttgttcggt 10560
   cagttttgaa gggtgttgta tgccactacc gcaagtggca cataacttgg ggttttactt 10620
   ttccccagac agcccagtat ttgcaccttt gccgaaggag ttggcaccac attggccggt 10680
   ggttacttac cagaacaaca aggcatggcc tgatcggctc gtcgctagca tgcggccaat 10740
   tgatgcccac tacagcaagc ctatggttgg cgcagggtat gtggtcgggc cgtccacctt 10800
   tcttggtgcc cctggtgtgg tgtcatacta cctcacactg tacatcaagg gcgagcctca 10860
   ggccttgccg gagacactcg tctcaacagg acgtatagcc acagattgtc gagagtacct 10920
   cgacgcagct gaggaagaag cagccaaaga actcccccat gcattcattg gtgatgtcaa 10980
   aggcaccaca gttgggggat gtcatcacat cacatcaaaa tacttaccca ggttcctgcc 11040
   caaggactct gttgccgtgg ttggagtgag ttctcccggc aaggctgcta aggctgtgtg 11100
   tactcttact gacgtgtacc tccctgagct ccggccgtac ttgcaaccag agacagcatc 11160
   gaagtgctgg aaactcaaac tagatttcag ggatgtcaga ctgatggtct ggaaagggtc 11220
   caccgcctac ttccagcttg aaggacttac atggtcggcg ctgcccgact atgccaggtt 11280
   catccaggta cccaaagacg ccgtcgtgta tatcgaccca tgcataggac cagcaacagc 11340
   caaccgtaag gttgtgcgaa ccacggactg gcgtgccgac ctggcggtga caccgtatga 11400
   ttacggcgct ccgaatattt tgacaacagc ttggttcgag gacctcgggc cgcaatggaa 11460
   gattttgggg ttgcagccct ttaagcgggc atttggtttc gaaaccactg aggattgggc 11520
   gatccttgcg cgccgcatga acgacggcaa ggactacact gactataact ggaattgtgt 11580
   tcgacaacgc ccacacgctg tccacgggcg cgcccgtgac catacgtacc actttgcccc 11640
   tggcatagaa ctgcaagtgg agctgggaaa accccggctg caacctgagc aagtaccgtg 11700
   aattcagagc gatgcaatgg ggtcactgtg gagcaaaatc acccagttat ttgtggacgc 11760
   cttcactgag ttccttgtta gtgtggttga cattgtcatc ttccttgcca tactgtttgg 11820
   gttcaccgtc gcaggatggt tactggtctt tcttctcaga gtggtttgct ccgcgcttct 11880
   ccgttcgcgc tctgccattc actcttccga actatcgaag gtcttatgag ggcttacttc 11940
   ccaattgcag accggatgtt ccacaatttg catttaagca cccattgggt atgttttggc 12000
   acatgcgggt ttcccatctg attgatgaaa tggtctctcg ccgcatctac cagaccatgg 12060
   aacattcagg tcaagcggct tggaagcagg tggttagtga ggccactctc acaaaactgt 12120
   caaatctcga catagttctt cacttccaac acctggccgc ggtggaggcg gattcttgcc 12180
   gcttcctcag ctcacgactt gtgatgctga aaatcttgcc cgttggcaat gtgagcctgc 12240
   actacaacac cacgttggac agcgttgagc tcatcttccc cacaccaggt acgagaccta 12300
   agttgaccga tttcaggcaa tggcttgtca gtgtacacgc ttctattttt tcctctgtgg 12360
   cctcatctgt taccttattc atagtgcttt ggcttcgaat tccagctcta cgctatgttt 12420
   ttggtttcca ttggcccacg gcaacacgtc attcgaattg accatcaact acaccatatg 12480
   caagccctgc cttactagtc aggcggcttc ccagaggctc gaacccggtc gcaacgtgtg 12540
   gtgtaaaata gggcatgata aatgtgagga gcgcgaccat gatgagttat taatgtccat 12600
   tccgtctggg tacgacaacc tcaaactcga gggttattat gcttggttgg cgtttttgtc 12660
   cttttcctac gcggcccaat tccatccaga gttgtttggg atagggaatg tgtcgcgtgt 12720
   ctttgtggac aagcgacacc agttcatttg tgccgcacat gatggacaca attcaaccgt 12780
   gtctaccagc cacaacatct ccgcatcata tgcagtgtat taccaccacc aaatagacgg 12840
   gggcaattgg tttcatttgg aatggctgcg gccattcttt tcctcatggt tggtgctcaa 12900
   catatcatgg tttctgaggc gttcgcctgc aagccctgtt tctcgacgca tttatcagat 12960
   attaagacca acacgaccgc ggctgccggt ttcatgggcc ttcagaacat cgattgcctc 13020
   cgacatcaca gggtcacggc agcgcagggg cgaatttaca ccagagggcc gtcccaatgc 13080
   cgtgaagtcg tcggtactcc gcagtacatc acggtaacgg ccaatgtgac cgacgagtca 13140
   tacttgtaca acgcggacct gctgatgctt tctgcgtgcc ttttctatgc ctcggaaatg 13200
   agcgagaagg gcttcaaagt catctttggg aatgtctctg gcgttgtttc cgcttgcgtc 13260
   aatttcactg attatgtggc tcatgtgact caacacaccc agcagcatca tctggtgatc 13320
   gatcatgtcc ggttgctgca tttcttgaca ccgtctgcaa tgaggtgggc cacaaccatt 13380
   gcttgtttgt tcgccatcct cttggcggta tgagatgttc tcacaaattg gggcgcttct 13440
   tgactccaca ctcttgcttc tggtggcttt ttttgctgtg taccggcttg tcttggtcct 13500
   ttgccgatgg caacggcaac agctcgacat accaatacat atataatttg acgatatgcg 13560
   agctgaatgg gaccacttgg ttgtccgatc acttttattg ggcagtcgag acttttgtgc 13620
   tttacccagt ggcaactcac attctctcat tgggttttct tacaacaagc catctccttg 13680
   acgcgctcgg tctcggagcc gtgtccgttg ccggttttca tggcggacgg tacgtactca 13740
   gcagcgtgta cagcgcttgt gcattagcag cgcttgtgtg ttttgttatc cgcgctgcca 13800
   aaaattgcat ggcttgccgc tatgctcgta cccgatttac caacttcatt gtggacgacc 13860
   gggggagaat ccatcgatgg aggtctccaa tagtggtgga gagattgggc aaagctgacg 13920
   tcagcggcga ccttgtcacc atcaaacatg ttgtcctcga gggagttaaa gctcaaccct 13980
   tgacaaggac ttcggccgag caatgggaag cctagatgac ttttgtggcg atcccaccgc 14040
   cgcacaaaag cttgtgctag cctttagcat cacatataca cctattatga tatacgccct 14100
   taaggtgtca cgcggccggc tcctggggct gttgcacatc ttgatatttc tgaactgttc 14160
   cttcacgttc ggatacatga catatgtaca ttttgagtcc accaaccgtg tcgcatttac 14220
   tatgggggcc gtcgttgccc ttttgtgggg cgtctatagc ttcatagagt catggaagtt 14280
   tatcacttcc agatgcagat tgtgttgcct aggccggcga tacattctgg cccctgccca 14340
   ccacgtagaa agtgctgcag gcctccatcc gattccagcg tctggtaacc atgcatatgc 14400
   tgtgagaaag cccggactaa catcagtgaa cggcactcta gtaccaggac ttcggggcct 14460
   cgtgttgggc ggcaaacgag ctgttagacg aggagtggtt aacctcgtta agtatggccg 14520
   gtaacaaccg gagccagaag aaaaagaaaa atccagctcc aatggggaat ggccagccag 14580
   tcaatcaact gtgccagttg ctgggtgcaa tgatgaagtc ccagcgccag caacctaggg 14640
   gaggacaggc aagaaaaaga aagcctgaga agccacactt tcccctggct gctgaagatg 14700
   acgttcggca ccatctcacc cagactgagc gctccctctg cttgcaatcg atccagacgg 14760
   ccttcaatca aggcgcagga gttgcatcgc tttcatccag tgggaaggtc agttttcagg 14820
   ttgagttcat gttgccggtc gctcatacag tgcgcctgat tcgcgtgact tccacatccg 14880
   ccggtcagga tgcaaattaa tttaatagtc aggtgaatgg ccgcgattgg cgtgtggcct 14940
   ctgagtcacc tattcaatta gggcgatcac atgggggtca gacttaatta ggcaagaacc 15000
   atgtgaccga aatt 15014
<210> 2
   <211> 2368
   <212> PRT
   <213> Porcine reproductive and respiratory syndrome virus
<220>
   <221> MISC_FEATURE
   <222> (1)..(2368)
   <223> Open reading frame 1a
<400> 2
   Met Ser Gly Thr Phe Ser Arg Cys Met Cys Thr Pro Ala Ala Arg Val
   1               5                   10                  15
   Phe Trp Asn Ala Gly Gln Val Tyr Cys Thr Arg Cys Leu Ser Ala Arg
               20                  25                  30
   Ser Leu Leu Pro Leu Glu Leu Gln Asp Thr Asp Leu Gly Ala Ile Gly
           35                  40                  45
   Leu Phe His Lys Pro Lys Asp Lys Leu Arg Trp Lys Val Pro Ile Gly
       50                  55                  60
   Ile Pro Gln Val Glu Cys Ser Pro Ser Gly Cys Cys Trp Leu Ser Val
   65                  70                  75                  80
   Ile Phe Pro Leu Ala Arg Met Thr Ser Gly Asn His Asn Phe Leu Gln
                   85                  90                  95
   Arg Leu Val Lys Val Ala Glu Val Leu Tyr Arg Asp Gly Cys Leu Thr
               100                 105                 110
   Pro Arg His Leu Arg Glu Leu Gln Val Tyr Glu Arg Gly Cys Asn Trp
           115                 120                 125
   Tyr Pro Ile Thr Gly Pro Val Pro Gly Met Gly Val Phe Ala Asn Ser
       130                 135                 140
   Met His Val Ser Asp Gln Pro Phe Pro Gly Ala Thr His Val Leu Thr
   145                 150                 155                 160
   Asn Ser Pro Leu Pro Gln Gln Ala Cys Arg Gln Pro Phe Cys Pro Phe
                   165                 170                 175
   Glu Glu Ala His Ser Asp Val Tyr Lys Trp Lys Glu Phe Val Ile Phe
               180                 185                 190
   Val Asp Ser Leu Ser Asp Gly Gln Pro Arg Met Met Trp Thr Pro Gly
           195                 200                 205
   Ser Ser Asp Ser Ala Ala Leu Glu Ala Leu Pro Pro Gly Leu Glu Cys
       210                 215                 220
   Gln Val Glu Ile Leu Ile Arg Ser Phe Pro Ala His His Pro Val Asn
   225                 230                 235                 240
   Leu Ala Asp Trp Glu Leu Thr Glu Ser Pro Glu His Gly Phe Ser Phe
                   245                 250                 255
   Ser Thr Ser Gln Ser Ser Gly Tyr Leu Ala Gln Gly Pro Asp Gly Phe
               260                 265                 270
   Asp Gly Lys Cys Trp Leu Ser Cys Phe Leu Ser Leu Pro Ala Lys Val
           275                 280                 285
   Trp Arg His Glu Glu His Leu Ala Ser Ala Tyr Gly Tyr Gln Thr Lys
       290                 295                 300
   Trp Gly Val His Gly Lys Tyr Leu Gln Arg Arg Leu Gln Ile Asn Gly
   305                 310                 315                 320
   Ile Arg Ala Val Val Asp Pro Asp Gly Pro Ile His Val Glu Ala Leu
                   325                 330                 335
   Ser Cys Pro Gln Ser Trp Ile Arg His Leu Thr Met Glu Asp Asp Val
               340                 345                 350
   Thr Pro Gly Phe Val Arg Leu Ala Ser Leu Arg Ile Val Pro Asn Thr
           355                 360                 365
   Glu Pro Thr Ala Leu Pro Ile Phe Arg Phe Gly Ala His Lys Trp Tyr
       370                 375                 380
   Gly Ala Ala Gly Lys Arg Ala Arg Ala Lys Arg Ala Ala Lys Ser Glu
   385                 390                 395                 400
   Lys Asp Pro Ala Leu Thr Pro Lys Val Ala Gln Ser Ala Pro Thr Asn
                   405                 410                 415
   Gly Thr Thr Ile Tyr Ser Pro Pro Ala Asp Gly Ser Cys Gly Trp His
               420                 425                 430
   Val Leu Ala Ala Ile Met Asn Arg Met Val Asn Asn Asp Phe Thr Ser
           435                 440                 445
   Pro Leu Ala Gln Tyr Asn Arg Pro Glu Asp Asp Trp Ala Ser Asp Tyr
       450                 455                 460
   Asp Leu Ala Gln Ala Ile Gln Cys Leu Gln Leu Pro Ala Ala Ile Val
   465                 470                 475                 480
   Arg Asn Arg Ala Cys Pro Asn Ala Lys Tyr Leu Val Lys Leu Asn Gly
                   485                 490                 495
   Val His Trp Glu Val Glu Val Arg Ser Gly Met Ala Pro Arg Ser Leu
               500                 505                 510
   Ser Arg Glu Cys Val Val Gly Val Cys Ser Glu Gly Cys Ile Ala Leu
           515                 520                 525
   Pro Tyr Pro Lys Asp Gly Leu Pro Lys Arg Ala Pro Glu Ala Leu Ala
       530                 535                 540
   Ser Ala Tyr Arg Leu Pro Ser Asp Cys Val Ser Ala Gly Ile Ala Asp
   545                 550                 555                 560
   Phe Leu Ala Asn Pro Thr Pro Gln Glu Phe Trp Thr Leu Asp Lys Met
                   565                 570                 575
   Leu Thr Ser Pro Ser Pro Glu Arg Ser Gly Phe Ser Ser Leu Tyr Lys
               580                 585                 590
   Leu Leu Leu Glu Val Val Pro Gln Lys Cys Gly Ala Thr Glu Gly Ala
           595                 600                 605
   Phe Val Tyr Ala Val Glu Arg Met Leu Lys Asp Cys Pro Ser Ser Lys
       610                 615                 620
   Gln Ala Met Ala Leu Leu Ser Lys Ile Lys Val Pro Ser Ser Lys Ala
   625                 630                 635                 640
   Pro Ser Val Ser Leu Asp Glu Cys Phe Pro Thr Asp Val Ser Ala Asp
                   645                 650                 655
   Phe Glu Pro Thr Phe His Glu Arg Ser Gln Gly Ser Gly Thr Ala Gly
               660                 665                 670
   Val Ser Cys Ser Pro Asp Ala Lys Arg Phe Glu Lys Ala Thr Pro Glu
           675                 680                 685
   Glu Val Gln Glu Asp Gly His Glu Ala Val Arg Ser Thr Pro Phe Thr
       690                 695                 700
   Asp Asp Pro Thr Leu Ala Ser Ala Gly Ser Thr Asn Pro Val Asp Gly
   705                 710                 715                 720
   Asn Leu Ser Ser Ser Asp Ser Ala Glu Glu Ser Thr Leu Asn Ser Arg
                   725                 730                 735
   Glu Asp Glu Pro Leu Asp Leu Ser Gln Pro Ala Pro Val Val Thr Thr
               740                 745                 750
   Gly Leu Val Arg Glu Gln Thr Pro Cys Asp Leu Gly Phe Asp Ala Gly
           755                 760                 765
   Ala Pro Phe Val Thr Val Arg Lys Phe Val Pro Ile Gly Ser Thr Phe
       770                 775                 780
   Arg His Val Glu His Cys Gly Thr Glu Ser Asp Asn Ile Ser Ser Pro
   785                 790                 795                 800
   Leu Thr Leu Pro Asp Ala Gln Ala Leu Asp Gln Pro Leu Asp Leu Ser
                   805                 810                 815
   Leu Ala Ala Trp Pro Val Arg Ala Thr Ala Ser Asp Pro Gly Trp Val
               820                 825                 830
   His Gly Arg Arg Glu Pro Val Phe Val Lys Pro Arg Gly Thr Leu Ser
           835                 840                 845
   Asp Gly Asp Ser Val Leu Gln Phe Gly Glu Leu Ser Glu Ala Ser Ser
       850                 855                 860
   Val Val Glu Phe Asp Arg Leu Arg Glu Ile Pro Ala Val Asp Ala Pro
   865                 870                 875                 880
   Val Asp Leu Thr Pro Ser Asn Glu Ala Leu Ser Ala Asp Asp Pro Phe
                   885                 890                 895
   Arg Ser Ala Glu Phe Lys Arg Pro Arg Phe Ser Ala Gln Ala Leu Ile
               900                 905                 910
   Asp Arg Gly Gly Pro Leu Ala Asp Val His Ala Lys Ile Lys Ser Arg
           915                 920                 925
   Val Tyr Glu Gln Cys Leu Gln Ala Cys Glu Pro Gly Ser Arg Ala Thr
       930                 935                 940
   Pro Ala Thr Lys Ala Trp Leu Asp Lys Met Trp Asp Arg Val Asp Met
   945                 950                 955                 960
   Lys Thr Trp Arg Cys Thr Ser Gln Phe Gln Ala Gly Arg Ile Leu Ala
                   965                 970                 975
   Ser Leu Lys Phe Leu Pro Asp Met Ile Gln Asp Thr Pro Pro Pro Val
               980                 985                 990
   Pro Arg Lys Ser Arg Thr Ser Gly  Ser Ala Gly Leu Lys  Gln Leu Val
           995                 1000                1005
   Ala Gln  Trp Asp Arg Lys Leu  Ser Ala Thr Ser Gln  Lys Lys Pro
       1010                 1015                 1020
   Val Glu  Pro Met Arg Asn Gln  Ala Thr Pro Pro Pro  Thr Asp Ile
       1025                 1030                 1035
   Gln Gln  Glu Glu Val Thr Pro  Pro Asp Gly Ser Pro  His Ala Pro
       1040                 1045                 1050
   Asp Phe  Pro Ser Arg Val Ser  Met Ser Trp Asn Trp  Lys Gly Phe
       1055                 1060                 1065
   Met Leu  Ser Gly Thr Arg Phe  Ala Gly Ser Ile Ser  Gln Arg Leu
       1070                 1075                 1080
   Met Ala  Trp Val Phe Glu Ile  Val Ser His Leu Pro  Ala Phe Met
       1085                 1090                 1095
   Leu Thr  Leu Phe Ser Pro Arg  Gly Ser Met Ala Pro  Gly Asp Trp
       1100                 1105                 1110
   Leu Phe  Ala Gly Val Val Leu  Leu Ala Leu Leu Leu  Cys Arg Ser
   1115 1120 1125
   Tyr Pro Ile Leu Gly Cys Leu Pro Leu Leu Gly Val Phe Ser Gly
   1130 1135 1140
   Ser Leu Arg Arg Val Arg Leu Gly Val Phe Gly Ser Trp Met Ala
   1145 1150 1155
   Phe Ala Val Phe Leu Phe Ser Thr Pro Ser Asn Pro Val Gly Ser
   1160 1165 1170
   Ser Cys Asp His Asp Ser Pro Glu Cys His Ala Glu Leu Leu Ala
   1175 1180 1185
   Leu Glu Gln Arg Gln His Trp Glu Pro Val Arg Gly Leu Val Val
   1190 1195 1200
   Gly Pro Ser Gly Leu Leu Cys Val Ile Leu Gly Lys Leu Leu Gly
   1205 1210 1215
   Gly Ser Arg Tyr Phe Trp His Val Phe Leu Arg Leu Cys Met Leu
   1220 1225 1230
   Ala Asp Leu Ala Leu Ser Leu Val Tyr Val Val Ser Gln Gly Arg
   1235 1240 1245
   Cys His Lys Cys Trp Gly Lys Cys Ile Arg Thr Ala Pro Ala Glu
   1250 1255 1260
   Val Ala Leu Asn Val Phe Pro Phe Ser Arg Ala Thr Arg Asn Ser
   1265 1270 1275
   Leu Val Ser Leu Cys Asp Arg Phe Gln Ala Pro Lys Gly Val Asp
   1280 1285 1290
   Pro Val His Leu Ala Thr Gly Trp Arg Gly Cys Trp His Gly Glu
   1295 1300 1305
   Ser Pro Ile His Gln Gln His Gln Lys Pro Ile Ala Tyr Ala Asn
   1310 1315 1320
   Leu Asp Glu Lys Lys Ile Ser Ala Gln Thr Val Val Ala Val Pro
   1325 1330 1335
   Tyr Asp Pro Ser Gln Ala Ile Lys Cys Leu Lys Val Leu Gln Ala
   1340 1345 1350
   Gly Gly Ala Ile Val Asp Gln Pro Ile Pro Glu Val Val Arg Val
   1355 1360 1365
   Ser Glu Ile Pro Phe Ser Ala Pro Phe Phe Pro Lys Val Pro Val
   1370 1375 1380
   Asn Pro Asp Cys Arg Ile Val Val Asp Ser Asp Thr Phe Val Ala
   1385 1390 1395
   Ala Val Arg Cys Gly Tyr Ser Thr Thr Gln Leu Val Leu Gly Arg
   1400 1405 1410
   Gly Asn Phe Ala Lys Leu Asn Gln Thr Pro Leu Arg Gly Ser Val
   1415 1420 1425
   Ser Thr Lys Thr Ile Gly Gly Ala Ser Tyr Thr Met Ala Val Ala
   1430 1435 1440
   Gln Val Ser Val Trp Thr Leu Val His Phe Val Leu Gly Leu Trp
   1445 1450 1455
   Leu Thr Ser Pro Gln Val Cys Gly Arg Gly Thr Ser Asp Pro Trp
   1460 1465 1470
   Cys Ser Asn Pro Phe Ser Tyr Pro Thr Tyr Gly Pro Gly Val Val
   1475 1480 1485
   Cys Ser Ser Arg Leu Cys Val Ser Ala Asp Gly Val Thr Leu Pro
   1490 1495 1500
   Leu Phe Ser Ala Val Ala Gln Leu Ser Gly Arg Glu Val Gly Val
   1505 1510 1515
   Phe Ile Leu Val Leu Val Ser Leu Cys Ala Leu Ala His Arg Met
   1520 1525 1530
   Ala Leu Lys Ala Asp Met Leu Met Val Phe Leu Ala Phe Cys Ala
   1535 1540 1545
   Tyr Ala Trp Pro Met Ser Ser Trp Leu Ile Cys Phe Phe Pro Ile
   1550 1555 1560
   Leu Leu Lys Trp Val Thr Leu His Pro Leu Thr Leu Phe Trp Val
   1565 1570 1575
   His Ser Phe Leu Val Phe Cys Leu Pro Ala Ala Gly Val Leu Ser
   1580 1585 1590
   Leu Gly Ile Thr Gly Leu Leu Trp Ala Val Gly Arg Phe Thr Gln
   1595 1600 1605
   Ile Ala  Gly Ile Ile Thr Pro  Tyr Asp Ile His Gln  Tyr Thr Ser
       1610                 1615                 1620
   Gly Pro  Arg Gly Ala Ala Ala  Val Ala Thr Ala Pro  Glu Gly Thr
       1625                 1630                 1635
   Tyr Met  Ala Ala Val Arg Arg  Ala Ala Leu Thr Gly  Arg Thr Leu
       1640                 1645                 1650
   Ile Phe  Thr Pro Ser Ala Val  Gly Ser Leu Leu Glu  Gly Ala Phe
       1655                 1660                 1665
   Arg Thr  His Lys Pro Cys Leu  Asn Thr Val Asn Val  Val Gly Ser
       1670                 1675                 1680
   Ser Leu  Gly Ser Gly Gly Val  Phe Thr Ile Asp Gly  Arg Lys Thr
       1685                 1690                 1695
   Ile Val  Thr Ala Thr His Val  Leu Asn Gly Asp Thr  Ala Arg Val
       1700                 1705                 1710
   Thr Gly  Asp Ser Tyr Asn Arg  Met His Thr Phe Lys  Thr Asn Gly
       1715                 1720                 1725
   Asp Tyr  Ala Trp Ser His Ala  Asp Asp Trp Arg Gly  Val Ala Pro
       1730                 1735                 1740
   Ala Val  Asn Val Ala Lys Gly  Tyr Cys Gly Arg Ala  Tyr Trp Gln
       1745                 1750                 1755
   Thr Ser  Thr Gly Val Glu Pro  Gly Ile Ile Gly Asn  Gly Phe Ala
       1760                 1765                 1770
   Phe Cys  Phe Thr Asn Cys Gly  Asp Ser Gly Ser Pro  Val Ile Ser
       1775                 1780                 1785
   Glu Thr  Gly Asp Leu Ile Gly  Ile His Thr Gly Ser  Asn Lys Leu
       1790                 1795                 1800
   Gly Ser  Gly Leu Val Thr Thr  Pro Ala Gly Glu Thr  Cys Ala Ile
       1805                 1810                 1815
   Lys Glu  Thr Lys Leu Ser Asp  Leu Ser Lys His Phe  Ala Gly Pro
       1820                 1825                 1830
   Ser Val  Pro Leu Gly Asp Ile  Lys Leu Ser Pro Ala  Ile Ile Pro
       1835                 1840                 1845
   Asp Val  Thr Ser Val Pro Ser  Asp Leu Ala Ser Leu  Leu Ala Ser
       1850                 1855                 1860
   Val Pro  Val Met Glu Gly Gly  Leu Ser Thr Val Gln  Leu Leu Cys
       1865                 1870                 1875
   Val Phe  Phe Leu Leu Trp Arg  Met Met Gly His Ala  Trp Thr Pro
       1880                 1885                 1890
   Ile Val  Ala Val Gly Phe Phe  Leu Leu Asn Glu Ile  Leu Pro Ala
       1895                 1900                 1905
   Val Leu  Val Arg Ala Val Phe  Ser Phe Ala Leu Phe  Val Leu Ala
       1910                 1915                 1920
   Trp Val  Thr Pro Trp Ser Ala  Gln Val Leu Met Ile  Arg Leu Leu
       1925                 1930                 1935
   Thr Ala  Ser Leu Asn Arg Asn  Lys Leu Ser Leu Ala  Phe Tyr Ala
       1940                 1945                 1950
   Leu Gly  Gly Val Ala Gly Leu  Ala Ala Glu Ile Gly  Thr Phe Ala
       1955                 1960                 1965
   Gly Arg  Phe Ser Glu Leu Ser  Gln Ala Leu Ser Thr  Tyr Cys Phe
       1970                 1975                 1980
   Leu Pro  Arg Val Leu Ala Val  Thr Ser Cys Val Pro  Ile Ile Ile
       1985                 1990                 1995
   Ile Gly  Gly Leu His Ala Leu  Gly Val Ile Leu Trp  Leu Phe Lys
       2000                 2005                 2010
   Tyr Arg  Cys Leu His Asn Met  Leu Val Gly Asp Gly  Ser Phe Ser
       2015                 2020                 2025
   Ser Ala  Phe Phe Leu Arg Tyr  Phe Ala Glu Gly Asn  Leu Arg Lys
       2030                 2035                 2040
   Gly Val  Ser Gln Ser Cys Gly  Met Asn Asn Glu Ser  Leu Thr Ala
       2045                 2050                 2055
   Ala Leu  Ala Cys Lys Leu Ser  Gln Ala Asp Leu Asp  Phe Leu Ser
       2060                 2065                 2070
   Ser Leu  Thr Asn Phe Lys Cys  Phe Val Ser Ala Ser  Asn Met Lys
       2075                 2080                 2085
   Asn Ala  Ala Gly Gln Tyr Ile  Glu Ala Ala Tyr Ala  Lys Ala Leu
   2090 2095 2100
   Arg Gln Glu Leu Ala Ser Leu Val Gln Ile Asp Lys Met Lys Gly
   2105 2110 2115
   Val Leu Ser Lys Leu Glu Ala Phe Ala Glu Thr Ala Thr Pro Ser
   2120 2125 2130
   Leu Asp Thr Gly Asp Val Ile Val Leu Leu Gly Gln His Pro His
   2135 2140 2145
   Gly Ser Ile Leu Asp Ile Asn Val Gly Thr Glu Arg Lys Thr Val
   2150 2155 2160
   Ser Val Gln Glu Thr Arg Ser Leu Gly Gly Thr Lys Phe Ser Val
   2165 2170 2175
   Cys Thr Val Val Ser Asn Thr Pro Val Asp Ala Leu Thr Ser Ile
   2180 2185 2190
   Pro Leu Gln Thr Pro Thr Pro Leu Phe Glu Asn Gly Pro Arg His
   2195 2200 2205
   Arg Ser Glu Glu Asp Asp Leu Lys Val Glu Arg Met Lys Lys His
   2210 2215 2220
   Cys Val Ser Leu Gly Tyr His Asn Ile Asn Gly Lys Val Tyr Cys
   2225 2230 2235
   Lys Ile Trp Asp Lys Ser Thr Gly Asp Thr Phe Tyr Thr Asp Asp
   2240 2245 2250
   Ser Arg Tyr Thr Gln Asp Tyr Ala Phe Gln Asp Arg Ser Ala Asp
   2255 2260 2265
   Tyr Arg Asp Gly Gly Tyr Glu Gly Val Gln Thr Ala Pro Gln His
   2270 2275 2280
   Gly Phe Asp Pro Lys Ser Glu Thr Pro Ile Gly Thr Val Val Ile
   2285 2290 2295
   Gly Gly Ile Thr Tyr Asn Arg Tyr Leu Ile Lys Gly Lys Glu Val
   2300 2305 2310
   Leu Val Pro Lys Pro Asp Asn Cys Leu Glu Ala Ala Lys Leu Ser
   2315 2320 2325
   Leu Glu Gln Ala Leu Ala Gly Met Gly Gln Thr Cys Asp Leu Thr
   2330 2335 2340
   Ala Ala Glu Val Glu Lys Leu Lys Arg Ile Ile Ser Gln Leu Gln
   2345 2350 2355
   Gly Leu Thr Thr Glu Gln Ala Leu Asn Cys
   2360 2365
<210> 3
   <211> 1463
   <212> PRT
   <213> Porcine reproductive and respiratory syndrome virus
<220>
   <221> MISC_FEATURE
   <222> (1)..(1463)
   <223> Open reading frame 1b
<400> 3
   Ala Gly Phe Lys Leu Leu Ala Ala Ser Gly Leu Thr Arg Cys Gly Arg
   1               5                   10                  15
   Gly Gly Leu Val Val Thr Asp Thr Ala Val Lys Ile Ile Lys Tyr His
               20                  25                  30
   Ser Arg Thr Phe Thr Leu Gly Pro Ile Asp Leu Lys Val Thr Ser Glu
           35                  40                  45
   Val Glu Val Lys Lys Ser Thr Glu Gln Gly His Ala Val Val Ala Asn
       50                  55                  60
   Leu Cys Ser Gly Val Val Leu Met Arg Pro His Pro Pro Ser Leu Val
   65                  70                  75                  80
   Asp Val Leu Leu Lys Pro Gly Leu Asp Ile Ala Pro Gly Ile Gln Pro
                   85                  90                  95
   Gly His Gly Ala Gly Asn Met Gly Val Asp Gly Ser Val Trp Asp Phe
               100                 105                 110
   Glu Thr Ala Pro Thr Lys Ala Glu Leu Glu Leu Ser Lys Gln Ile Ile
           115                 120                 125
   Gln Ala Cys Glu Val Arg Arg Gly Asp Ala Pro Asn Leu Gln Leu Pro
       130                 135                 140
   Tyr Lys Leu Tyr Pro Val Arg Gly Asp Pro Glu Arg His Glu Gly Tyr
   145                 150                 155                 160
   Leu Ile Asn Thr Arg Phe Gly Asp Leu Pro Tyr Lys Thr Pro Gln Asp
                   165                 170                 175
   Thr Lys Ser Ala Ile His Ala Ala Cys Cys Leu His Pro Asn Gly Val
               180                 185                 190
   Leu Val Ser Asp Gly Lys Ser Ala Leu Gly Thr Thr Leu Gln His Gly
           195                 200                 205
   Phe Glu Leu Tyr Val Pro Thr Val Pro Tyr Ser Val Met Glu Tyr Leu
       210                 215                 220
   Asp Ser Arg Pro Asp Thr Pro Pro Met Phe Thr Lys His Gly Thr Ser
   225                 230                 235                 240
   Lys Ala Ala Ala Glu Asp Leu Gln Lys Tyr Asp Leu Ser Thr Gln Gly
                   245                 250                 255
   Phe Val Leu Pro Gly Val Leu Arg Leu Met Arg Arg Phe Ile Phe Gly
               260                 265                 270
   His Ile Gly Lys Ala Pro Pro Leu Phe Leu Pro Ser Thr Tyr Pro Ala
           275                 280                 285
   Lys Asn Ser Met Ala Gly Ile Asn Gly Gln Arg Phe Pro Thr Lys Asp
       290                 295                 300
   Val Gln Ser Ile Pro Glu Ile Asp Glu Val Cys Ala Arg Ala Val Lys
   305                 310                 315                 320
   Glu Asn Trp Gln Thr Val Thr Pro Cys Thr Leu Lys Lys Gln Tyr Cys
                   325                 330                 335
   Ser Lys Pro Lys Thr Arg Thr Ile Leu Gly Thr Asn Asn Phe Ile Ala
               340                 345                 350
   Leu Ala His Arg Ser Ala Leu Ser Gly Val Thr Gln Ala Phe Met Lys
           355                 360                 365
   Lys Ala Trp Lys Ser Pro Ile Ala Leu Gly Lys Asn Lys Phe Lys Glu
       370                 375                 380
   Leu His Cys Thr Val Ala Gly Arg Cys Leu Glu Ala Asp Leu Ala Ser
   385                 390                 395                 400
   Cys Asp Arg Ser Thr Pro Ala Ile Val Arg Trp Phe Val Ala Asn Leu
                   405                 410                 415
   Leu Tyr Glu Leu Ala Gly Cys Glu Glu Tyr Leu Pro Ser Tyr Val Leu
               420                 425                 430
   Asn Cys Cys His Asp Leu Val Ala Thr Gln Asp Gly Ala Phe Thr Lys
   435 440 445
   Arg Gly Gly Leu Ser Ser Gly Asp Pro Val Thr Ser Val Ser Asn Thr
   450 455 460
   Val Tyr Ser Leu Ile Ile Tyr Ala Gln His Met Val Leu Ser Ala Leu
   465 470 475 480
   Lys Met Gly His Glu Ile Gly Leu Lys Phe Leu Glu Glu Gln Leu Lys
   485 490 495
   Phe Glu Asp Leu Leu Glu Ile Gln Pro Met Leu Val Tyr Ser Asp Asp
   500 505 510
   Leu Val Leu Tyr Ala Glu Lys Pro Thr Phe Pro Asn Tyr His Trp Trp
   515 520 525
   Val Glu His Leu Asp Leu Met Leu Gly Phe Lys Thr Asp Pro Lys Lys
   530 535 540
   Thr Val Ile Thr Asp Lys Pro Ser Phe Leu Gly Cys Lys Ile Glu Ala
   545 550 555 560
   Gly Arg Gln Leu Val Pro Asn Arg Asp Arg Ile Leu Ala Ala Leu Ala
   565 570 575
   Tyr His Met Lys Ala Gln Asn Ala Ser Glu Tyr Tyr Ala Ser Ala Ala
   580 585 590
   Ala Ile Leu Met Asp Ser Cys Ala Cys Ile Asp His Asp Pro Glu Trp
   595 600 605
   Tyr Glu Asp Leu Ile Cys Gly Ile Ala Arg Cys Ala Arg Gln Asp Gly
   610 615 620
   Tyr Ser Phe Pro Gly Pro Pro Phe Phe Leu Ser Met Trp Glu Lys Leu
   625 630 635 640
   Lys Ser His Asn Glu Gly Lys Lys Phe Arg His Cys Gly Ile Cys Asp
   645 650 655
   Ala Lys Ala Asp His Ala Ser Ala Cys Gly Leu Asp Leu Cys Leu Phe
   660 665 670
   His Ser His Phe His Gln His Cys Pro Val Thr Leu Ser Cys Gly His
   675 680 685
   His Ala Gly Ser Arg Glu Cys Pro Gln Cys Gln Ser Pro Val Gly Ala
       690                 695                 700
   Gly Lys Ser Pro Leu Asp Ala Val Leu Lys Gln Ile Pro Tyr Lys Pro
   705                 710                 715                 720
   Pro Arg Thr Val Ile Met Lys Val Asp Asn Lys Thr Thr Thr Leu Asp
                   725                 730                 735
   Pro Gly Arg Tyr Gln Ser Arg Arg Gly Leu Val Ala Val Lys Arg Gly
               740                 745                 750
   Ile Ala Gly Asn Glu Val Asp Leu Thr Asp Gly Asp Tyr Gln Val Val
           755                 760                 765
   Pro Leu Leu Pro Thr Cys Lys Asp Ile Asn Met Val Lys Val Ala Val
       770                 775                 780
   Asn Val Leu Leu Ser Lys Phe Ile Val Gly Pro Pro Gly Ser Gly Lys
   785                 790                 795                 800
   Thr Thr Trp Leu Leu Ser Gln Val Gln Asp Asp Asp Val Ile Tyr Thr
                   805                 810                 815
   Pro Thr His Gln Thr Met Phe Asp Ile Val Asn Ala Leu Lys Val Cys
               820                 825                 830
   Arg Tyr Ser Ile Pro Gly Ala Ser Gly Leu Pro Phe Pro Pro Pro Ala
           835                 840                 845
   Arg Ser Gly Pro Trp Val Arg Leu Val Ala Ser Gly His Ile Pro Gly
       850                 855                 860
   Arg Ile Ser Tyr Leu Asp Glu Ala Gly Tyr Cys Asn His Leu Asp Ile
   865                 870                 875                 880
   Leu Arg Leu Leu Ser Lys Thr Pro Leu Val Cys Leu Gly Asp Leu Gln
                   885                 890                 895
   Gln Leu His Pro Val Gly Phe Asp Ser Tyr Cys Tyr Val Phe Asp Gln
               900                 905                 910
   Met Pro Gln Lys Gln Leu Thr Thr Ile Tyr Arg Phe Gly Pro Asn Ile
           915                 920                 925
   Cys Ala Ala Ile Gln Ser Cys Tyr Arg Glu Lys Leu Glu Ser Lys Ala
       930                 935                 940
   Arg Asn Thr Arg Val Val Phe Thr Thr Arg Pro Val Asp Phe Gly Gln
   945 950 955 960
   Val Leu Thr Pro Tyr His Lys Asp Arg Ile Gly Ser Ala Ile Thr Ile
   965 970 975
   Asp Ser Ser Gln Gly Ala Thr Phe Asp Val Val Thr Leu His Leu Pro
   980 985 990
   Ser Pro Met Ser Leu Asn Lys Ser Arg Ala Leu Val Ala Ile Thr Arg
   995 1000 1005
   Ala Arg Tyr Gly Leu Phe Ile Tyr Asp Pro His Asp Gln Leu Arg
   1010 1015 1020
   Glu Phe Phe Asn Leu Thr Pro Glu Arg Thr Asp Cys Asn Leu Val
   1025 1030 1035
   Phe Asn Arg Gly Glu Glu Leu Val Val Leu Asn Ala Asp Asn Ala
   1040 1045 1050
   Val Thr Thr Val Ala Lys Ala Leu Glu Ala Gly Pro Ser Arg Phe
   1055 1060 1065
   Gln Val Ser Asp Pro Arg Cys Lys Ser Leu Leu Ala Ala Cys Ser
   1070 1075 1080
   Val Ser Phe Glu Gly Cys Cys Met Pro Leu Pro Gln Val Ala His
   1085 1090 1095
   Asn Leu Gly Phe Tyr Phe Ser Pro Asp Ser Pro Val Phe Ala Pro
   1100 1105 1110
   Leu Pro Lys Glu Leu Ala Pro His Trp Pro Val Val Thr Tyr Gln
   1115 1120 1125
   Asn Asn Lys Ala Trp Pro Asp Arg Leu Val Ala Ser Met Arg Pro
   1130 1135 1140
   Ile Asp Ala His Tyr Ser Lys Pro Met Val Gly Ala Gly Tyr Val
   1145 1150 1155
   Val Gly Pro Ser Thr Phe Leu Gly Ala Pro Gly Val Val Ser Tyr
   1160 1165 1170
   Tyr Leu Thr Leu Tyr Ile Lys Gly Glu Pro Gln Ala Leu Pro Glu
   1175 1180 1185
   Thr Leu Val Ser Thr Gly Arg Ile Ala Thr Asp Cys Arg Glu Tyr
   1190 1195 1200
   Leu Asp  Ala Ala Glu Glu Glu  Ala Ala Lys Glu Leu  Pro His Ala
       1205                 1210                 1215
   Phe Ile  Gly Asp Val Lys Gly  Thr Thr Val Gly Gly  Cys His His
       1220                 1225                 1230
   Ile Thr  Ser Lys Tyr Leu Pro  Arg Phe Leu Pro Lys  Asp Ser Val
       1235                 1240                 1245
   Ala Val  Val Gly Val Ser Ser  Pro Gly Lys Ala Ala  Lys Ala Val
       1250                 1255                 1260
   Cys Thr  Leu Thr Asp Val Tyr  Leu Pro Glu Leu Arg  Pro Tyr Leu
       1265                 1270                 1275
   Gln Pro  Glu Thr Ala Ser Lys  Cys Trp Lys Leu Lys  Leu Asp Phe
       1280                 1285                 1290
   Arg Asp  Val Arg Leu Met Val  Trp Lys Gly Ser Thr  Ala Tyr Phe
       1295                 1300                 1305
   Gln Leu  Glu Gly Leu Thr Trp  Ser Ala Leu Pro Asp  Tyr Ala Arg
       1310                 1315                 1320
   Phe Ile  Gln Val Pro Lys Asp  Ala Val Val Tyr Ile  Asp Pro Cys
       1325                 1330                 1335
   Ile Gly  Pro Ala Thr Ala Asn  Arg Lys Val Val Arg  Thr Thr Asp
       1340                 1345                 1350
   Trp Arg  Ala Asp Leu Ala Val  Thr Pro Tyr Asp Tyr  Gly Ala Pro
       1355                 1360                 1365
   Asn Ile  Leu Thr Thr Ala Trp  Phe Glu Asp Leu Gly  Pro Gln Trp
       1370                 1375                 1380
   Lys Ile  Leu Gly Leu Gln Pro  Phe Lys Arg Ala Phe  Gly Phe Glu
       1385                 1390                 1395
   Thr Thr  Glu Asp Trp Ala Ile  Leu Ala Arg Arg Met  Asn Asp Gly
       1400                 1405                 1410
   Lys Asp  Tyr Thr Asp Tyr Asn  Trp Asn Cys Val Arg  Gln Arg Pro
       1415                 1420                 1425
   His Ala  Val His Gly Arg Ala  Arg Asp His Thr Tyr  His Phe Ala
       1430                 1435                 1440
   Pro Gly  Ile Glu Leu Gln Val  Glu Leu Gly Lys Pro  Arg Leu Gln
       1445                 1450                 1455
   Pro Glu  Gln Val Pro
       1460
<210> 4
   <211> 249
   <212> PRT
   <213> Porcine reproductive and respiratory syndrome virus
<220>
   <221> MISC_FEATURE
   <222> (1)..(249)
   <223> Open reading frame 2a
<400> 4
   Met Gln Trp Gly His Cys Gly Ala Lys Ser Pro Ser Tyr Leu Trp Thr
   1               5                   10                  15
   Pro Ser Leu Ser Ser Leu Leu Val Trp Leu Thr Leu Ser Ser Ser Leu
               20                  25                  30
   Pro Tyr Cys Leu Gly Ser Pro Ser Gln Asp Gly Tyr Trp Ser Phe Phe
           35                  40                  45
   Ser Glu Trp Phe Ala Pro Arg Phe Ser Val Arg Ala Leu Pro Phe Thr
       50                  55                  60
   Leu Pro Asn Tyr Arg Arg Ser Tyr Glu Gly Leu Leu Pro Asn Cys Arg
   65                  70                  75                  80
   Pro Asp Val Pro Gln Phe Ala Phe Lys His Pro Leu Gly Met Phe Trp
                   85                  90                  95
   His Met Arg Val Ser His Leu Ile Asp Glu Met Val Ser Arg Arg Ile
               100                 105                 110
   Tyr Gln Thr Met Glu His Ser Gly Gln Ala Ala Trp Lys Gln Val Val
           115                 120                 125
   Ser Glu Ala Thr Leu Thr Lys Leu Ser Asn Leu Asp Ile Val Leu His
       130                 135                 140
   Phe Gln His Leu Ala Ala Val Glu Ala Asp Ser Cys Arg Phe Leu Ser
   145                 150                 155                 160
   Ser Arg Leu Val Met Leu Lys Asn Leu Ala Val Gly Asn Val Ser Leu
                   165                 170                 175
   His Tyr Asn Thr Thr Leu Asp Ser Val Glu Leu Ile Phe Pro Thr Pro
               180                 185                 190
   Gly Thr Arg Pro Lys Leu Thr Asp Phe Arg Gln Trp Leu Val Ser Val
           195                 200                 205
   His Ala Ser Ile Phe Ser Ser Val Ala Ser Ser Val Thr Leu Phe Ile
       210                 215                 220
   Val Leu Trp Leu Arg Ile Pro Ala Leu Arg Tyr Val Phe Gly Phe His
   225                 230                 235                 240
   Trp Pro Thr Ala Thr Arg His Ser Asn
                   245
<210> 5
   <211> 70
   <212> PRT
   <213> Porcine reproductive and respiratory syndrome virus
<220>
   <221> MISC_FEATURE
   <222> (1)..(70)
   <223> Open reading frame 2b
<400> 5
   Met Gly Ser Leu Trp Ser Lys Ile Thr Gln Leu Phe Val Asp Ala Phe
   1               5                   10                  15
   Thr Glu Phe Leu Val Ser Val Val Asp Ile Val Ile Phe Leu Ala Ile
               20                  25                  30
   Leu Phe Gly Phe Thr Val Ala Gly Trp Leu Leu Val Phe Leu Leu Arg
           35                  40                  45
   Val Val Cys Ser Ala Leu Leu Arg Ser Arg Ser Ala Ile His Ser Ser
       50                  55                  60
   Glu Leu Ser Lys Val Leu
   65                  70
<210> 6
   <211> 265
   <212> PRT
   <213> Porcine reproductive and respiratory syndrome virus
<220>
   <221> MISC_FEATURE
   <222> (1)..(265)
   <223> Open reading frame 3
<400> 6
   Met Ala Cys Gln Cys Thr Arg Phe Tyr Phe Phe Leu Cys Gly Leu Ile
   1               5                   10                  15
   Cys Tyr Leu Ile His Ser Ala Leu Ala Ser Asn Ser Ser Ser Thr Leu
               20                  25                  30
   Cys Phe Trp Phe Pro Leu Ala His Gly Asn Thr Ser Phe Glu Leu Thr
           35                  40                  45
   Ile Asn Tyr Thr Ile Cys Lys Pro Cys Leu Thr Ser Gln Ala Ala Ser
       50                  55                  60
   Gln Arg Leu Glu Pro Gly Arg Asn Val Trp Cys Lys Ile Gly His Asp
   65                  70                  75                  80
   Lys Cys Glu Glu Arg Asp His Asp Glu Leu Leu Met Ser Ile Pro Ser
                   85                  90                  95
   Gly Tyr Asp Asn Leu Lys Leu Glu Gly Tyr Tyr Ala Trp Leu Ala Phe
               100                 105                 110
   Leu Ser Phe Ser Tyr Ala Ala Gln Phe His Pro Glu Leu Phe Gly Ile
           115                 120                 125
   Gly Asn Val Ser Arg Val Phe Val Asp Lys Arg His Gln Phe Ile Cys
       130                 135                 140
   Ala Ala His Asp Gly His Asn Ser Thr Val Ser Thr Ser His Asn Ile
   145                 150                 155                 160
   Ser Ala Ser Tyr Ala Val Tyr Tyr His His Gln Ile Asp Gly Gly Asn
                   165                 170                 175
   Trp Phe His Leu Glu Trp Leu Arg Pro Phe Phe Ser Ser Trp Leu Val
               180                 185                 190
   Leu Asn Ile Ser Trp Phe Leu Arg Arg Ser Pro Ala Ser Pro Val Ser
           195                 200                 205
   Arg Arg Ile Tyr Gln Ile Leu Arg Pro Thr Arg Pro Arg Leu Pro Val
       210                 215                 220
   Ser Trp Ala Phe Arg Thr Ser Ile Ala Ser Asp Ile Thr Gly Ser Arg
   225                 230                 235                 240
   Gln Arg Arg Gly Glu Phe Thr Pro Glu Gly Arg Pro Asn Ala Val Lys
                   245                 250                 255
   Ser Ser Val Leu Arg Ser Thr Ser Arg
   260 265
<210> 7
   <211> 183
   <212> PRT
   <213> Porcine reproductive and respiratory syndrome virus
<220>
   <221> MISC_FEATURE
   <222> (1)..(183)
   <223> Open reading frame 4
<400> 7
   Met Ala Ala Ala Ile Leu Phe Leu Met Val Gly Ala Gln His Ile Met
   1 5 10 15
   Val Ser Glu Ala Phe Ala Cys Lys Pro Cys Phe Ser Thr His Leu Ser
   20 25 30
   Asp Ile Lys Thr Asn Thr Thr Ala Ala Ala Gly Phe Met Gly Leu Gln
   35 40 45
   Asn Ile Asp Cys Leu Arg His His Arg Val Thr Ala Ala Gln Gly Arg
   50 55 60
   Ile Tyr Thr Arg Gly Pro Ser Gln Cys Arg Glu Val Val Gly Thr Pro
   65 70 75 80
   Gln Tyr Ile Thr Val Thr Ala Asn Val Thr Asp Glu Ser Tyr Leu Tyr
   85 90 95
   Asn Ala Asp Leu Leu Met Leu Ser Ala Cys Leu Phe Tyr Ala Ser Glu
   100 105 110
   Met Ser Glu Lys Gly Phe Lys Val Ile Phe Gly Asn Val Ser Gly Val
   115 120 125
   Val Ser Ala Cys Val Asn Phe Thr Asp Tyr Val Ala His Val Thr Gln
   130 135 140
   His Thr Gln Gln His His Leu Val Ile Asp His Val Arg Leu Leu His
   145 150 155 160
   Phe Leu Thr Pro Ser Ala Met Arg Trp Ala Thr Thr Ile Ala Cys Leu
   165 170 175
   Phe Ala Ile Leu Leu Ala Val
   180
<210> 8
   <211> 201
   <212> PRT
   <213> Porcine reproductive and respiratory syndrome virus
<220>
   <221> MISC_FEATURE
   <222> (1)..(201)
   <223> Open reading frame 5
<400> 8
   Met Arg Cys Ser His Lys Leu Gly Arg Phe Leu Thr Pro His Ser Cys
   1               5                   10                  15
   Phe Trp Trp Leu Phe Leu Leu Cys Thr Gly Leu Ser Trp Ser Phe Ala
               20                  25                  30
   Asp Gly Asn Gly Asn Ser Ser Thr Tyr Gln Tyr Ile Tyr Asn Leu Thr
           35                  40                  45
   Ile Cys Glu Leu Asn Gly Thr Thr Trp Leu Ser Asp His Phe Tyr Trp
       50                  55                  60
   Ala Val Glu Thr Phe Val Leu Tyr Pro Val Ala Thr His Ile Leu Ser
   65                  70                  75                  80
   Leu Gly Phe Leu Thr Thr Ser His Leu Leu Asp Ala Leu Gly Leu Gly
                   85                  90                  95
   Ala Val Ser Val Ala Gly Phe His Gly Gly Arg Tyr Val Leu Ser Ser
               100                 105                 110
   Val Tyr Ser Ala Cys Ala Leu Ala Ala Leu Val Cys Phe Val Ile Arg
           115                 120                 125
   Ala Ala Lys Asn Cys Met Ala Cys Arg Tyr Ala Arg Thr Arg Phe Thr
       130                 135                 140
   Asn Phe Ile Val Asp Asp Arg Gly Arg Ile His Arg Trp Arg Ser Pro
   145                 150                 155                 160
   Ile Val Val Glu Arg Leu Gly Lys Ala Asp Val Ser Gly Asp Leu Val
                   165                 170                 175
   Thr Ile Lys His Val Val Leu Glu Gly Val Lys Ala Gln Pro Leu Thr
               180                 185                 190
   Arg Thr Ser Ala Glu Gln Trp Glu Ala
           195                 200
<210> 9
   <211> 173
   <212> PRT
   <213> Porcine reproductive and respiratory syndrome virus
<220>
   <221> MISC_FEATURE
   <222> (1)..(173)
   <223> Open reading frame 6
<400> 9
   Met Gly Ser Leu Asp Asp Phe Cys Gly Asp Pro Thr Ala Ala Gln Lys
   1               5                   10                  15
   Leu Val Leu Ala Phe Ser Ile Thr Tyr Thr Pro Ile Met Ile Tyr Ala
               20                  25                  30
   Leu Lys Val Ser Arg Gly Arg Leu Leu Gly Leu Leu His Ile Leu Ile
           35                  40                  45
   Phe Leu Asn Cys Ser Phe Thr Phe Gly Tyr Met Thr Tyr Val His Phe
       50                  55                  60
   Glu Ser Thr Asn Arg Val Ala Phe Thr Met Gly Ala Val Val Ala Leu
   65                  70                  75                  80
   Leu Trp Gly Val Tyr Ser Phe Ile Glu Ser Trp Lys Phe Ile Thr Ser
                   85                  90                  95
   Arg Cys Arg Leu Cys Cys Leu Gly Arg Arg Tyr Ile Leu Ala Pro Ala
               100                 105                 110
   His His Val Glu Ser Ala Ala Gly Leu His Pro Ile Pro Ala Ser Gly
           115                 120                 125
   Asn His Ala Tyr Ala Val Arg Lys Pro Gly Leu Thr Ser Val Asn Gly
       130                 135                 140
   Thr Leu Val Pro Gly Leu Arg Gly Leu Val Leu Gly Gly Lys Arg Ala
   145                 150                 155                 160
   Val Arg Arg Gly Val Val Asn Leu Val Lys Tyr Gly Arg
                   165                 170
<210> 10
   <211> 128
   <212> PRT
   <213> Porcine reproductive and respiratory syndrome virus
<220>
   <221> MISC_FEATURE
   <222> (1)..(128)
   <223> Open reading frame 7
<400> 10
   Met Ala Gly Asn Asn Arg Ser Gln Lys Lys Lys Lys Asn Pro Ala Pro
   1               5                   10                  15
   Met Gly Asn Gly Gln Pro Val Asn Gln Leu Cys Gln Leu Leu Gly Ala
               20                  25                  30
   Met Met Lys Ser Gln Arg Gln Gln Pro Arg Gly Gly Gln Ala Arg Lys
           35                  40                  45
   Arg Lys Pro Glu Lys Pro His Phe Pro Leu Ala Ala Glu Asp Asp Val
       50                  55                  60
   Arg His His Leu Thr Gln Thr Glu Arg Ser Leu Cys Leu Gln Ser Ile
   65                  70                  75                  80
   Gln Thr Ala Phe Asn Gln Gly Ala Gly Val Ala Ser Leu Ser Ser Ser
                   85                  90                  95
   Gly Lys Val Ser Phe Gln Val Glu Phe Met Leu Pro Val Ala His Thr
               100                 105                 110
   Val Arg Leu Ile Arg Val Thr Ser Thr Ser Ala Gly Gln Asp Ala Asn
           115                 120                 125
<210> 11
   <211> 3189
   <212> DNA
   <213> Porcine reproductive and respiratory syndrome virus
<400> 11
   atgcaatggg gtcactgtgg agcaaaatca cccagttatt tgtggacgcc ttcactgagt      60
   tccttgttag tgtggttgac attgtcatct tccttgccat actgtttggg ttcaccgtcg     120
   caggatggtt actggtcttt cttctcagag tggtttgctc cgcgcttctc cgttcgcgct     180
   ctgccattca ctcttccgaa ctatcgaagg tcttatgagg gcttacttcc caattgcaga     240
   ccggatgttc cacaatttgc atttaagcac ccattgggta tgttttggca catgcgggtt     300
   tcccatctga ttgatgaaat ggtctctcgc cgcatctacc agaccatgga acattcaggt     360
   caagcggctt ggaagcaggt ggttagtgag gccactctca caaaactgtc aaatctcgac     420
   atagttcttc acttccaaca cctggccgcg gtggaggcgg attcttgccg cttcctcagc     480
   tcacgacttg tgatgctgaa aaatcttgcc gttggcaatg tgagcctgca ctacaacacc     540
   acgttggaca gcgttgagct catcttcccc acaccaggta cgagacctaa gttgaccgat     600
   ttcaggcaat ggcttgtcag tgtacacgct tctatttttt cctctgtggc ctcatctgtt     660
   accttattca tagtgctttg gcttcgaatt ccagctctac gctatgtttt tggtttccat      720
   tggcccacgg caacacgtca ttcgaattga ccatcaacta caccatatgc aagccctgcc      780
   ttactagtca ggcggcttcc cagaggctcg aacccggtcg caacgtgtgg tgtaaaatag      840
   ggcatgataa atgtgaggag cgcgaccatg atgagttatt aatgtccatt ccgtctgggt      900
   acgacaacct caaactcgag ggttattatg cttggttggc gtttttgtcc ttttcctacg      960
   cggcccaatt ccatccagag ttgtttggga tagggaatgt gtcgcgtgtc tttgtggaca     1020
   agcgacacca gttcatttgt gccgcacatg atggacacaa ttcaaccgtg tctaccagcc     1080
   acaacatctc cgcatcatat gcagtgtatt accaccacca aatagacggg ggcaattggt     1140
   ttcatttgga atggctgcgg ccattctttt cctcatggtt ggtgctcaac atatcatggt     1200
   ttctgaggcg ttcgcctgca agccctgttt ctcgacgcat ttatcagata ttaagaccaa     1260
   cacgaccgcg gctgccggtt tcatgggcct tcagaacatc gattgcctcc gacatcacag     1320
   ggtcacggca gcgcaggggc gaatttacac cagagggccg tcccaatgcc gtgaagtcgt     1380
   cggtactccg cagtacatca cggtaacggc caatgtgacc gacgagtcat acttgtacaa     1440
   cgcggacctg ctgatgcttt ctgcgtgcct tttctatgcc tcggaaatga gcgagaaggg     1500
   cttcaaagtc atctttggga atgtctctgg cgttgtttcc gcttgcgtca atttcactga     1560
   ttatgtggct catgtgactc aacacaccca gcagcatcat ctggtgatcg atcatgtccg     1620
   gttgctgcat ttcttgacac cgtctgcaat gaggtgggcc acaaccattg cttgtttgtt     1680
   cgccatcctc ttggcggtat gagatgttct cacaaattgg ggcgcttctt gactccacac     1740
   tcttgcttct ggtggctttt tttgctgtgt accggcttgt cttggtcctt tgccgatggc     1800
   aacggcaaca gctcgacata ccaatacata tataatttga cgatatgcga gctgaatggg     1860
   accacttggt tgtccgatca cttttattgg gcagtcgaga cttttgtgct ttacccagtg     1920
   gcaactcaca ttctctcatt gggtttcttt acaacaagcc atctccttga cgcgctcggt     1980
   ctcggagccg tgtccgttgc cggttttcat ggcggacggt acgtactcag cagcgtgtac     2040
   agcgcttgtg cattagcagc gcttgtgtgt tttgttatcc gcgctgccaa aaattgcatg     2100
   gcttgccgct atgctcgtac ccgatttacc aacttcattg tggacgaccg ggggagaatc     2160
   catcgatgga ggtctccaat agtggtggag agattgggca aagctgacgt cagcggcgac     2220
   cttgtcacca tcaaacatgt tgtcctcgag ggagttaaag ctcaaccctt gacaaggact     2280
   tcggccgagc aatgggaagc ctagatgact tttgtggcga tcccaccgcc gcacaaaagc     2340
   ttgtgctagc ctttagcatc acatatacac ctattatgat atacgccctt aaggtgtcac     2400
   gcggccggct cctggggctg ttgcacatct tgatatttct gaactgttcc ttcacgttcg     2460
   gatacatgac atatgtacat tttgagtcca ccaaccgtgt cgcatttact atgggggccg     2520
   tcgttgccct tttgtggggc gtctatagct tcatagagtc atggaagttt atcacttcca     2580
   gatgcagatt gtgttgccta ggccggcgat acattctggc ccctgcccac cacgtagaaa 2640
   gtgctgcagg cctccatccg attccagcgt ctggtaacca tgcatatgct gtgagaaagc 2700
   ccggactaac atcagtgaac ggcactctag taccaggact tcggggcctc gtgttgggcg 2760
   gcaaacgagc tgttagacga ggagtggtta acctcgttaa gtatggccgg taacaaccgg 2820
   agccagaaga aaaagaaaaa tccagctcca atggggaatg gccagccagt caatcaactg 2880
   tgccagttgc tgggtgcaat gatgaagtcc cagcgccagc aacctagggg aggacaggca 2940
   agaaaaagaa agcctgagaa gccacacttt cccctggctg ctgaagatga cgttcggcac 3000
   catctcaccc agactgagcg ctccctctgc ttgcaatcga tccagacggc cttcaatcaa 3060
   ggcgcaggag ttgcatcgct ttcatccagt gggaaggtca gttttcaggt tgagttcatg 3120
   ttgccggtcg ctcatacagt gcgcctgatt cgcgtgactt ccacatccgc cggtcaggat 3180
   gcaaattaa 3189
<210> 12
   <211> 3171
   <212> DNA
   <213> Porcine reproductive and respiratory syndrome virus
<400> 12
   atgcaatggg ttcattgtgg agcaaaatca gccaattgtt cgtggatgcc ttcactgaat 60
   ttcttgttag tgtggttgat attatcatct tccttgccat attgtttggg ttcacagtcg 120
   caggatggtt actggtcttc cttctcagag tggtttgctc cgcgcttctc cgttcgcgct 180
   ctgccattca ctcttccgaa ttatcgaagg tcctatgagg gcttactacc taattgcaga 240
   ccggatgccc cacaatttgc attcaagcac cccttgggta tgttttggca cgtgcgggtt 300
   tcccacttaa ttgatcaaat ggtctcccgc cgcatttacc agaccatgga acattcaggt 360
   caagcggcct ggaagcacgt ggttagtgag gctaccctca caaagctgtc agagctcgat 420
   atagttctcc atttccaaca cctggccgca gtggaagcgg actcttgcca cttccttagt 480
   tcacgacttg tgatgctgaa aaatctagcc gttggcaacg tgagcatact ttataacaac 540
   acattggacc gcgtcgagct cgtcttccct acgctaaaca cgaggcccaa gttgaccgat 600
   ttcagacaat ggcttatcag tgtgcatgct tccatcttct cctctgtggc ctcgtcggtc 660
   accttgtttg tggtactctg gctccgaatt ccagctctac gctatgtttt tggtttccat 720
   tggcccacgg caacacgtcg ttcgagctga ccatcaatta caccatatgt gagccctgtc 780
   ctactaggca agcggctcaa caaaggctcg aacccggtcg taacatgtgg tgtaggatag 840
   ggagcactac atgtgaggag cgtgatcacg atgagttgtc catgacaatt ccgtccggat 900
   acgacaacct caaacttgag ggttattatg cttggctggc ctttttgtcc ttttcctatg 960
   cagctcaatt ccatccggag ttgttcggaa taggaaacgt gtcgcgcgtc tttgtggaca 1020
   agcggcacca gtttatttgc gctgagcatg acgggcagaa ttcaaccgta cccatcaggc 1080
   acaacatctc tgcattgtat gcagcctatt accaccacca aatagacggg ggcaattggt 1140
   tccatctaga atggctgcga ccattctttt cttcctggct ggtgctcaac atttcatggt 1200
   ttctgaggcg ttcgcctgca agccctgctt ctcgacgcac ctatcagata ttaagactaa 1260
   cacgaccgcg gctgccggtt tcatggtcct tcaaaacatc aattgtcccc aaacaggggc 1320
   tagcgcttca ttcagaaagt cgccccaatg tcgtgaggcc gttggcaccc ctcagtacat 1380
   cacgataaca gccaacgtga ccgacgagtc atatttatac aacgctgatt tgctgatgct 1440
   ctctgcgtgt cttttctacg cttcggaaat gagtgagaag ggcttcaaag tcatctttgg 1500
   gaatgtctct ggtgttgttt ccgcatgcgt taatttcacg gattacgtgg ctcatgtaac 1560
   tcaacataca cagcaacacc atctggtgat cgatcgcgtt cggttgctgc acttcctgac 1620
   accgtctgtg atgaggtggg ctacaaccat cgcttgctta tttgccattc ttctggcgat 1680
   atgagatgtt cttacaaatt ggggcgttcc ttgactcctc actcttactt ctggtggctt 1740
   tttttgctgt gtacaggctt gtcttggtcc tttgccgatg gcaacggcaa cagctcgaca 1800
   taccaataca tatataattt gacaatatgc gagctgaatg ggaccacctg gctgtccgac 1860
   agatttggtt gggcagtcga ggcctttgtg ctttacccgg tggtgactca cattctctca 1920
   ctgggatttc taacaacaag ccattttttt gacgcactcg gccttggagc tgtgtccgtt 1980
   accggctact atgacgggcg gtatgtactc agtagcgtgt acggcacttg tgcgcttgca 2040
   gcgctcgtgt gcttcgccat ccgtgccgct aaaaattgca tggcttgccg ctatgcccgt 2100
   acccgattca caaattttat tgtggacgac cggggtggag tccatcggtg gaagtctcca 2160
   atagtggtgg agaaattagg caaagccgaa gttggtgacg cccttgttac catcaagcat 2220
   gtcgtcatcg aaggggttaa agctcaaccc ttgacaagga cctcagctga gcaatggcag 2280
   gcctagatga tttttgccat gattccaccg ccgcagaaaa gcttgtgcta gcctttagca 2340
   tcacatacac acccatcatg atatacgctc ttaaagtgtc tcgcggccga ctcttggggt 2400
   tgctgcacat cttgatattc ctgaactgtt ctttcacatt cggatacatg acatatgtgc 2460
   attttgaatc caccaaccgc gtcgcattta ccatgggagc tgttgtcgcc cttttgtggg 2520
   gcatttatag tttcatagag tcctggaagt ttattacttc tagatgcaga ttgtgttgcc 2580
   taggccggcg atacattttg gcccctgccc accacgtaga aagtgctgca ggtctccatc 2640
   cgatcccagc gtctggtaac caagcatacg ctgtgaggaa gcccggacta acatcagtga 2700
   acggcactct ggtaccagga cttcggggcc tcgtgttggg cggcaaacga gctgtaaaac 2760
   gaggagtggt taacctcgtc aagtatggcc ggtaaaaacc agtgccagaa gagaaataaa 2820
   aatccagctc caatggggaa tggccagtca gtcaatcaac tgtgccagtt gttgggttca 2880
   atgatgaaat cccagcgcca gcgacccagg ggtggacagg ttaaaaagaa gaagcctgag 2940
   aagccgcatt tccccttggc tgctgaagat gacgtccggc accatctcac ccaaaccgag 3000
   cgctcccttt gcttgcaatc gatccagacg gcttttaatc aaggcgcagg aactgcgtcg 3060
   ctttcatcca gcgggaaggt cggttttcag gttgagttca tgctgccggt tgctcataca 3120
   gtgcgcctga ttcgcgtgac ttccacatcc gccggtcagg gtgcaaatta a 3171
<210> 13
   <211> 3178
   <212> DNA
   <213> Porcine reproductive and respiratory syndrome virus
<220>
   <221> misc_feature
   <222> (1803)..(1803)
   <223> n is a, c, g, or t
<400> 13
   atgcgatggg atcactgtgg agtaaaatta cccagctgtt tgttgacgcc ttcactgaat      60
   tccttgttag tgtggttgac attgtcatct ttcttgccat actgtttggg tttacagtcg     120
   caggttggtt attggtcttt cttttcagag tggtttgctc cgcgtttctc cgttcgcgct     180
   ctgccattca ctctcccgaa ttatcgaaga tcttatgaag gtttgttgcc caattgcaga     240
   cctgatgtcc cacaattcgc agttaaacac ccattaggca tgttttggca catgcgggtc     300
   tcccacctaa ttgatgaaat ggtctctcgt cgcatttacc agaccatgga acattcaggt     360
   caagctgcct ggaaacaggt ggttagtgag gctactctca cgaaattgtc aaagctcgac     420
   atagtcctcc acttccaaca tctagccgca gttgaggcgg attcttgtcg cttcctcagt     480
   tcacggcttg tgatgttgaa aaatcttgcc gttggcaatg tgagcttgca gtacaacacc     540
   actttggacc acgttgagct catttttcca acaccaggca caaggcctaa attaaccgat     600
   tttagacaat ggctcgtcag tgtgcatgct tcaatttttt cttctgtagc ctcatcaatt     660
   accttgttca tagtgctttg gcttcgagcc ccaattctac gctatgtttt tggtttccat     720
   tggcccacgg caacacatca ttcgagctaa ccatcaatta cactatatgt gagccttgtc     780
   ttaccagtca agcagctaaa caaaggctcg aacccggtcg taacatgtgg tgcaaaatag     840
   gacataccac atgtgaagag catgaccatg atgagttatc aatgactatc ccacccgggt     900
   acgacaatct caaacttgag ggttattatg cctggttggc tttcttgtcc ttctcttacg     960
   cagcccaatt ccacccggag ttgttcggaa tagggaatgt gtcacgcgtt ttcgtggaca    1020
   aacgacacca gttcatctgt gccgagcatg acggacaaaa ttcaaccgta tccacagaac    1080
   acaacatttc tgcactgtat gcggtgtact accatcacca agtggacgga gacaattggt    1140
   tccatctgga atggctgcgg ccattctttt cctcttggct ggtgctcaac atctcctggt    1200
   ttctgaggcg ttcgcctgta agccctgttt ctcaacgcat ttatcagata ttaagaccaa    1260
   cacgactgcg gctgccggtt tcatggtcct tcagaacatc aattgtctcc ggccctacag    1320
   gaccaacaca acgcaaggga aagtaccatc tcaatgtcgt gaagccgtcg gtactcccca    1380
   gtacattatg atgacggcta acgtgaccga caagtcatac ttgtataatg cggacctgtt    1440
   gatgctttct gcatgccttt tctacgcctc ggaaatgagt gagaagggct tcaaagtcat 1500
   ctttgggaat atctctggcg ttgtctccgc ttgtgtcaat ttcacggatt atgtggccca 1560
   tgtgacccaa catacccagc agcatcatct ggtgattgat cacattcggt tactgcattt 1620
   tctgtcaccg tctacaatga ggtgggctgc aaccatcgcc tgcttgtttg ccattctttt 1680
   ggcgatatga gatgttctca caaattgggg cgttccttga ctccacactc ttatttctgg 1740
   tggctttttt ctttgtgtat cggcttatcc tggtcctttg ccgatggcaa cggcaacagc 1800
   tcnacatacc aatacatata taacttgacc atatgcgagc tgaatgggac cacctggttg 1860
   tccggccatt ttgactgggc agtcgaaaca tttgtgcttt acccggttac cactcatatt 1920
   ctttcactgg gttttctcac aacaagccat ttttttgacg cgctcggtct cggcgccgtg 1980
   tccgctgcag gatttgttga cagtcggtac gtgctcagca gcgtctacgg cgcttgtgct 2040
   ttcgcggcgt tcgtatgttt tgccatccgt gccaccaaaa attgcatggc ctgccgctat 2100
   gcccgcaccc ggttcaccaa ctttattgtt gatgaccggg ggagaatcca tcgatggaaa 2160
   tctccaatag tgatagaaaa attgggcaga gccgacgtgg gcggcgacct tgtcaccatc 2220
   aaacacgttg tcctcgaagg agttaaagct caacctttgg caaggacttc ggctgagcaa 2280
   tgggaagcct agatgatttc tgtcatgatc ctaccgccgc acaaaagctt gtgctggctt 2340
   tcagcattac atatacaccc attatgatat acgcccttaa agtgtcacgc ggccgactct 2400
   tgggactgtt acacatctta atattcctga actgttcctt cacgttcgga tatatgacat 2460
   atgtgcattt tgaatccact aaccgcgttg cacttactat gggagccgtt gtcgctcttt 2520
   tgtggggcgt ctatagcttc atagagtcat ggaagtttgt tacttccaga tgcagactgt 2580
   gttgcctagg tcggcgatac attttggccc ctgcccacca cgtagaaagt gctgcaggcc 2640
   ttcatcggat cccagcgtct ggtaaccaag catacgctgt gagaaagccc ggactaacat 2700
   cagtaaacgg cactctagta ccagggcttc gaggcctcgt gttgggcggc aaacgagctg 2760
   ttaaacgagg agtggttaac ctcgtcaagt atggccggta ggaaccggag ccagaagaaa 2820
   aagaaaaatc cagctccaat ggggaatgac caaccagtca atcaactgtg ccagctgctg 2880
   ggcgcaatga tgaagtcccg gcgccagcaa cctaggggag gacaggctaa aaagagaaag 2940
   cctgagaagc cacattttcc cctagctgct gaagatgacg ttcggcacca tctcacccag 3000
   actgagcgct ccctctgctt gcaatcgatc caaacggctt ttaatcaagg cgcaggagtt 3060
   gcgtcgcttt catccagcgg gaaggtcagt tttcaggttg agttcatgct gccggtcgct 3120
   catacagtgc gcctgattcg cgtgacttcc acatccgcca gtcaggatgc aaattaat 3178

## Description

The present invention relates to a vaccine for prevention of viremia induced by Porcine Reproductive and Respiratory Syndrome Virus (PRRSV) infection. More specifically, it relates to a vaccine that is protecting against infections with the European PRRSV type strain and derivatives thereof, as well as against infections with isolates where currently commercially available vaccines, based on the European type strain do not offer an efficient protection. The invention relates further to methods for preparing said vaccine, and methods to protect pigs from reproductive and respiratory syndrome, with the use of this vaccine.

Porcine reproductive and respiratory syndrome virus (PRRSV) is a member of the family *Arteriviridae,* order Nidovirales (Cavanagh, 1997; Snijder and Meulenberg, 1998) causing major economic losses in the pig industry worldwide (Neumann *et al.,* 2005). PRRSV infection may result in reproductive failure in sows and is involved in the porcine respiratory disease complex (PRDC) (Christianson *et al.,* 1992, 1993; Lager and Mengeling, 1995; Mengeling *et al*., 1994; *Rossow et al.,* 1994; Terpstra *et al.*, 1991). Genetic typing allows making a distinction between the European (EU, type 1) and the North American (NA, type 2) genotype, which share about 63% nucleotide homology on the base of the full length sequence (Allende *et al.,* 1999). However, even within the European, respectively North American genotypes, a lot of genetic variability has been demonstrated. PRRSV appears to evolve by random mutagenesis and recombination events (Cho and Dee, 2006). This heterogeneity is posing a major obstacle for effective prevention and control of PRRSV (Meng, 2000).

Indeed, several vaccines have been developed and are commercially available, but those vaccines are only effective within either the European or the North American subgroup, and even within the specific subgroup, they fail to provide a substantial disease control, especially against recent, genetically more diverse isolates. Therefore there is a need for a vaccine that can protect against infection with those isolates that are not fully covered by currently available commercial vaccines, at least those isolates belonging to the same genotype.

Attempts to produce a vaccine based on the novel isolates have been made, but at least in some cases, those vaccines have been reported to be less efficient against heterologous challenge than the available commercial vaccine (Diaz et al., 2006).

Moreover, the present vaccines are normally life attenuated vaccines. An inactivated vaccine would be safer in use, but inactivated vaccines are less efficient than life attenuated vaccines. In this respect, it is important to compare the efficacy of a vaccine within its own group (life attenuated or inactivated). An inactivated vaccine approaching the efficacy of a life attenuated vaccine would certainly have an advantage in use.

Surprisingly we found that a vaccine, based on a new isolate 07V-063, characterized by its genomic sequence as shown in SEQ ID N° 1, confers an efficient protection against homologous as well as against heterologous challenge, even when the vaccine is based on inactivated virus. In addition, two further new PRRSV isolates, i.e. 08V-204 and 08V-194, characterized by the genomic sequences as shown in SEQ ID N° 13 and SEQ ID N°12 respectively, demonstrate to be good vaccine candidates.

The scope of the present invention is defined by the claims and any embodiment that does not fall within the claims is provided for information only.

Disclosed is a vaccine against PRRSV in pigs, preferably a broad spectrum vaccine, even more preferably a broad spectrum vaccine against Type 1 PRRSV, said vaccine comprising a type 1 PRRSV strain with less than 90% identity at DNA level with the EuroPRRSV strain (Genbank accession number AY366525; version AY366525.1), or a functional part of said PRRSV strain, capable of inducing an anti-PRRSV immune response.

A "vaccine" as used herein refers to a composition comprising the PRRSV strain, nucleic acids or proteins derived therefrom, as described herein which is administered to stimulate an immune response that will protect the subject from illness. The vaccine of the present invention can be used for either prophylactic or therapeutic immunization and suitable vaccination routes include, but are not limited to, intranasal, subcutaneous and intramuscular immunization. Suitable vaccination routes also comprise combination administrations (e.g. oral/ intramuscular administration). The vaccine may be designed so that it may be delivered to pigs in a convenient manner. For example, the vaccine may be administered to pigs orally through the food supply (e.g., with feed, milk or milk replacer, and/or water). Alternatively, the vaccine may be administered in other suitable manner including parenterally, intravenously, intramuscularly, topically, or subcutaneously. Of course, the route of administration may be dictated or otherwise determined by the form of the vaccine. Optionally, the vaccine is administered with a pharmacologically acceptable carrier or diluents, known in the art. As used here, a type 1 strain is a strain that shows a higher percentage identity at nucleotide level with the European type strain (EuroPRRSV) than with the North American type strain, (VR-2332, GenBank accession number U87392 AF030244 U00153; version U87392.3) as measured by BLASTn align (Zhang et al, 2000) for at least one open reading frame, preferably as measured on the base of the whole genome. A type 2 strain is a strain that shows a higher percentage identity at nucleotide level with the North American type strain than with the European type strain, as measured by BLASTn for at least one open reading frame, preferably as measured on the base of the whole genome. The 90% identity, as used here, is calculated using a BLASTn align (Zhang et al, 2000), using the homologous open reading frames of ORF3, ORF4 or ORF5 (Mardassi et al, 1995; Morozov et al., 1995), preferably of ORF3, ORF4 and ORF5, more preferably using the whole genome sequence. Preferably, the identity is in a range of 50% - 89% with EuroPRRSV, even more preferably between 60% - 89%, most preferably between 70% and 89%. As most of the European strains show a high percentage identity at nucleotide level (Ropp et al. 2004), it is surprising that a strain with lower genetic identity can be used as a broad spectrum vaccine.

In a specific embodiment of the present disclosure the genome of the type I PRRSV strain comprises SEQ ID N° 1, or a homologue thereof. In a further embodiment, the genome of the type I PRRSV strain comprises SEQ ID N° 13, or a homologue thereof. In an even further embodiment, the genome of the type I PRRSV strain comprises SEQ ID N° 12, or a homologue thereof.

A homologue, as used here is a strain with at least 95% identity, preferable at least 96%, more preferably at least 97% identity, more preferably at least 98% identity, even more preferably at least 99% identity at nucleotide level, measured using BLASTn, using the homologous open reading frames, in particular of ORF3, ORF4 or ORF5, preferably of ORF3, ORF4 and ORF5, more preferably using the whole genome sequence. In a particular embodiment, the genome of said PRRSV strain consists of a nucleic acid with the sequence as shown in SEQ ID N°1.

In a specific embodiment, the vaccine as described herein comprises the natural occurring whole virus or a modified life virus. In another embodiment, said vaccine is a life attenuated vaccine or a naturally occurring variant that shows reduced infection in pigs, both in the level and duration of viremia. Methods for attenuation are known to the person skilled in the art and include, but are not limited to the isolation of less virulent mutants, possibly after mutagenesis. A life attenuated virus is not longer causing viremia nor disease symptoms. Disease symptoms as used here are fever (>40°C), loss of appetite and loss of activity. In a further embodiment, said vaccine comprises an inactivated virus. Methods for inactivation are known to the person skilled in the art and include, but are not limited to formaldehyde treatment, gluteraldehyde treatment, AT-2 treatment, pH and/ or temperature denaturation, gamma irradiation, UV irradiation and binary ethyleneimine (BEI) treatment. Preferably said inactivated virus is a BEI-inactivated virus. A preferred method for inactivation is disclosed in WO2009106629. The type I PRRSV strain comprising in its genome the nucleic acid sequence characterized by SEQ ID N° 13 is an example of a viral strain that can be part of a vaccine as a natural occurring attenuated virus, or a natural attenuated strain, that shows reduced infection in pigs, both in the level and duration of viremia. Naturally occurring virus means a virus that has not been extensively passaged in vitro to obtain an attenuated virus, preferably, less than a 10^{th} passage of the virus is used. Upon infection of pigs, the viremia of such a virus is preferably lower than 10exp3/ml serum at the peak level and duration of viremia is preferably less than 14 days post inoculation.

A functional part of the PRRSV virus, as used here is a part of the virus that can induce immunity in the host, i.e. that is capable of inducing an anti-PRRSV immune response, i.e. an antibody and/or cellular response against the PRRSV strain as described herein. Such parts are, as a non-limiting example, one or more proteins of the virus, proteins fragments or peptides carrying at least one epitope capable of inducing a (protective) immune response against PRRSV infection, or parts of the viral DNA, encoding such protein or proteins fragments or peptides. Therefore, a preferred embodiment of the disclosure is a vaccine against PRRSV, wherein said vaccine comprises at least one protein or protein fragment derived from strain 07V-063, 08V-204 or 08V-194, or a variant thereof. Preferably, said vaccine comprises at least one protein or protein fragment derived from strain 07V-063 or 08V-204, most preferably it comprises at least one protein from strain 07V-063. Strain 07V-063 is characterized by its genetic material, as shown in SEQ ID N° 1 or SEQ ID N°11. Strain 08V-204 is characterized by its genetic material, comprising the sequence as shown in SEQ ID N° 13. Strain 08V-194 is characterized by its genetic material, comprising the sequence as shown in SEQ ID N° 12. A variant, as used here, is a strain with minor mutations in the genome, wherein said mutations can be caused, as a non-limiting example, by several passages in cell culture. Preferably, said variant shows at least 95% identity, preferable at least 96%, more preferably at least 97% identity, more preferably at least 98% identity, even more preferably at least 99% identity at nucleotide level of the nucleic acid encoding the protein, measured using BLASTn, using the homologous open reading frames of ORF3, ORF4 or ORF5, preferably of ORF3, ORF4 and ORF5, more preferably using of the whole genome sequence. Preferably, said protein is derived from the strain 07V-063, 08V-204 or 08V-194. Even more preferably, said protein is derived from strain 07V-063 or 08V-204, most preferably said protein is derived from strain 07V-063. In another embodiment, said vaccine consists of one or more purified proteins of the strain 07V-063, 08V-204 or 08V-194, or a variant thereof. Preferably said protein or proteins are derived from the strain 07V-063, 08V-204 or 08V-194, even more preferably, said protein or proteins are derived from strain 07V-063 or 08V-204, most preferably said protein or proteins are derived from strain 07V-063. Said protein may be isolated on the base of the natural strain, or alternatively, they may be produced using recombinant techniques. In the latter case, said proteins may be fused, either chemically or genetically, to carriers to improve the genetic response. In another preferred embodiment, said vaccine is a DNA vaccine comprising SEQ ID N° 1, SEQ ID N° 13 or SEQ ID N°12, or a variant thereof, or functional fragments of said SEQ ID or variant. A functional fragment as used here is an open reading frame (ORF) that encodes one or more antigenic determinants of the virus. Preferably, it is one of the known ORF's of the virus. Preferably, said vaccine is a DNA vaccine comprising SEQ ID N° 1 or SEQ ID N° 13, even more preferably said vaccine is a DNA vaccine comprising SEQ ID N° 1.

Described is a type 1 PRSSV strain with less than 90% identity at DNA level with the EuroPRRSV strain (accession number AY366525; version AY366525.1), or a functional fragment thereof, for use as a medicament, preferably for the preparation of a vaccine against PRRSV type 1 infection in pigs. The 90% identity, as used here, is calculated using a BLASTn align (Zhang et al, 2000), using the homologous open reading frames, preferably using the whole genome sequence. Preferably, the identity is in a range of 50% - 89% with EuroPRRSV, even more preferably between 60% - 89%, most preferably between 70% and 89%. Preferably, the genome of said type 1 PRRSV strain comprises SEQ ID N° 1, SEQ ID N°13 or SEQ ID N°12, or a homologue thereof. A homologue, as used here is a strain with at least 95% identity, preferable at least 96%, more preferably at least 97% identity, more preferably at least 98% identity, even more preferably at least 99% identity at nucleotide level, measured using BLASTn, using the homologous open reading frames of ORF3, ORF4 or ORF5, preferably of ORF3, ORF4 and ORF5, more preferably on the base of the whole genome sequence. Most preferably, the genome of said PRRSV strain consists of a nucleic acid with the sequence as shown in SEQ ID N°1. In one preferred embodiment, said strain is a live attenuated strain. In another preferred embodiment, said strain is an inactivated strain, preferably a BEI inactivated strain.

In one preferred embodiment, the vaccine according to the invention comprises a complete, inactivated form of strain 07V-063 as deposited with the BCCM™ with accession number LMBP 6660. Preferably, said inactivation is a BEI inactivation. The use of strain 07V-063 has several advantages above the strain used in commercial vaccines. First, it is easy to grow, either in Marc-145 cells, or in PK15^{Sn-CD163} cells. Moreover, after vaccination, there is a protection against homologous challenge, as well as against heterologous challenge. It is important to note that commercial vaccines do not protect against infection with strain 07V-063, whereas this strain, when used as safe inactivated vaccine, still shows a broad spectrum protection.

In another preferred embodiment, the vaccine according to the invention comprises a complete, naturally attenuated form of strain 08V-204. A particular advantage of strain 08V-204 is that this strain, when used as naturally attenuated vaccine, gives an excellent protection in heterologous challenge experiments, that is even better than the protection obtained with commercial attenuated vaccines.

Described is a whole type 1 PRSSV strain with less than 90% identity at DNA level with the EuroPRRSV strain (accession number AY366525; version AY366525.1), or a functional fragment thereof, for use in treatment of PRRSV infection, preferably type 1 PRRSV infection in pigs. The 90% identity, as used here, is calculated using a BLASTn align (Zhang et al, 2000), using the homologous open reading frames, preferably using the whole genome sequence. Preferably, the identity is in a range of 50% - 89% with EuroPRRSV, even more preferably between 60% - 89%, most preferably between 70% and 89%. Preferably, the genome of said type 1 PRRSV strain comprises SEQ ID N° 1, SEQ ID N°13 or SEQ ID N°12, or a homologue thereof, even more preferably, said genome comprises SEQ ID N° 1 or SEQ ID N° 13, most preferably said genome comprises SEQ ID N°1. A homologue, as used here is a strain with at least 95% identity, preferable at least 96%, more preferably at least 97% identity, more preferably at least 98% identity, even more preferably at least 99% identity at nucleotide level, measured using BLASTn, using the homologous open reading frames of ORF3, ORF4 or ORF5, preferably of ORF3, ORF4 and ORF5, more preferably using the whole genome sequence. More preferably, the genome of said PRRSV strain consists of a nucleic acid with the sequence as shown in SEQ ID N°1, SEQ ID N°13 or SEQ ID N°12, even more preferably the genome of said PRRSV strain consists of a nucleic acid with the sequence as shown in SEQ ID N°1 or SEQ ID N°13, most preferably the genome of said PRRSV strain consists of a nucleic acid with the sequence as shown in SEQ ID N°1. In specific embodiment, said strain is a natural strain or a live attenuated strain, preferably strain 08V-204. In another embodiment, said strain is an inactivated strain, preferably a BEI inactivated strain, even more preferably, it is an inactivated strain 07V-063, most preferably it is a BEI inactivated 07V-063 strain.

Still another aspect of the disclosure is a method for preventing or reducing reproductive or respiratory failure in a pig, preferably type 1 PRRSV, said method comprising (a) providing a vaccine against PRRSV wherein said vaccine comprises at least one protein derived from strain 07V-063, 08V-204 or 08V-194, preferably derived from 07V-063 or 08V-204, even more preferably derived from 07V-063, or a variant thereof and (b) administering said vaccine to said pig. Reducing reproductive or respiratory failure, as used here, means that the clinical symptoms of the disease in the treated animals are less severe than in the non-treated animals. Such reduction can be measured, as a non-limiting example, as a reduction in fever, or a reduction in viremia. Preferably, said protein is derived from strain 07V-063, 08V-204 or 08V-194 preferably it is derived from 07V-063 or 08V-204, even more preferably it is derived from 07V-063. In a particular embodiment, said vaccine is a natural attenuated virus, a modified live virus, or a live attenuated vaccine. In another embodiment, said vaccine comprises an inactivated virus. Methods for inactivation are known to the person skilled in the art and include, but are not limited to UV irradiation and binary ethyleneimine (BEI) treatment. Preferably said inactivated virus is a BEI-inactivated virus. A preferred method for inactivation is disclosed in WO2009106629. In another preferred embodiment, said vaccine comprises or consists of one or more purified proteins of the strain 07V-063, 08V-204 or 08V-194, preferably one or more proteins of the strain 07V-063 or 08V-204, even more preferably one or more proteins of the strain 07V-063 or a variant thereof. Preferably said proteins are derived from the strain 07V-063, 08V-204, or 08V-194, preferably derived from 07V-063 or 08V-204, even more preferably derived from 07V-063. Said protein may be isolated on the basis of the natural strain, or alternatively, they may be produced using recombinant techniques. In the latter case, said proteins may be fused, either chemically or genetically, to carriers to improve the genetic response. In another preferred embodiment, said vaccine is a DNA vaccine comprising SEQ ID N° 1, SEQ ID N° 13 or SEQ ID N°12, or a variant thereof, or functional fragments of said SEQ ID or variant. A functional fragment as used here is an open reading frame (ORF) that encodes one or more antigenic determinants of the virus. Preferably, it is one of the known ORF's of the virus.

The work leading to this invention has received funding from the European Community's Seventh Framework Programme (FP7/2007-2013) under grant agreement nº 245141.

### BRIEF DESCRIPTION OF THE FIGURES

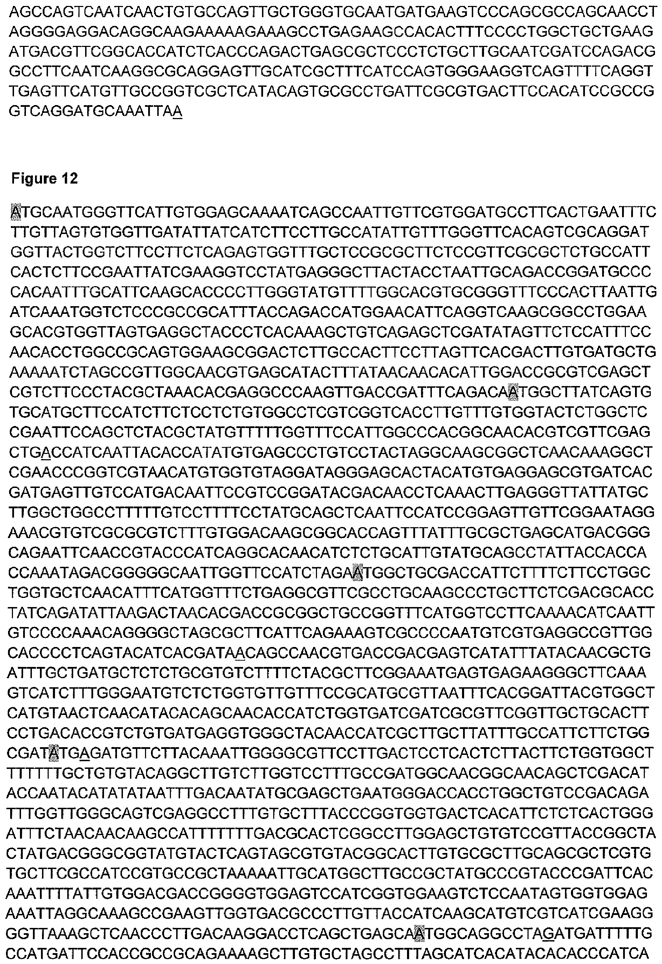

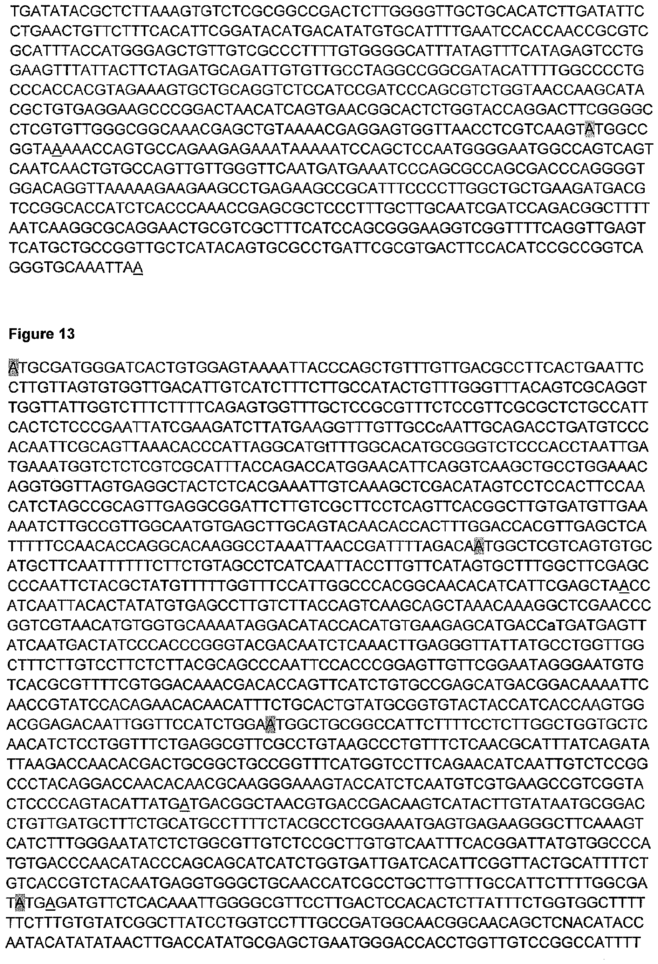

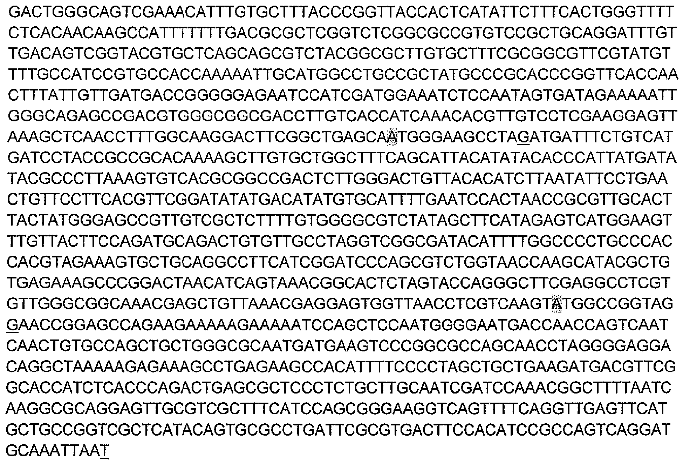

Figure 1: schedule of the vaccination experiment: time of sampling, with indication of the analyses carried out on the sample (IPMA, SN and virus titration)

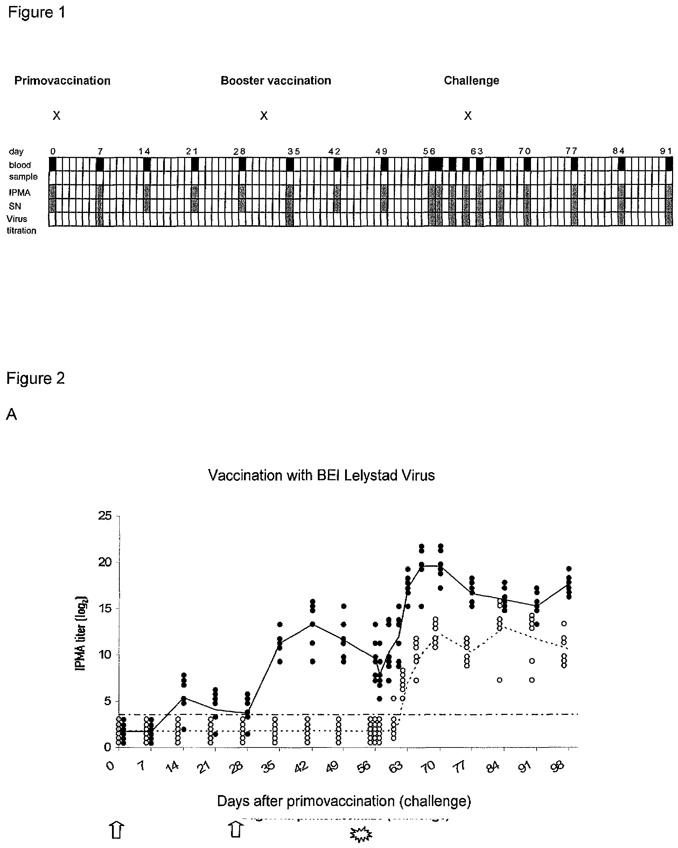

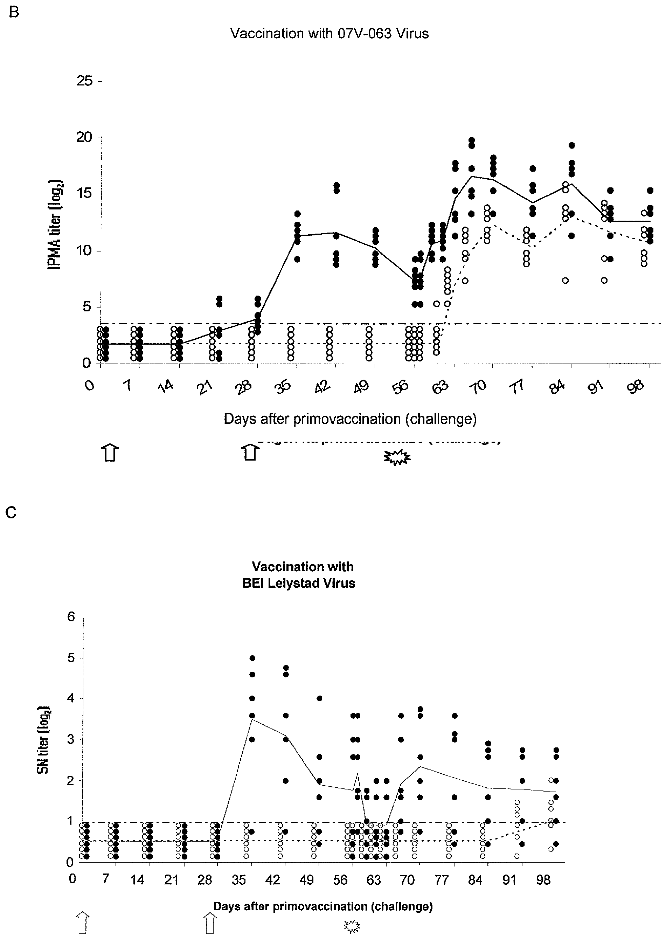

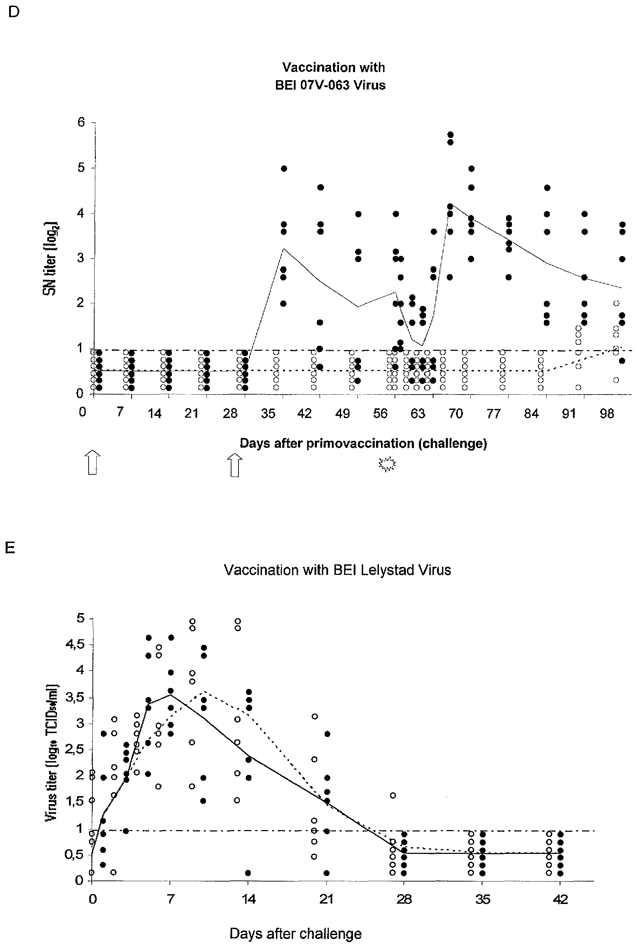

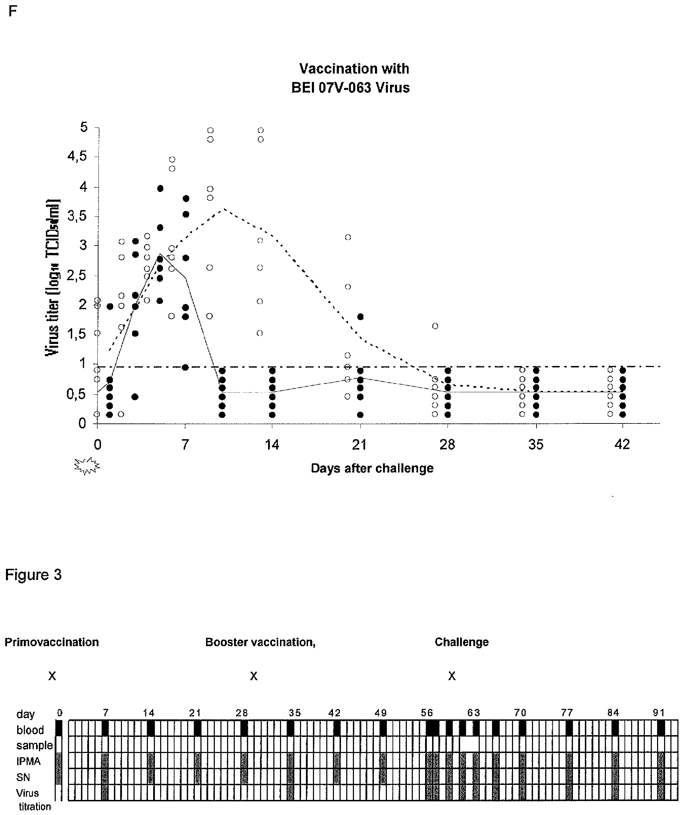

Figure 2: Evolution of antibodies and viremia after vaccination with BEI-inactivated LV and BEI-inactivated 07V-063. Virus specific antibodies were determined using the IPMA test (A and B), virus neutralizing antibodies were determined by an SN test (C and D) and viremia was measured after challenge, by virus titration on macrophages (E and F). The individual values of each group are shown as circles; the lines are showing the average of the group. Closed circles and full lines represent the vaccinated groups, open circles and dashed lines represent the non-vaccinated control. The arrows indicate the time of vaccination (first arrow: primovaccination, second arrow: booster vaccination), the virus like symbol the time of challenge. The straight line parallel to the x-axis indicates the detection limit.
Figure 3: schedule of the vaccination experiment of example 3: time of sampling, with indication of the analyses carried out on the sample (IPMA, SN and virus titration)
Figure 4: Results of the IPMA tests after vaccination with Progressis (A), Porcilis® PRRS (B), Ingelvac® PRRS (C) and BEI-inactivated 07V-063 (D). Symbols are as in Figure 2.
Figure 5: : Results of the SN tests after vaccination with Progressis (A), Porcilis® PRRS (B), Ingelvac® PRRS (C) and BEI-inactivated 07V-063 (D). Symbols are as in Figure 2.
Figure 6: : Results of the viremia determination after vaccination with Progressis (A), Porcilis® PRRS (B), Ingelvac® PRRS (C) and BEI-inactivated 07V-063 (D). Symbols are as in Figure 2.
Figure 7: schedule of the vaccination experiment of example 4: time of sampling, with indication of the analyses carried out on the sample (IPMA, SN and virus titration)
Figure 8: Results of the IPMA tests after vaccination for the control group (A),BEI-inactivated 08V-204 (B), BEI-inactivated 07V-063 (C), Bei-inactivated combi vaccine (D), BEI-inactivated LV (E) and Porcilis® PRRS (F). Symbols are as in Figure 2.
Figure 9: Results of the SN tests after vaccination for the control group (A),BEI-inactivated 08V-204 (B), BEI-inactivated 07V-063 (C), Bei-inactivated combi vaccine (D), BEI-inactivated LV (E) and Porcilis® PRRS (F). Symbols are as in Figure 2.
Figure 10: Results of the viremia titration after vaccination for the control group (A),BEI-inactivated 08V-204 (B), BEI-inactivated 07V-063 (C), Bei-inactivated combi vaccine (D), BEI-inactivated LV (E) and Porcilis® PRRS (F). Symbols are as in Figure 2.

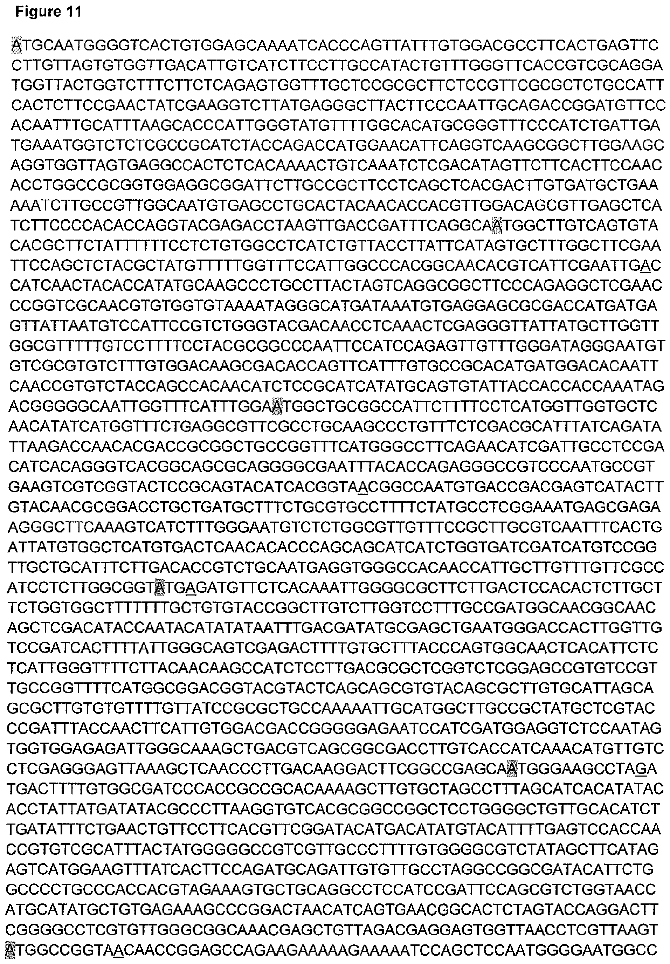

Figure 11: nucleic acid sequence of ORF2-7 for strain 07V-063 (SEQ ID N° 11); Grey is first nucleotide of start codon, underlined is last nucleotide of stop codon
   - ORF2a(1-750) → GP2
   - 0RF3(609-1406) → GP3
   - ORF4(1151-1702) → GP4
   - ORF5(1699-2304) → GP5
   - ORF6(2292-2813) → M
   - ORF7(2803-3189) → N
Figure 12: nucleic acid sequence of ORF2-7 for strain 08V-194 (SEQ ID N° 12); Grey is first nucleotide of start codon, underlined is last nucleotide of stop codon
   - ORF2a(1-750) → GP2
   - ORF3(609-1388) → GP3
   - ORF4(1151-1684) → GP4
   - ORF5(1681-2286) → GP5
   - ORF6(2274-2795) → M
   - ORF7(2785-3171) → N
Figure 13: nucleic acid sequence of ORF2-7 for strain 08V-204 (SEQ ID N° 13); Grey is first nucleotide of start codon, underlined is last nucleotide of stop codon
   - ORF2a(1-750) → GP2
   - ORF3(609-1391) → GP3
   - ORF4(1151-1690) → GP4
   - ORF5(1687-2292) → GP5
   - ORF6(2280-2802) → M
   - ORF7(2791-3178) → N
Figure 14: development of antibodies (as measured by the SN titer; A) and virus titer (B), for 7 non-vaccinated pigs, challenged with the strain 08V-194. The numbers refer to the measurements in the individual pigs, "gem" indicates the average.
Figure 15: development of antibodies (as measured by the SN titer; A) and virus titer (B), for 6 pigs, vaccinated with the inactivated Marc- cell grown strain 08V-194, challenged with the strain 08V-194. The numbers refer to the measurements in the individual pigs, "gem" indicates the average.
Figure 16: development of antibodies (as measured by the SN titer; A) and virus titer (B), for 6 pigs, vaccinated with the inactivated PK15-cell grown strain 08V-194, challenged with the strain 08V-194. The numbers refer to the measurements in the individual pigs, "gem" indicates the average.
Figure 17: development of antibodies (as measured by the SN titer; A) and virus titer (B), for 6 pigs, vaccinated with the inactivated Marc- cell grown strain 07V-063, challenged with the strain 08V-194. The numbers refer to the measurements in the individual pigs, "gem" indicates the average.
Figure 18: development of antibodies (as measured by the SN titer; A) and virus titer (B), for 6 pigs, vaccinated with the inactivated PK15- cell grown strain 07V-063, challenged with the strain 08V-194. The numbers refer to the measurements in the individual pigs, "gem" indicates the average.
Figure 19: development of antibodies (as measured by the SN titer; A) and virus titer (B), for 6 pigs, vaccinated with the naturally attenuated, Marc-cell grown strain 08V-204, challenged with the strain 08V-194. The numbers refer to the measurements in the individual pigs, "gem" indicates the average.

### EXAMPLES

### Example 1: isolation and characterization of the strain 07V-063

The PRRSV strain 07V-063 was isolated from a sow, coming from a pig farm where the animals were vaccinated with an attenuated PRRSV vaccine of the European type 1 virus. Strain 07V-063 was isolated from fetal tissue and cultivated on alveolar pig macrophages. The strain was adapted to Marc-145 cells by repeated passages. The strain was characterized by sequencing; sequence identity of parts of the genome or of the total genome to sequenced European and North American isolates is shown in Table 1. Similarly, PRRSV strain 08V-204 and 08V-194 were isolated from sera of piglets collected at the age of 8 and 14 weeks respectively.

**Table 1: Overview of a number of full length PRRSV sequences and percentage identity with 07V-063**

| **AccNumber** | **Name** | **Ref cited with accession** | **Size (bp)** | **Type** | **% DNA identity (07V063 as reference)** |
|---|---|---|---|---|---|
| GQ914997 | SD1-100 | Zhang et al, unpublished | 15458 | Type II (isolated in China) | 3479-4475: 68% |
| | | | | | 7288-10128: 67% |
| | | | | | 10595-11410: 66% |
| | | | | | 11734-15014: 66% |
| GQ857656 | SX-1 | Zhang et al, unpublished | 15344 | Type II (isolated in China) | 11734-15014: 66% |
| GQ359108 | SD-CXA/2008 | Zhu et al, unpublished | 15358 | Type II (in China) | 180-718: 69% |
| | | | | | 11734-15014: 66% |
| FJ524377 | VR-2332 (mutant) | Han et al, 2008 | 15180 | Type II | 180-704: 68% |
| | | | | | 11734-15014: 66% |
| GQ351601 | BJ0706 | Li et al, unpublished | 15444 | Type II (in China) | 180-718: 69% |
| | | | | | 11734-15017: 66% |
| EU939312 | Jsyx | Zhao et al, unpublished | 15351 | Type II (in China) | 180-718: 68% |
| | | | | | 11734-15014: 66% |
| EU708726 | JX143 | Lv et al, unpublished | 15373 | Type II (in China) | 180-718: 68% |
| | | | | | 11734-15014: 66% |
| NC_001961 | 16244B | Allende et al, unpublished | 15428 | Type II | 180-704: 66% |
| | | | | | 11734-15014: 66% |
| U87392 | VR-2332 | Murtaugh et al, 1995 | 15411 | Type II | 180-704: 68% |
| | | | | | 11734-15014: 66% |
| AY150564 | VR-2332 | Nielsen et al, 2003 | 15451 | Type II | See U87392 |
| FJ536165 | NB/04 | Yun et al, unpublished | 15434 | Type II | 180-718: 69% |
| | | | | | 11734-15014: 66% |
| AF159149 | MLV RespPRRS/Repro | Allende et al, 2000 | 15390 | Type II | 180-704: 68% |
| | | | | | 11734-15014: 66% |
| AF494042 | P129 | Calvert et al, Patent: JP (2000189178/A/1)-A | 15450 | Type II | 193-752: 68% |
| | | | | | 11734-15014: 66% |
| DQ988080 | Ingelvac ATP | Faaberg et al, 2006 | 15413 | Type II | 180-704: 68% |
| | | | | | 11734-15014: 66% |
| AF066183 | respPRRS vaccine | Nelsen et al, | 15412 | Type II | 180-704: 68% |
| | | | | | 11734-15014: 66% |
| EF532801 | IngelvacATP | Murtaugh et al, | 15071 | Type II | See U87392 |
| DQ779791 | PrimePac | Kwon et al, 2006 | 15521 | Type II | 180-704: 68% |
| | | | | | 11750-15014 : 66% |
| AY262352 | HB-2(Sh)/2002 | Gao et al, 2004 | 15398 | Type II (China) | 180-704: 69% |
| | | | | | 11750-15014: 66% |
| FJ950747 | BJSD | Liu et al, 2009 | 15345 | Type II (China) | ND |
| EU880443 | GS2004 | Chen, 2008 | 15423 | Type II (China) | ND |
| EU880437 | HN2007 | Chen, 2008 | 15334 | Type II (China) | ND |
| EF536000 | MN30100 | Murtaugh et al, 2007 | 15207 | Type II | ND |
| GU168569 | 08SDWF | Zhu et al, unpublished | 15345 | Type II (China) | ND |
| | | | | | |
| GU067771 | Amervac | Chen et al, unpublished | 15098 | Type I | 1-2321: 86%; |
| | | | | | 2379-12386: 88% |
| GU047345 | NMEU09-1 | Chen et al, unpublished | 15068 | Type I (isolated in China) | 1-2178: 83%; |
| | | | | | 2580-10491: 84%; |
| | | | | | 6537-15014:86% |
| GU047344 | BJEU06-1 | Chen et al, unpublished | 15059 | Type I (isolated in China) | 1-2288: 84% |
| | | | | | 5727-15014: 87% |
| | | | | | 2374-11298:86% |
| AY366525 | EuroPRRSV | Ropp et al, 2004 | 15428 | Type I | 1-15014: 86% |
| M96262 | LV | Meulenberg et al, 1993 | 15111 | Type I | 1-2321: 87% |
| | | | | | 2379-13100: 89% |
| A268843 | LV | Patent: WO 9221375-A 1 | 15108 | TypeI | 11-2321: 86% |
| | | | | | 2344-15014: 89% |
| GQ461593 | SHE | Zhuang et al, 2008 | 15120 | Type I (in China) | 1-2321: 85% |
| | | | | | 2345-15014: 88% |
| FJ349261 | KNU-07 | Nam et al, 2009 | 15038 | Type I (in Korea) | 1-2327: 83% |
| | | | | | 2435-15014: 85% |
| DQ864705 | 01CB1 | Amonsin et al, unpublished | 14943 | Type I (in Thailand) | 78-2321: 86% |
| | | | | | 2345- 14936: 89% |
| DQ489311 | SD01-08 | Fang et al, 2006 | 15047 | Type I | 1-15014: 86% |
| EU076704 | Hkeu-16 | Li et al, | 15074 | Type I (in Hong Kong) | 1-2270: 84% |
| | | unpublished | | | 2345-15014: 86% |

### Example 2: Vaccination with strain specific vaccine

### Viral inactivation

A 0.1 M stock of BEI was prepared by cyclization of 2-bromoethylamine in 0.175 M NaOH for 1 h at 37 °C and stored at 4 °C. 07V-063 (10⁸ TCID₅₀/ml) as well as LV (WO9221375) (10⁸ TCID₅₀/ml) were inactivated with 1mM binary ethyleneimine (BEI) for 24 hrs at 37°C. Afterwards, BEI was neutralized by incubation with 0.1 mM Na-thiosulphate for 2 h at 37 °C. Complete inactivation was checked by inoculating a bottle of Marc-145 with a complete dose of vaccine (1ml), whereby the Marc-145cells were followed during two blind passages. Detection of infectious virus was carried out by immunostaining of the nucleocapsid of PRRSV. No infectious virus could be detected in the inactivated virus suspension.

### Vaccination

Two groups of 6 piglets each were vaccinated using BEI-inactivated virus; one group was vaccinated with 07V-063 based vaccine, the other with the LV based vaccine. Piglets were vaccinated with a dose of 10⁸ TCID₅₀ in Suvaxin o/w adjuvant. A third control group was only treated with adjuvant. Four weeks after the second vaccination, all piglets were challenged with infectious 07V-063, grown on SPF macrophages. Table 2 gives an overview of the different treatments.

**Table 2. Overview of the different treatments during vaccination.**

| **group** | **# animals** | **Primovaccination (T= 5 weeks)** | **Booster vaccination (T= 9 weeks)** | **Challenge (T= 13 weeks)** |
|---|---|---|---|---|
| A | 6 | Adjuvant control | Adjuvant control | Strain 07V-063 |
| | | | | 10⁶ TCID₅₀/ 2 ml |
| B | 6 | BEI-inactivated | BEI-inactivated | Strain 07V-063 |
| | | 07V-063 | 07V-063 | 10⁶ TCID₅₀/ 2 ml |
| C | 6 | BEI-inactivated | BEI-inactivated | Strain 07V-063 |
| | | LV | LV | 10⁶ TCID₅₀/ 2 ml |

### Results

In all serum samples, the total amount of virus specific antibodies was determined with the IPMA test en the amount of virus neutralizing antibodies was determined with the SN test. As the challenge was carried out using the 07V-063 strain, antibodies against this strain were detected. The amount of infectious virus in the serum was detected by virus titration on macrophages. In none of the animals, virus could be detected one week after the primovaccination and booster vaccination, indicating that no viremia was induced by the vaccination.

In the non-vaccinated control group, no virus-specific antibodies were detected before the challenge (Figure 2A and B). 7 days after the challenge, antibodies could be detected in all animals from the non-vaccinated control group; In the BEI-inactivated LV vaccinated group, virus specific antigens against 07V-063 could be detected from two weeks after the primovaccination; after the booster vaccinations all animals scored positive. 7 days after the challenge, the antibody titer in this group scored significantly higher than in the non-vaccinated control group. In the BEI-inactivated 07V-063 group, in some animals, antibodies could already be detected three weeks after the primovaccination; all animals from this group scored positive after the booster vaccination. 10 days after challenge the antibody titer in this group scored significantly higher than in the non-vaccinated control group (Figure 1A and B).

Virus neutralizing antibodies could not be detected in the non-vaccinated control group till 5 weeks after the challenge. Even 6 weeks after the challenge, not all the animals from this group scored positive. (Figure 2, B and C). In the group, vaccinated with the BEI-inactivated LV, virus neutralizing antibodies against 07V-063 could already be detected after booster vaccination. The antibody titers decreased after the challenge, but increased again 10 days after infection. Some animals scored negative for virus neutralizing antibodies in the period between one week before the challenge and 10 days after the challenge, but after those 10 days, virus neutralizing antibodies could be detected in all animals. In one animal, virus neutralizing antibodies could only be detected 4 weeks after the challenge. The virus neutralizing antibody titer was significantly higher than for the control group.

In the group, vaccinated with the BEI-inactivated 07V-063, virus neutralizing antibodies against 07V-063 could already be detected after booster vaccination. The antibody titers decreased after the challenge, but increased again 10 days after infection. Some animals scored negative for virus neutralizing antibodies in the period between two weeks before the challenge and 10 days after the challenge, but after those 10 days, virus neutralizing antibodies could be detected in all animals. The virus neutralizing antibody titer was significantly higher than for the control group.

In the non vaccinated control group, viremia was detected one day after the challenge, for 3/6 pigs with a maximal average after 10 days. 4 weeks after challenge, all animals of the control groups scored again negative. In the group, vaccinated with BEI-inactivated LV, all animals showed viremia, with a peak on day 7, and the average virus titer was not significantly lower than the control group. 4 weeks after challenge, all animals of the groups scored again negative. In the group, vaccinated with BEI-inactivated 07V-063, all animals showed viremia, but the peak was earlier (day 5) and smaller. 10 days after the challenge, all animals were already virus negative, but one animal showed a positive virus titer on day 21 after challenge. The results are summarized in figure 2, D and E. The results prove that the 07V-063 based vaccine gives a better protection against a homologous challenge than the LV based vaccine.

### Example 3: vaccination with a virus specific vaccine in comparison with commercial vaccines

### Experimental design

5 groups of 6 piglets, coming from a PRRSV negative farm were used in the experiment. At the age of 7 weeks, two groups of 6 pigs were vaccinated intramuscularly in the neck, respectively with the American type attenuated vaccine (Ingelvac® PRRS MLV, Boehringer Ingelheim, 10⁴ TCID₅₀/2ml) and the European type attenuated vaccine (Porcilis® PRRS, Intervet, 10⁴ TCID₅₀/2ml). Two groups of 6 pigs were vaccinated at 5 weeks (primovaccination) and 9 weeks (booster vaccination), one group with the BEI-inactivated 07V-063 vaccine (10⁸ TCID₅₀ /2ml) and the other group with commercial inactivated vaccine (PROGRESSIS, Merial, strain P120, minimum 2,5 log IF units). A control group of 6 piglets received only RPMI in Suvaxyn adjuvant. All pigs were challenged intranasally, 4 weeks after booster vaccination with infectious 07V-063 (10⁶ TCID₅₀) suspended in phosphate buffered saline solution (1ml per nostril). An overview of the vaccination schedule is given in Table 3; the sampling schedule is shown in Figure 3.

### Results

In all serum samples, the total amount of virus specific antibodies was determined with the IPMA test en the amount of virus neutralizing antibodies was determined with the SN test. As the challenge was carried out using the 07V-063 strain, antibodies against this strain were detected. The amount of infectious virus in the serum was detected by virus titration on macrophages. In none of the animals, virus could be detected one week after the primovaccination and booster vaccination, indicating that no viremia was induced by the vaccination.

**Table 3. Overview of the groups in the vaccination experiment**

| **group** | **# animals** | **primovaccination (T= 5 weeks)** | **Booster vaccination (T= 9 weeks)** | **Challenge (T= 13 weeks)** |
|---|---|---|---|---|
| A | 6 | Adjuvant control | Adjuvant control | Strain 07V-063 10⁶ TCID₅₀/ 2 ml |
| B | 6 | BEI-inactivated 07V-063 | BEI-inactivated 07V-063 | Strain 07V-063 10⁶ TCID₅₀/ 2 ml |
| C | 6 | PROGRESS IS (Merial) | PROGRESSIS (Merial) | Strain 07V-063 10⁶ TCID₅₀/ 2 ml |

| **group** | **# animals** | **primovaccination (T= 7 weeks)** | **Booster vaccination** | **Challenge (T= 13 weeks)** |
|---|---|---|---|---|
| D | 6 | Ingelvac® PRRS MLV (Boehringer Ingelheim) | / | Strain 07V-063 10⁶ TCID₅₀/ 2 ml |
| E | 6 | Porcilis® PRRS (Intervet) | / | Strain 07V-063 10⁶ TCID₅₀/ 2 ml |

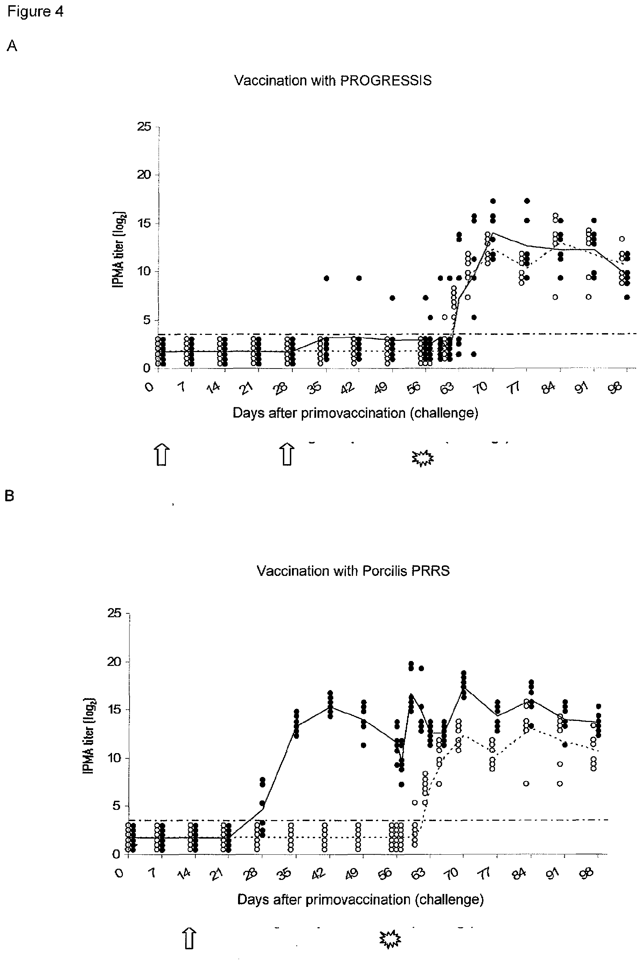

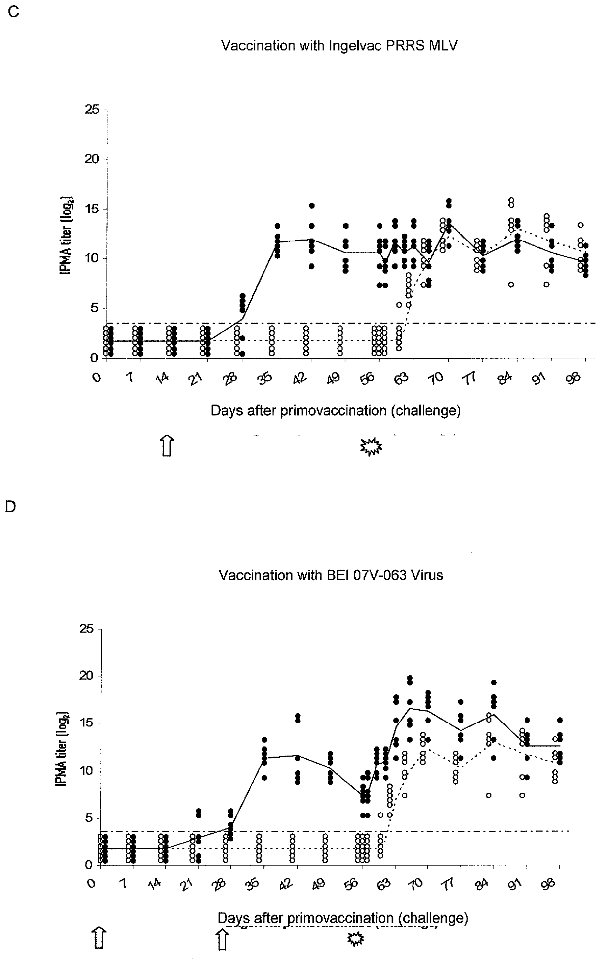

In the non-vaccinated control group, no virus-specific antibodies were detected before the challenge (Figure 4 A-D). 7 days after the challenge, antibodies could be detected in all animals from the non-vaccinated control group; 1 A and B). In the group vaccinated with Progressis, only one animal showed virus specific antibodies one week after the booster vaccination, and only after challenge, all animals scored positive (Figure 4 A). However, after challenge, the antibody titer of this group was not significantly higher than the titer of the non-vaccinated control group. In the group, vaccinated with a single shot of Porcilis® PRRS, two weeks after vaccination, virus specific antibodies could be detected in 3 out of the 6 pigs (Figure 4B). The virus specific antibodies could be detected till the end of the experiment, and the titer after challenge was significantly higher than for the non-vaccinated control group. In the group, vaccinated with a single shot of Inglevac® PRRS, two weeks after vaccination, virus specific antibodies could be detected in 3 out of the 6 pigs (Figure 4C). The virus specific antibodies could be detected till the end of the experiment, but 10 days after challenge the titer wasn't significantly higher anymore than for the non-vaccinated control group. In the group vaccinated with BEI-inactivated 07V-063, for a number of animals, virus specific antibodies could already be detected three weeks after primovaccination; after booster vaccination, all the animals of this group scored positive. From 10 days after the challenge the antibody titer in this group was significantly higher than for the non-vaccinated control group (Figure 4 D).

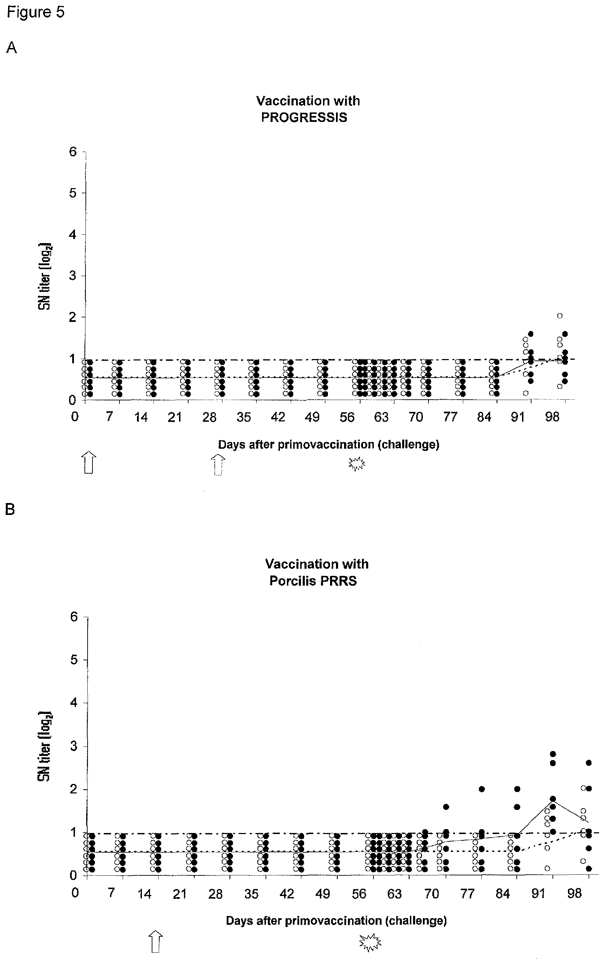

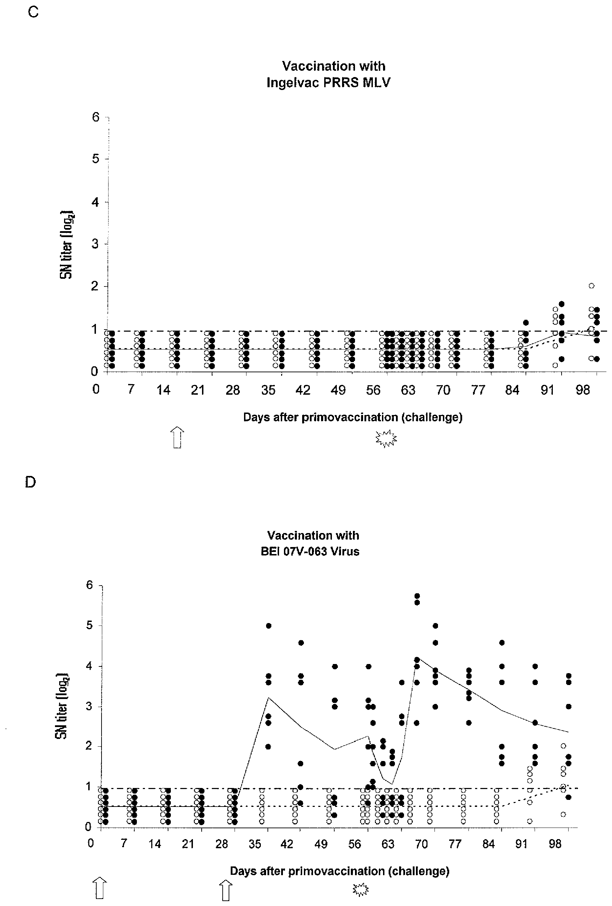

In the non-vaccinated group, virus neutralizing antibodies could only be detected 5 weeks after the challenge, and even 6 weeks after the challenge, not all animals scored positive (Figure 5, A-D). The groups vaccinated with Progressis (two shots), Porcilis® PRRS (single shot) and Inglevac® PRRS (single shot) showed a similar evolution as for the non-vaccinated control group, with the exception of the Porcilis® vaccinated group were a remarkable increase of virus neutralizing antibodies was noticed 4 weeks after the challenge (Figure 5, A-C). In the group, vaccinated with the BEI inactivated 07V-063, virus neutralizing antibodies could already be detected after booster vaccination. The average antibody titer was decreasing directly after the challenge, but increased again 10 days after the challenge (Figure 5 D). Some animals scored negative for virus neutralizing antibodies in the period from 2 weeks before challenge till 10 days after challenge, but after 10 days, all animals scored positive. The virus neutralizing antibody titer was significantly higher, compared with the non-vaccinated control as well in comparison with the other groups.

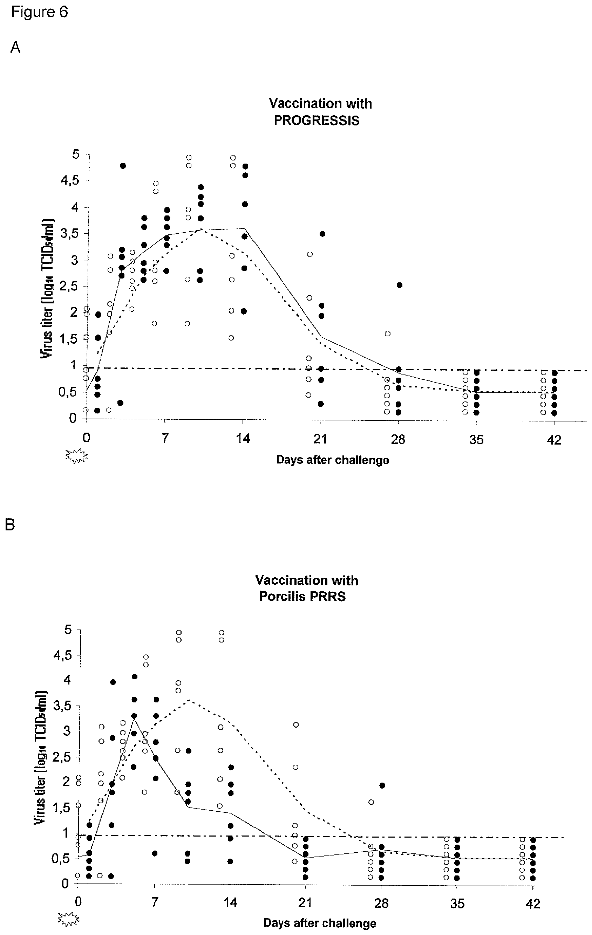

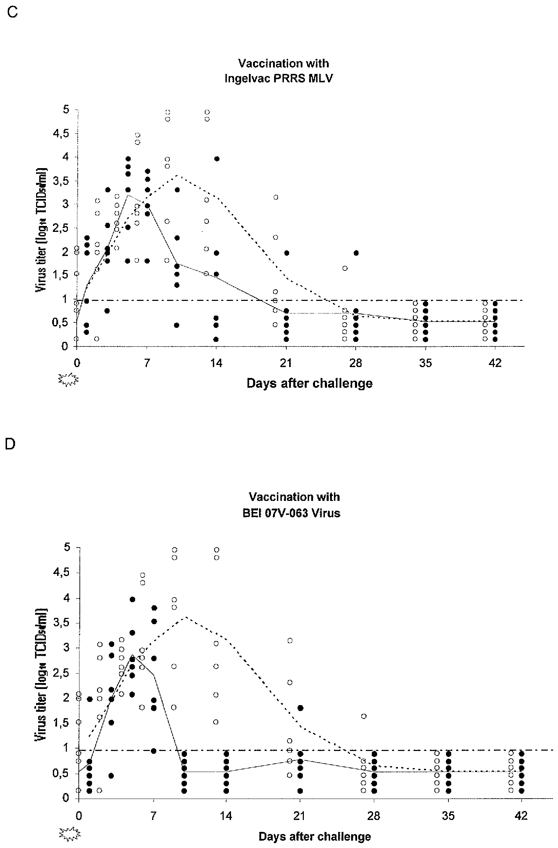

In the non-vaccinated group, viremia was detected from day 1 after the challenge (3 pigs out of 6), and a viremia peak in average after 10 days after challenge. 4 weeks after challenge, all animals scored again negative (Figure 6, A-D). In the group, vaccinated with Progressis, viremia was detected in all animals, with a peak around 10-14 days after challenge; the average viremia value was not reduced in comparison with the control group. 5 weeks after challenge, all animals of this group scored again negative (Figure 6, A). The two groups vaccinated with the commercial PRRS vaccines showed a partial reduction in viremia, with an average peak on day 5 after challenge (Figure 6, B and C). Only 5 weeks after challenge, all animals of these groups were consistent virus negative. In the group vaccinated with the BEI inactivated 07V-063, all animals showed some viremia (small average peak on day 5 after challenge), but 10 days after challenge, all the animals of this group were virus negative (Figure 6 D).

This experiment proves that the BEI inactivated 07V-063 gives a better protection against a homologous challenge than the commercial vaccines tested.

### Example 4: Homologous versus heterologous BEI-inactivated PRRSV vaccine

### Strains

The isolation of strain 07V-063 is described in example 1. The PRRSV strain 08V-204 was isolated from a sow, coming from a pig farm where the animals were vaccinated with an attenuated PRRSV vaccine of the European type 1 virus. Strain 08V-204 was isolated from sera form 4 week old piglets and cultivated on alveolar pig macrophages. The strain was adapted to Marc-145 cells by repeated passages. The strain was characterized by sequencing and is clearly different form both strain 07V-063 and strain LV.

### Virus growth, purification and vaccine production

PRRSV strain 07V-063, 08V-204 and LV were grown in MARC-145 cells, when used for vaccine production. The virus was purified by ultracentrifugation. PRRSV strain 08V-204 was grown on SPF macrophages when used for challenge.

### Vaccine production was carried out as described in example 2

### Experimental design

4 groups of 6 piglets ware vaccinated two times using a BEI-inactivated PRRSV vaccine. One group was vaccinated with BEI-inactivated LV, one group with BEI-inactivated 07V-063, one group with BEI-inactivated 08V-204 and one with a BEI inactivated combi-vaccine, consisting of equal amounts of 08V-204 and 07V-063. A fifth group was only injected with adjuvant and was used as control group. The last group was vaccinated once using the commercial European attenuated vaccine (Porcilis® PRRSV, Intervet). 4 weeks after the booster vaccination, all piglets were challenged with infectious 08V-204. The vaccination schedule is summarized in Table 4.

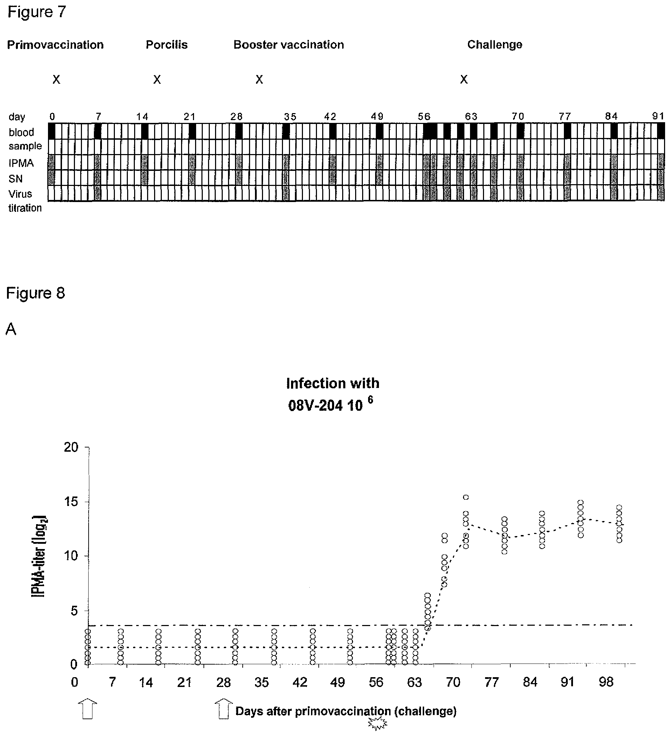

At different time steps, blood samples were taken for determination of virus specific antibodies (IPMA test), virus neutralizing antibodies (SN test) and viremia (virus titration on macrophages. Figure 7 gives a schematic overview of the sampling

### Results

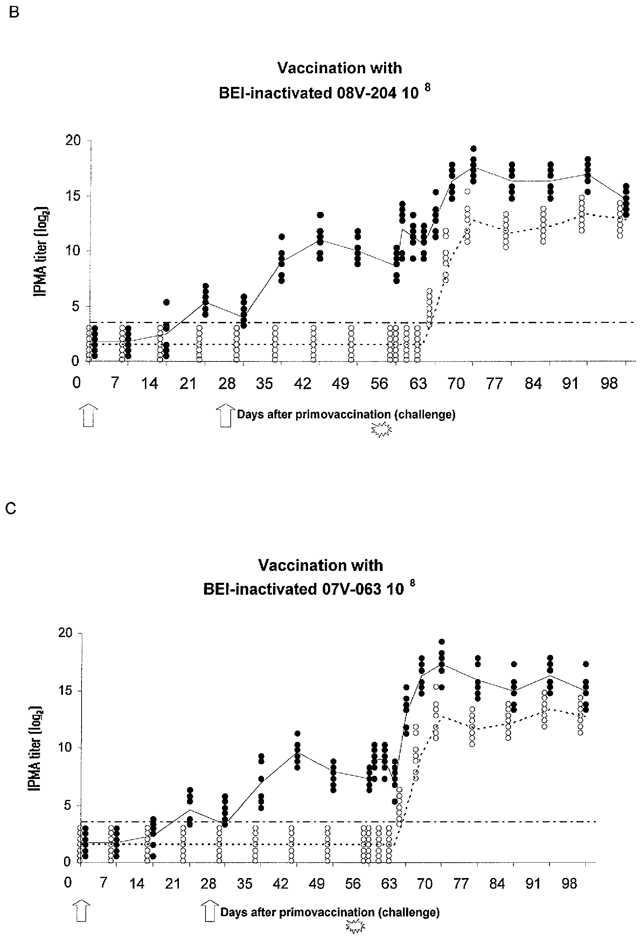

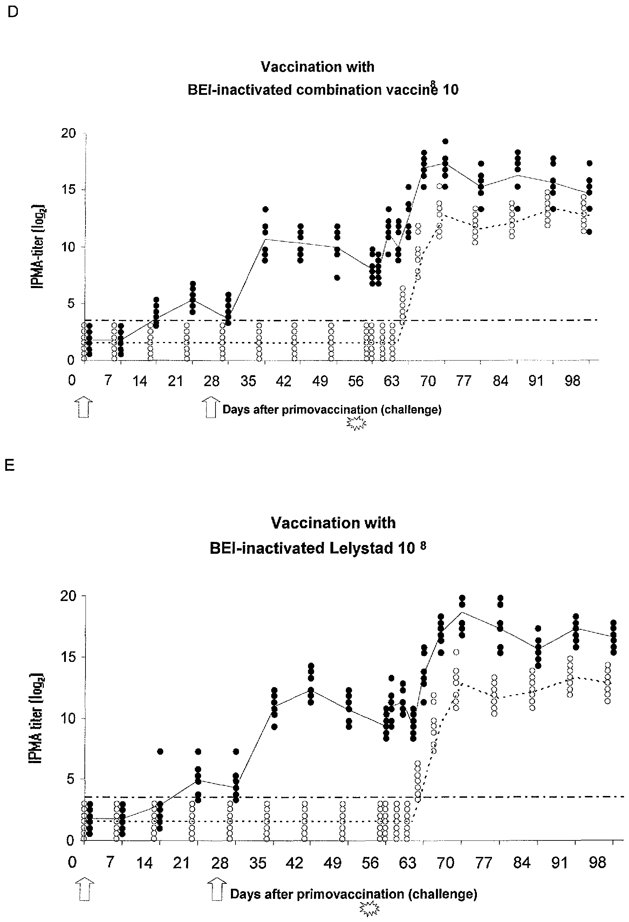

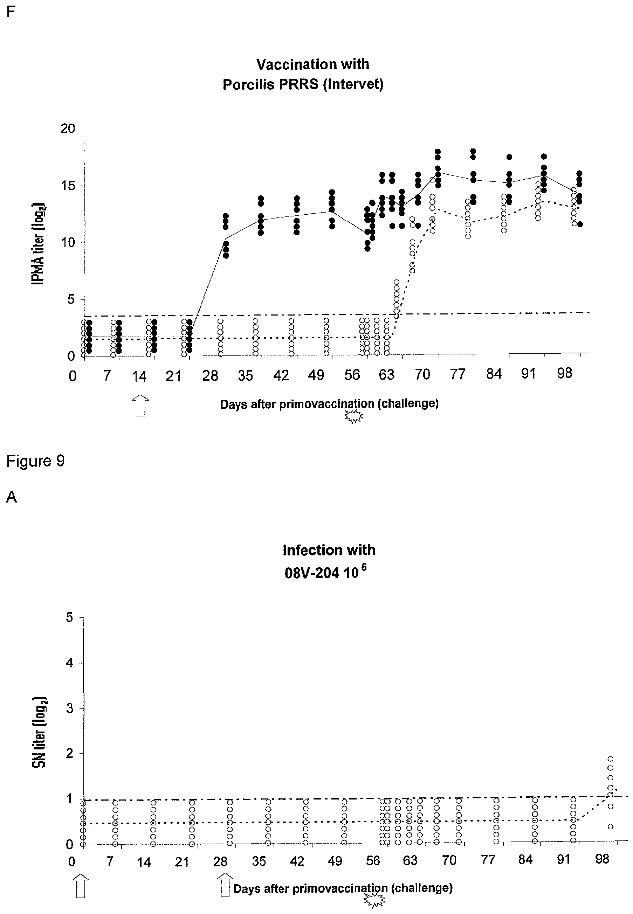

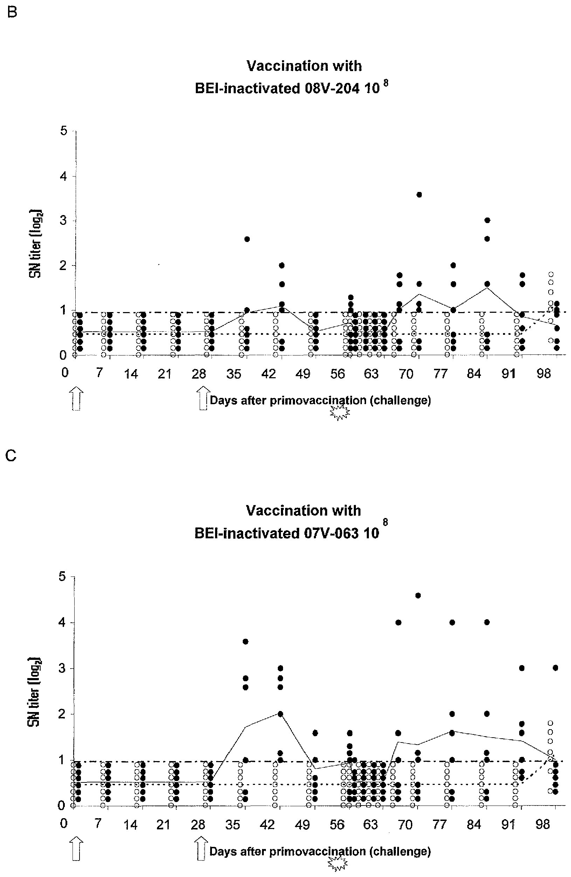

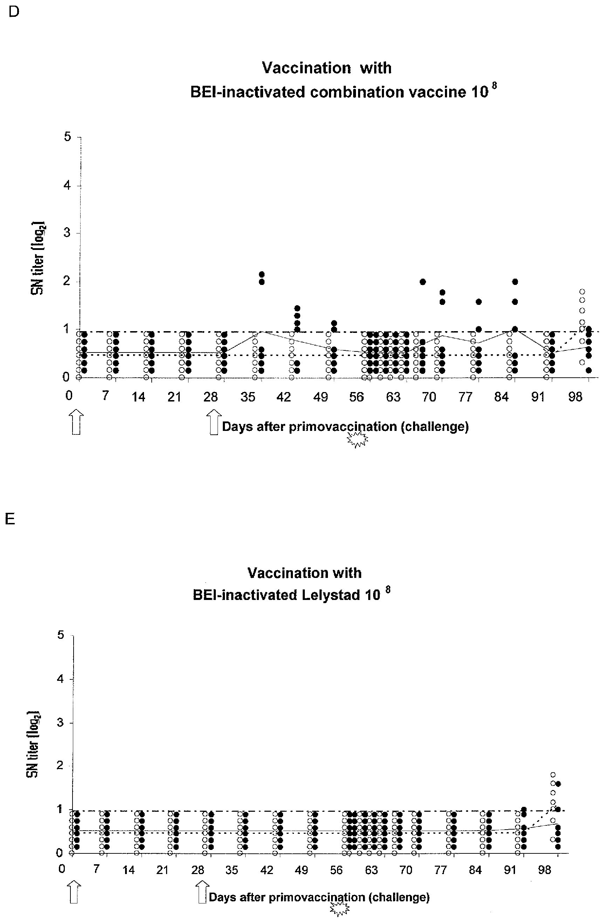

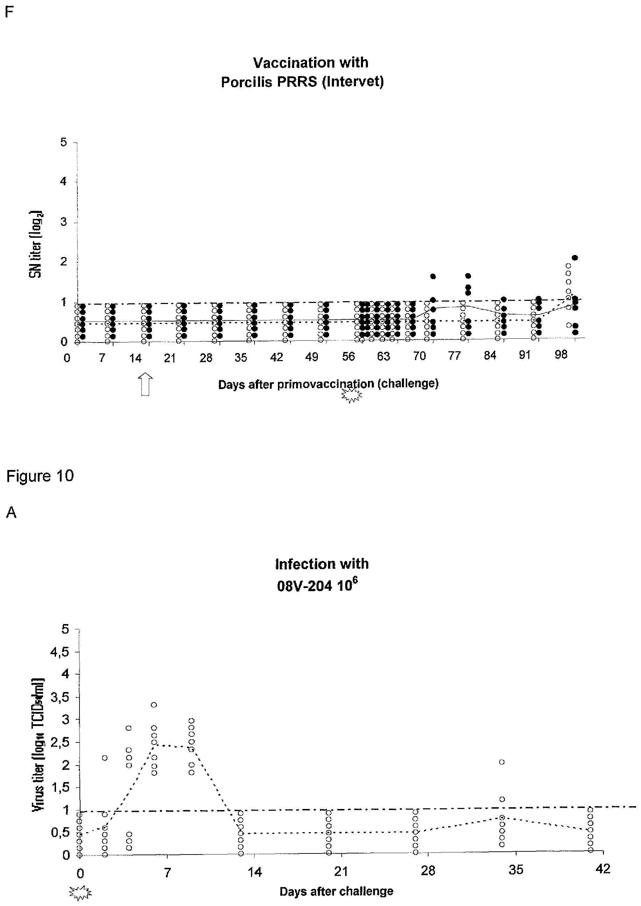

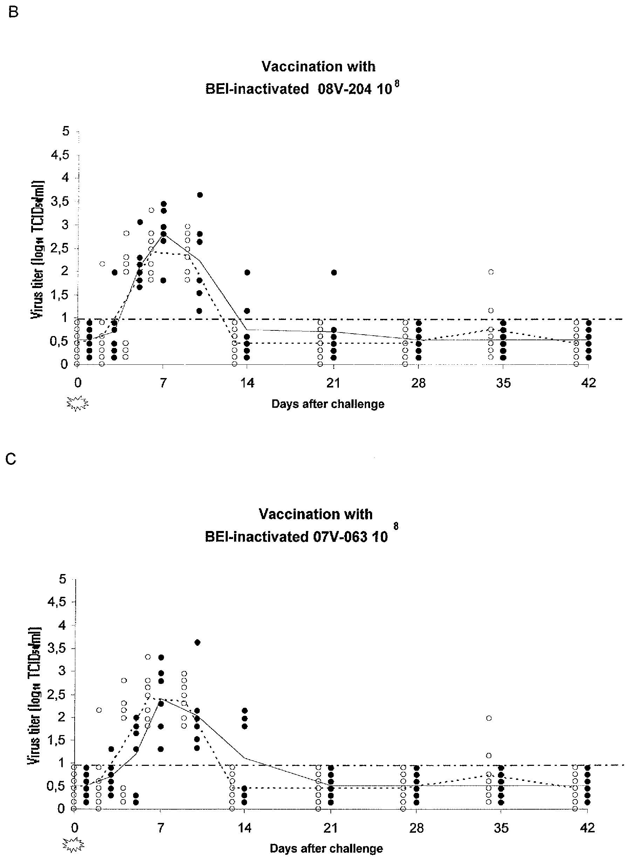

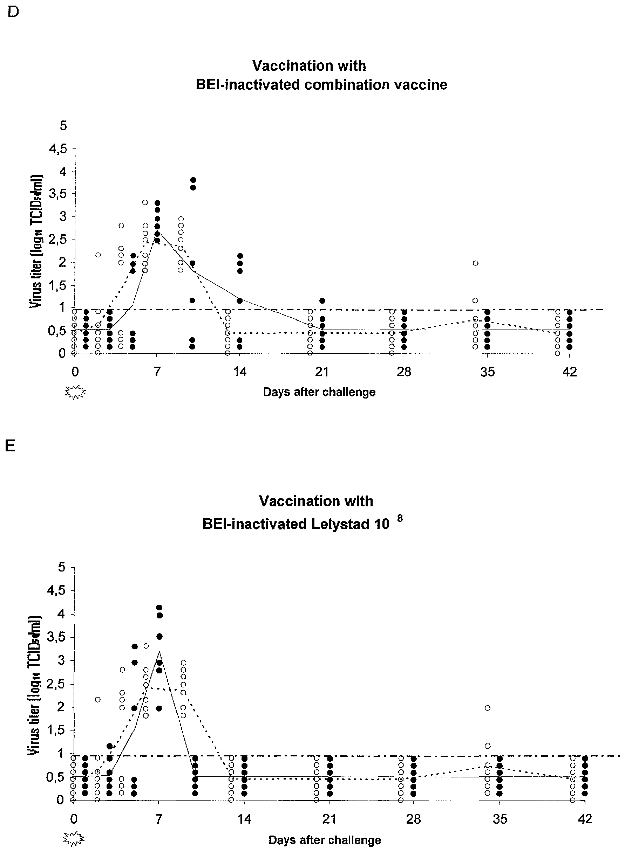

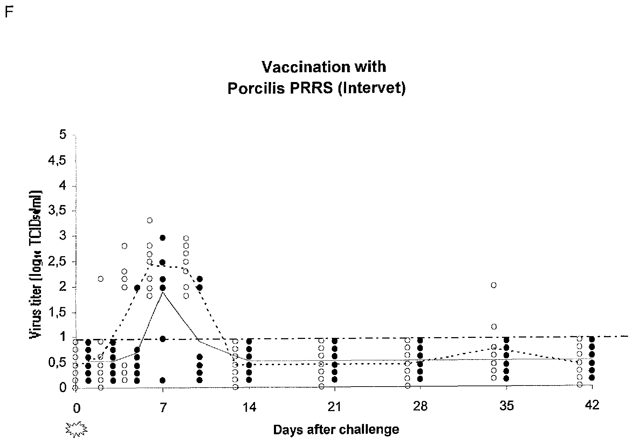

In all serum samples, the total amount of virus specific antibodies was determined using the IPMA test and the amount of virus neutralizing antibodies using the SN test. Due to the fact that the challenge was carried out with strain 08V-204, antibodies against this strain were specifically detected. At different time steps after the challenge, the amount of infectious virus was determined using virus titration on macrophages. 1 week after primovaccination and booster vaccination, a virus titration was carried out, showing that in none of the animals, viremia was induced by the vaccination. Only in one animal of the Porcilis® group, virus could be detected two weeks after vaccination. The results of the virus specific antibodies are shown in Figure 8, those of the virus neutralizing antibodies in figure 9 and the evolution of viremia is shown in figure 10.

**Table 4: vaccination schedule of example 4**

| **Group** | **# animals** | **Vaccine** | **Application** |
|---|---|---|---|
| A | 7 | Adjuvant control | 2X, 4 weeks in between (age: 5 and 9 weeks) |
| B | 6 | Inactivated LV | 2X, 4 weeks in between (age: 5 and 9 weeks) |
| C | 6 | Inactivated 07V-063 | 2X, 4 weeks in between (age: 5 and 9 weeks) |
| D | 6 | Inactivated 08V-204 | 2X, 4 weeks in between (age: 5 and 9 weeks) |
| E | 6 | Combivaccin 08V-204 / 07V-063 | 2X, 4 weeks in between (age: 5 and 9 weeks) |
| F | 6 | Porcilis® PRRS (Intervet) | 1X, 6 weeks before challenge (age: 7 weeks) |

In the non-vaccinated control group, no specific antibodies against 08V-204 could be detected before the challenge. On day 7 after the challenge, IPMA antibodies could be detected in 6 out of 7 animals. In the group, vaccinated with a single shot of Porcilis® PRRS, virus specific antibodies could be detected in all animals, 2 weeks after the challenge. The virus specific antibodies remained detectable till the end of the test; after challenge, the antibody titer was not significantly higher than for the non-vaccinated control group (p<0,0001). In the groups vaccinated with the BEI-inactivated vaccines 08V-204, 07V-063, LV and the combivaccine, virus specific antibodies could already be detected after primovaccination. After booster vaccination, all animals scored positive till the end of the experiment. In comparison with the non-vaccinated control group, a significant difference in antibody titer was found for the four groups vaccinated with BEI-inactivated vaccines (Figure 8).

In the non-vaccinated control group, virus neutralizing antibodies could only be detected from 6 weeks after the challenge, in 5 out of 7 animals. The group, vaccinated with the BEI-inactivated LV showed a similar evolution as the non-vaccinated control group, whereby virus neutralizing antibodies could be detected in 2 out of 6 animals, 6 weeks after challenge. In the group vaccinated with the Porcilis® PRRS (single shot) virus neutralizing antibodies could already be detected 2 weeks after challenge. However, statistical analysis showed that there was no significant difference with the non-vaccinated control group. In the groups vaccinated with the BEI-inactivated 08V-204, 07V-063 and combivaccine, virus neutralizing antibodies could already be detected after booster vaccination (Significantly different with the control group, P< 0,001). The average antibody titer decreased below the detection limit, directly after challenge, but increased again 10 days after challenge. In these groups, the largest variability in titer between the individual animals was seen; some animals showed a very high SN titer, whereas in some others no antibodies could be detected. The virus neutralizing antibody titers in the groups vaccinated with BEI-inactivated 08V-204 and 07V-063 was significantly different in comparison with all other groups (Figure 9).

After challenge, the evolution of rectal temperature was followed during 14 days for all piglets. In all groups, fever development was noted. In the non-vaccinated control group, viremia was detected 3 days after challenge (in one out of 6 pigs), with an average peak after 7 till 10 days. Two weeks after challenge, all animals scored again negative. As shown in Figure 10, the viremia in the Porcilis® PRRS vaccinated group is very short, with an average peak at day 7 after challenge. However, statistical analysis proved that there was no significant difference with the non-vaccinated control group. In the group vaccinated with BEI-inactivated LV, only on day 7 after challenge, viremia could be detected in all animals, but the average viremia was not reduced in comparison with the control group. 10 days after challenge, all animals of this group scored again negative. In the groups, vaccinated with the BEI-inactivated 08V-204, 07V-063 and combivaccine, no reduction in viremia could be demonstrated. Three weeks after challenge, 1 out of 6 pigs scored again positive in the 08V-204 vaccinated and the combivaccine vaccinated groups. 4 weeks after challenge, all animals in the three groups scored negative. (Figure 10)

### Example 5: comparison of the inactivated vaccine with commercial vaccines in infection immune sows

### Animals

Twenty four retired sows are purchased from a PRRSV-positive farm where 07V-063 is the circulating PRRSV-strain. The sows are divided into five groups (designated groups A, B, C, D and E) and are housed in a stable according to European guidelines. They are given meal and water *ad libitum.*

### Vaccines

The PRRSV vaccines that are used are the attenuated European virus strain (Porcilis® PRRS, Intervet, 10⁴ TCID₅₀/ 2 ml), the attenuated American virus strain (Ingelvac® PRRS MLV, Boehringer Ingelheim, 10^{4,9}TCID₅₀/ 2 ml) the inactivated European virus strain (PROGRESSIS, Merial, strain P120: min 2,5 log IF units) and the inactivated vaccine based on the PRRSV-strain 07V-063.

### Vaccination

One week after arrival, the sows of groups B, C, D and E (each group n= 5 sows) are vaccinated intramuscularly in the neck respectively with the experimentally developed auto vaccine, the European attenuated virus strain. The sows of group A (n= 4 sows) are kept unvaccinated and will serve as control pigs.

| **group** | **# sows** | **Vaccination** |
|---|---|---|
| A | 4 | Adjuvant control |
| B | 5 | Inactivated PRRSV Titer: 10⁸ TCID₅₀/ 2 ml |
| C | 5 | Porcilis® PRRS, Intervet |
| D | 5 | Ingelvac® PRRS MLV, Boehringer Ingelheim |
| E | 5 | PROGRESSIS, Merial |

### Clinical observations and sampling

After vaccination the sows are monitored clinically by visual inspection. The place of vaccination is examined for local inflammation. Blood samples by jugular venipuncture are taken at 0, 1, 2 and 3 weeks after vaccination.

### Determination antibody titers: IPMA-test

IPMAs with MARC-145 cells is set up to determine the PRRSV-specific antibodies. MARC-145 cells are seeded in 96-well cell culture plates, are inoculated with 50 µl of a European PRRSV-isolate (07V-063) and incubated for 18 h (37 °C, 5% CO₂). Then the culture medium is removed and cells are washed in PBS and dried at 37 °C for 1 h. The plates are kept at -70 °C. The plates are thawed and then fixed in 4% paraformaldehyde for 10 min. The paraformaldehyde is removed; the cells are washed twice with PBS. A serial fourfold dilutions of the sera is added. Sera will be incubated for 1 h at 37 °C. Plates are washed three times with PBS and 50 µl of 1/250 secondary antibody rabbit anti swine is added per well and incubated at 37 °C for 1 h. Plates are washed three times and 50 µl of a substrate solution (250 µl AEC, 5 ml acetate buffer and 4 µl H₂O₂ (30%) a plate) is added to each well, followed by incubation at room temperature for 20 min. Then the reaction is blocked by replacing the substrate by acetate buffer (100 µl/ well) and the results are determined by examination with a microscope.

### Determination of neutralizing antibody titers: SN-test

Serum samples are heat-inactivated at 56 °C for 30 minutes and are twofold serially diluted, starting at a 1:2 dilution. The diluent is minimum essential medium, containing 0,5% gentamycine, 1% glutamine, 1% P/S and 5% FCS. To each dilution, 100 TCID₅₀/ 50 µl PRRSV (07V-063) will be added. After mixing, the plates are incubated at 37 °C for 1 h and 50 µl of the mixture is placed on confluent monolayer of MARC-145 cells in 96-well plates. The cells are propagated in the same medium used to dilute the sera. Cells are observed 7 days after inoculation and the neutralization titer of the sera is recorded as the reciprocal of the highest dilution in which no cytopathic effect is observed.

In all tests, the inactivated vaccine based on the PRRSV-strain 07V-063 scores significantly better than the commercial vaccines.

### Example 6: comparison of vaccine grown on different cells in a homologous challenge

To compare if a vaccine based on strain 07V-063 grown on PK-15^{Sn-CD163} cells gives the same good results as the vaccine based on strain 07V-063 grown on MARC-cells against a homologous challenge with this strain, following experiment is set up:

### Animals

Eighteen pigs were purchased from a PRRSV-seronegative farm at the age of 4 weeks. The pigs were divided into three groups (designated groups A, B and C) and are housed in an isolation stable with an infrared lamp. Blood was taken by jugular venipuncture at the moment of arrival to control if the piglets were PRRSV-negative by means of an IPMA-test. They have been given meal and water *ad libitum.*

### Vaccines and challenge virus

The PRRSV vaccines that were used are two, in our laboratory of Virology, experimental developed inactivated vaccines, one based on the challenge strain grown on Marc-cells and one based on the challenge strain grown on PK-15^{Sn-CD163} cells. A second passage stock of this European PRRSV-isolate (07V-063) made on SPF macrophages was used at a concentration of 10⁶ TCID₅₀/2 ml. The challenge virus was obtained from stillborn pigs from an outbreak in a vaccinated (Porcilis® PRRS, Intervet) swine herd.

### Vaccination and inoculation

Group A (n= 6 pigs), B (n=6 pigs) and C (n= 6 pigs) were vaccinated twice, at 5 and 9 weeks of age, respectively with an experimental developed inactivated auto vaccine, based on a new procedure in our laboratory of Virology and the commercial inactivated virus strain. The pigs of group A (n= 6 pigs) were kept unvaccinated and served as control pigs.

Eighteen pigs were inoculated intranasally eight weeks after primovaccination with 10⁶ TCID₅₀ European isolate (07V-063) virus in 2 ml phosphate-buffered saline (PBS) (1 ml in each nostril).

In all cases, no difference could be detected between the vaccine grown on Marc-145cells and the vaccine grown on PK-15^{Sn-CD163} cells. Moreover, a good protection against the homologous challenge is obtained.

| **Group** | **Pigs** | **Vaccine** | **Way of administration** |
|---|---|---|---|
| A | 6 | Adjuvans controle | 2X with 4 weeks in between (age: 5 and 9 weeks) |
| B | 6 | inactivated 07V-063 MARC-cel grown | 2X with 4 weeks in between (age: 5 and 9 weeks) |
| C | 6 | inactivated 07V-063 PK1 5^{Sn-CD163} grown | 2X with 4 weeks in between (age: 5 and 9 weeks) |

### Example 7: comparison of vaccine grown on different cells in a heterologous challenge

To compare if a vaccine based on strain 07V-063 or strain 08V194 (grown on PK-15^{Sn-CD163} cells and grown on MARC-cells) gives the same good results against a heterologous challenge as in the homologous situation with strain 07V-063, following experiment was set up:

### Animals

Thirty one pigs were purchased from a PRRSV-seronegative farm at the age of 4 weeks. The pigs are divided into seven groups (designated groups A, B, C, D, and E,) and are housed in an isolation stable with an infrared lamp. Blood was taken by jugular venipuncture at the moment of arrival to control if the piglets are PRRSV-negative by means of an IPMA-test. They are given meal and water *ad libitum.*

### Vaccines and challenge virus

The PRRSV vaccines that were used are four, in our laboratory of Virology, experimental developed inactivated vaccines, two based on the challenge strain (08V-194) one grown on Marc-cells and one grown on PK-15^{Sn-CD163} cells and two based on the heterologous strain (07V-063) one grown on Marc-cells and one grown on PK-15^{Sn-CD163} cells.
A second passage stock of the European PRRSV-isolate (08V-194) made on conventional macrophages was used at a concentration of 10⁶ TCID₅₀/2 ml. The challenge virus was obtained from serum of a 14-week old pig of a farm with PRRSV-related outbreaks in the swine herd.

### Vaccination and inoculation

Four groups (each group n= 6 pigs) were vaccinated twice, at 5 and 9 weeks of age. One group (n= 6 pigs) was vaccinated at 7 weeks of age (attenuated vaccine). The pigs of group A (n= 7 pigs) were kept unvaccinated and serve as control pigs. Forty two pigs were inoculated intranasally eight or six weeks after (primo-) vaccination with 10⁶ TCID₅₀ European isolate (08V-194) virus in 2 ml phosphate-buffered saline (PBS) (1 ml in each nostril).

| **Group** | **Pigs** | **Vaccine** | **Way of administration** |
|---|---|---|---|
| A | 7 | Adjuvans controle | 2X with 4 weeks in between (age: 5 and 9 weeks) |
| B | 6 | Inactivated 08V-194 MARC-cel grown | 2X with 4 weeks in between (age: 5 and 9 weeks) |
| C | 6 | Inactivated 08V-194 PK15Sn-CD163 grown | 2X with 4 weeks in between (age: 5 and 9 weeks) |
| D | 6 | Inactivated 07V-063 Marc-cel grown | 2X with 4 weeks in between (age: 5 and 9 weeks) |
| E | 6 | Inactivated 07V-063 PK15Sn-CD163 grown | 2X with 4 weeks in between (age: 5 and 9 weeks) |

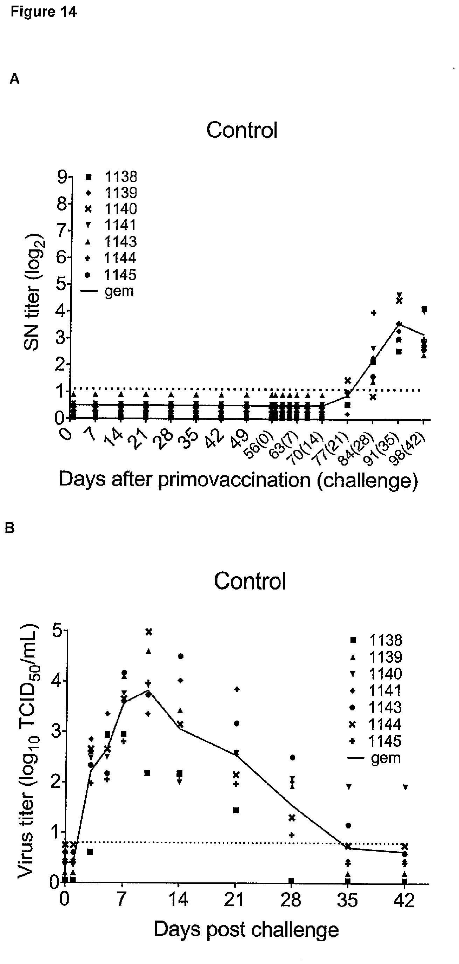

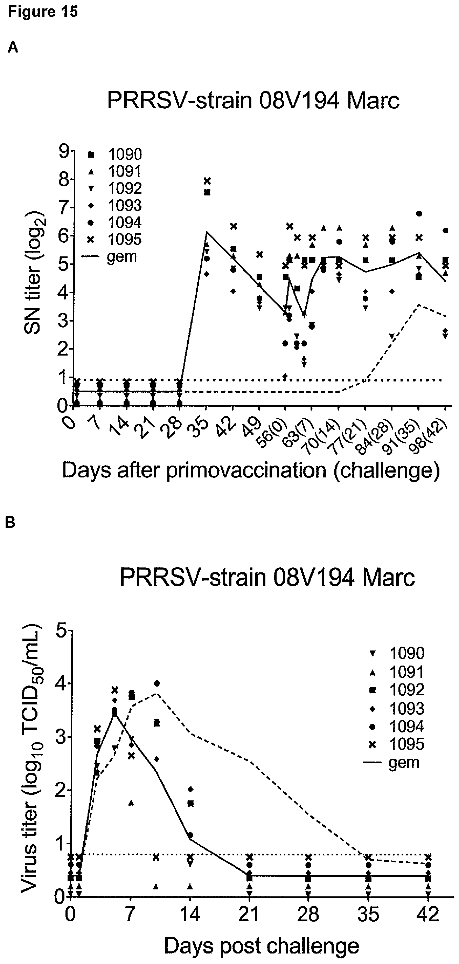

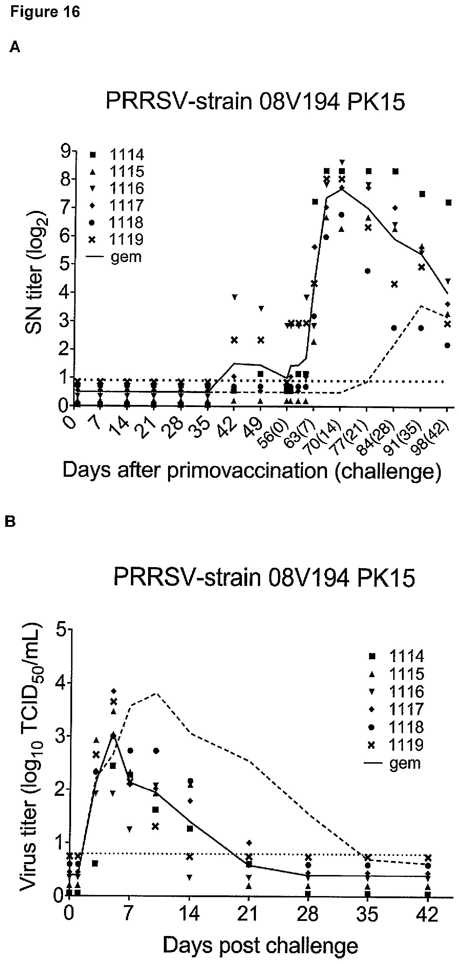

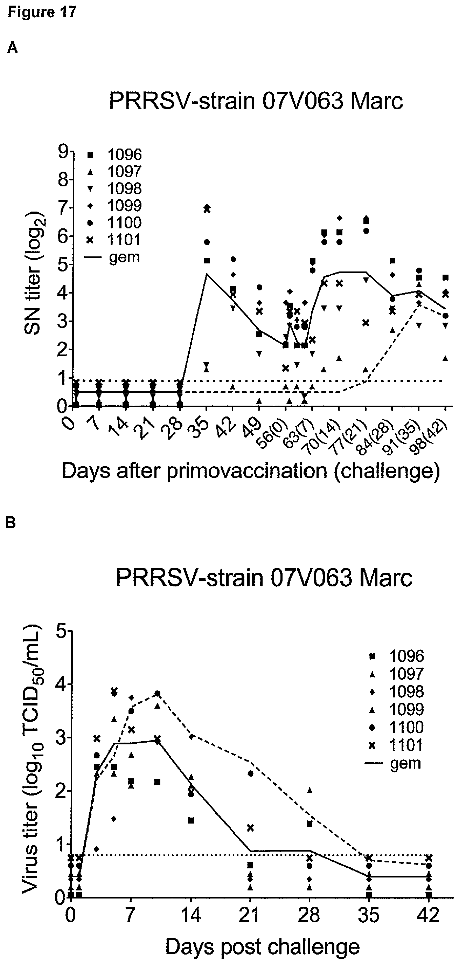

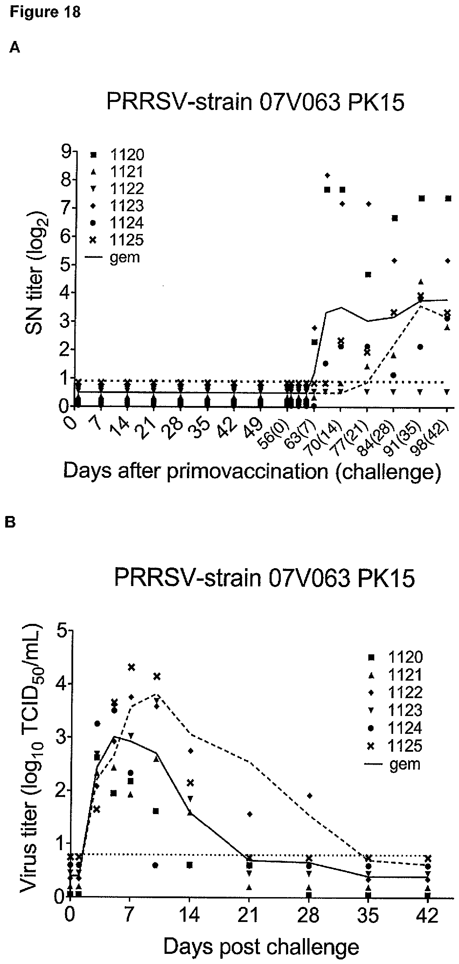

No significant difference was found between the vaccine grown on Marc-145cells and the vaccine grown on PK-15^{Sn-CD163} cells. Moreover, by using a 07V-063 based vaccine, a good protection was obtained against a heterologous challenge by 08V-194. The results are summarized in the figures 14-18.

### Example 8: Efficacy of a naturally attenuated PRRSV strain used as vaccine

To investigate if a vaccine based on strain 08V-204 (grown on MARC-cells or PK-15^{Sn-CD163} cells) provides sufficient protection against a heterologous challenge, the following experiment was set up:

### Animals

Twelve pigs are purchased from a PRRSV-seronegative farm at the age of 4 weeks. The pigs were divided into two groups (designated groups A and B, each group n=6) and were housed in an isolation stable with an infrared lamp. Blood was taken by jugular venipuncture at the moment of arrival to control if the piglets are PRRSV-negative by means of an IPMA-test. They were given meal and water *ad libitum.*

### Vaccines and challenge virus

The PRRSV vaccine that was used is the naturally attenuated PRRSV strain 08V204, grown on Marc-cells. A second passage stock of the European PRRSV-isolate (08V-194) made on SPF macrophages was used at a concentration of 10⁶ TCID₅₀/2 ml as challenge virus.

### Vaccination and inoculation

One group was vaccinated once, at 5 weeks of age, by intramuscular injection of 2 ml 08V-204 virus suspension (10⁵ TCID₅₀/ml) diluted in phosphate-buffered saline (PBS).. One group was kept unvaccinated and serves as control pigs. All pigs were inoculated intranasally eight or six weeks after (primo-) vaccination with 10⁶ TCID₅₀ European isolate (08V-194) virus in 2 ml phosphate-buffered saline (PBS) (1 ml in each nostril).

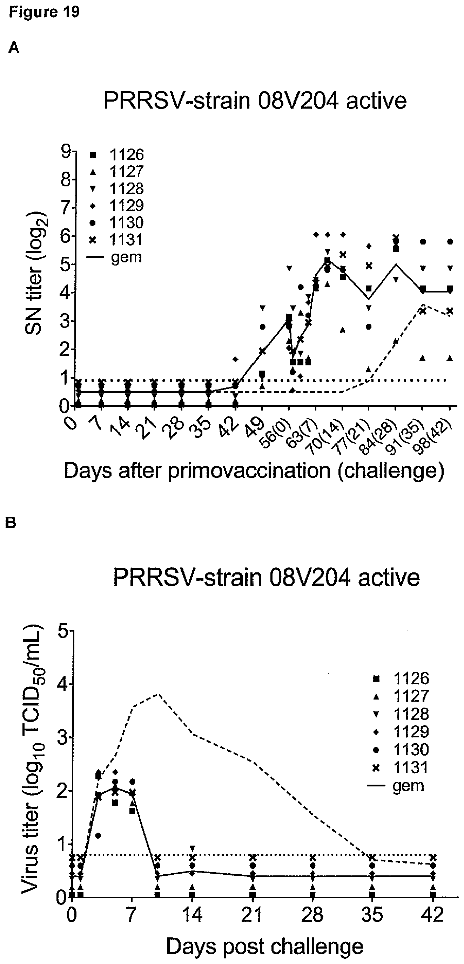

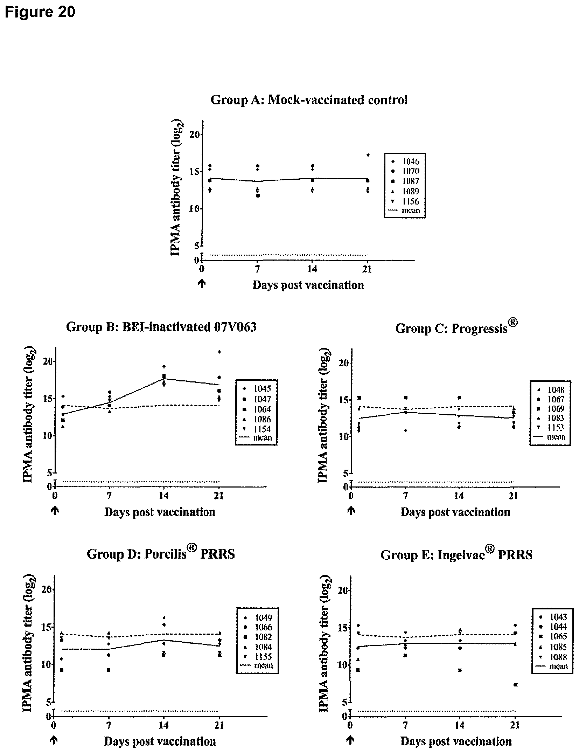

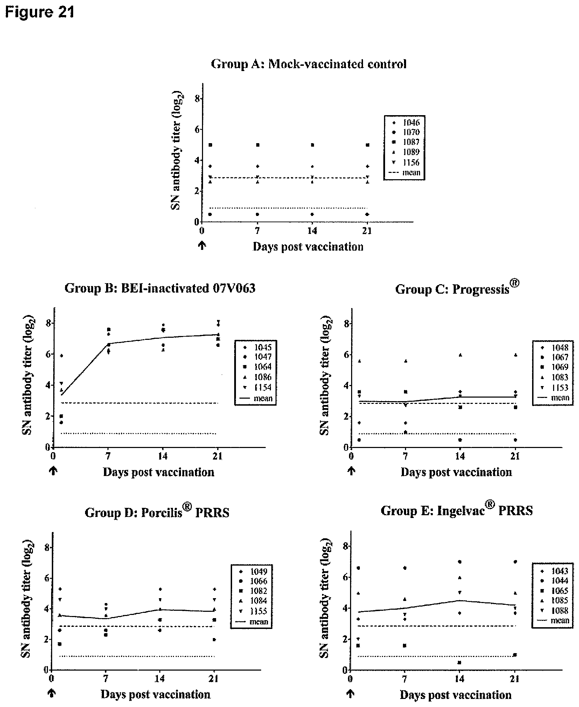

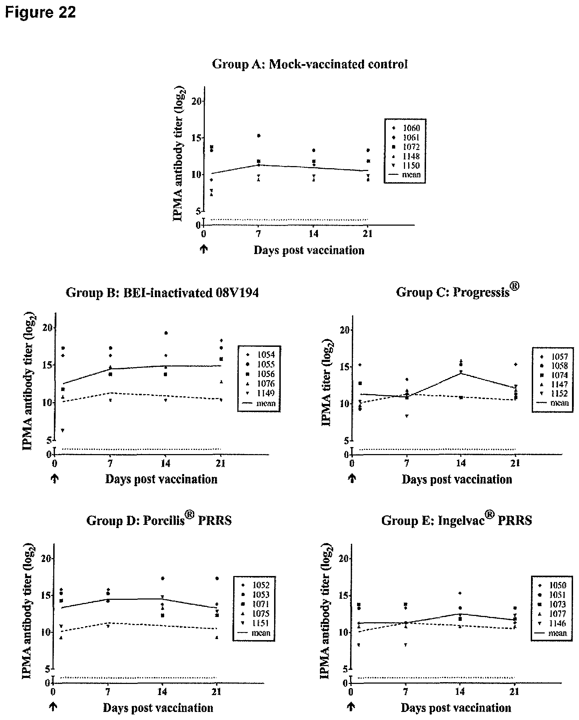

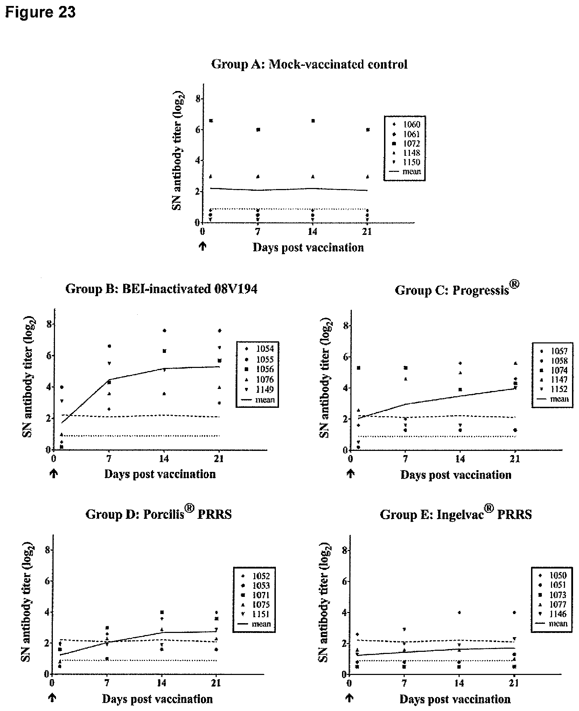

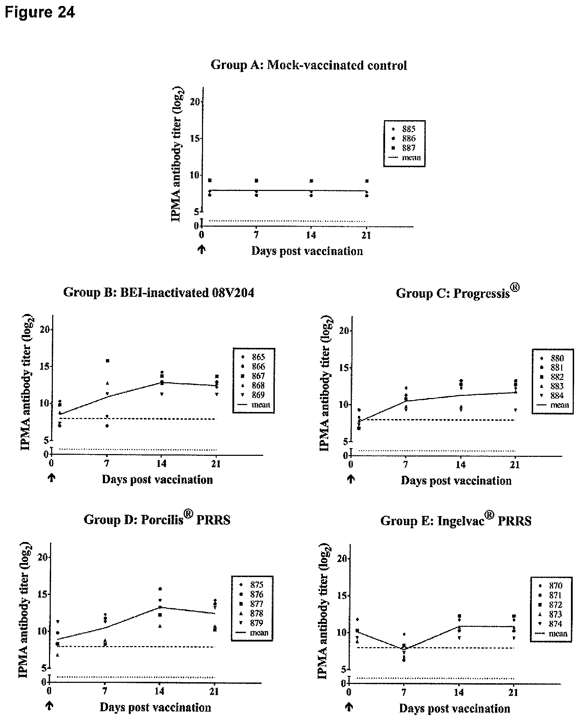

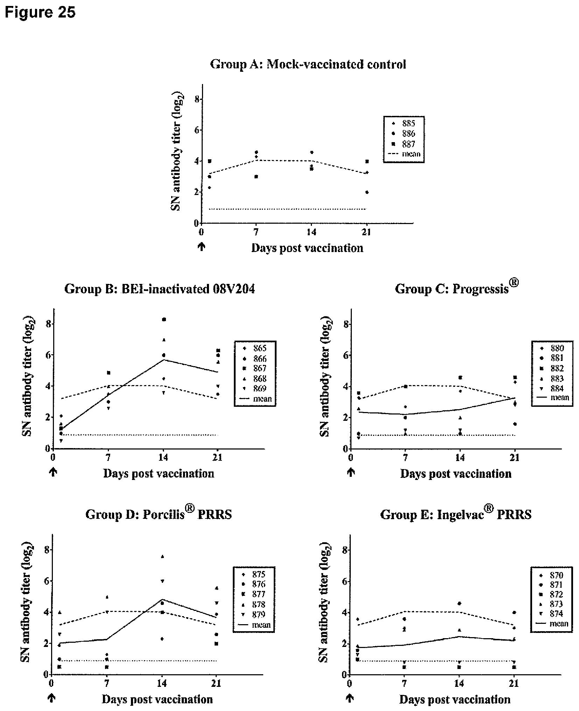

Using a 08V-204, naturally attenuated live vaccine, a good protection was obtained against a heterologous challenge by 08V-194. The results are summarized in figure 19: The protection as measured by the virus titer is better than the protection obtained with a commercial vaccine (Porcilis).

### Example 9: In vitro evaluation of cross-protection induced by inactivated PRRSV 07V-063 compared to inactivated PRRSV LV

To compare the cross-protection induced by PRRSV 07V-063 compared to PRRSV LV, serum from pigs vaccinated twice and challenged (homologous) with the respective viruses was used for an in vitro virus neutralization test.

Virus inactivation, vaccination and challenge were done as described in example 2.

Detection of neutralizing antibodies was done using different PRRSV isolates as target for the detection of neutralizing antibodies, both with the virus used for vaccination as with other, heterologous viruses.

Serum from pigs vaccinated with the BEI-inactivated 07V-063 clearly had neutralizing antibodies against all PRRSV isolated tested, both against the strain 07V-063 (virus used for vaccination) as against other, heterologous viruses. In contrast, vaccination with LV only induced clear levels of neutralizing antibodies against itself, and only low levels of neutralizing antibodies were detected against other viruses. Viruses used for the neutralization assays are EU (type I) strains, LENA W8 is a East-European subtype 3 strain.

| | Obtained SN titer read out after 7 days | | | | | |
|---|---|---|---|---|---|---|
| | results expressed in log₂ | | | | | |
| *PRRSV strain used for detection neutralizing antibodies* | **07V-063** | **08V-204** | **08V-120** | **08V-194** | **LENA W8** | **LV** |
| Serum 4 weeks after second vaccination with 07V-063 | 4,1 | 6,1 | 5,2 | 5,2 | 5,5 | 3,5 |
| Serum 4 weeks after second vaccination with LV | 3,9 | 5,4 | 4,7 | 5,1 | 5,4 | 3,9 |
| | | | | | | |
| two vaccinations with 07V-063 + challenge 07V-063 (serum 10 d post challenge) | 4,8 | 6,1 | 6,3 | 3,3 | 5 | 2,3 |
| two vaccinations with LV + challenge LV (serum 10 d post challenge) | 1,6 | 1,6 | 2 | 1,6 | 1,6 | 5,6 |

### Example 10: use of an inactivated vaccine for boosting the humoral immunity of sows, in comparison with commercial vaccines

### Experimental set up

The isolates 07V063, 08V194 and 08V204 used in this study originated from unrelated farms showing clinical signs compatible with PRRS in sows or growing pigs. At the moment of sampling, sows of the three herds were vaccinated with the EU-genotype attenuated vaccine (Porcilis® PRRS). From each farm-specific strain (07V063, 08V194 and 08V204) inactivated vaccines were made based on the method described by Delrue et al. (2009). Twenty-five sows, from each PRRSV-positive farm were included in the experiment and transported to the animal facilities of the Laboratory of Virology at the University Ghent. The experimental design was as follows: a first group (*n* = 5 sows) was a mock-vaccinated control group which received 1 mL RPMI in 1 mL o/w Suvaxyn. Group 2 (*n* = 5 sows) was vaccinated with 1 mL BEI-inactivated Marc-grown farm-specific virus (10⁸ TCID₅₀) in 1 mL o/w Suvaxyn. Group 3 (*n* = 5 sows) received 2 mL of a commercial European type inactivated PRRSV vaccine (Progressis®, Merial, strain P120: min 2,5 log IF Units). Group 4 and 5 were vaccinated with the European type attenuated vaccine (Porcilis® PRRS, Intervet, 10⁴ TCID₅₀/2ml) and the American type attenuated vaccine (Ingelvac® PRRS MLV, Boehringer Ingelheim, 10^{4,9} TCID₅₀/2ml), respectively. All vaccinations in all groups were administered once (single shot) one week after arrival. All sows were monitored clinically. Blood was taken at 0, 1, 2 and 3 weeks after vaccination for determination of both IPMA and virus neutralizing antibodies. Antibodies were detected against the PRRSV strain, originating from the farm where the sows came from. Serum samples collected after vaccination were examined for vaccine virus by titration in Marc-145 cells and pulmonary alveolar macrophages.

### Results

All sows remained in good health and condition after they were vaccinated and no local or general reactions were observed. No PRRSV was isolated from any of the samples. In all three set-ups, the strain-specific inactivated vaccine, was the only vaccine that induced both a virus-specific and a strong neutralizing antibody response after booster vaccination, compared to the commercial vaccines, both inactivated and attenuated (European and American type). The efficacy of the commercially attenuated and inactivated PRRS vaccines was dependent on the circulating virus isolate.

### Safe deposit

The following safe deposit has been made on 8 January 2010 with the Belgian Coordinated Collections of Microorganisms (BCCM™):
PK-15^{Sn-CD163}: accession number LMBP7117CB, SD number: SD10-01

### Deposits under the Budapest treaty:

The following deposits under the Budapest treaty have been made on 1 April 2010 with the Belgian Coordinated Collections of Microorganisms (BCCM™):
07V063: accession number LMBP 6660 (IDA10-04)
08V194: accession number LMBP 6661 (IDA10-05)
08V204: accession number LMBP 6662 (IDA10-06)

### REFERENCES

- Allende R, Lewis TL, Lu Z, Rock DL, Kutisch GF, Ali A, Doster AR, Osorio FA: North American and European porcine and respiratory syndrome viruses differ in non-structural protein coding regions. J Gen Virol 1999, 80(Pt2): 307-315.
- Cavanagh D: Nidovirales: a new order comprising Coronaviridae and Arteriviridae. Archives of virology 1997, 142(3):629-633.
- Choo JG, Dee SA: Porcine reproductive and respiratory syndrome virus. Terigenology 2006, 122: 175-182.
- Christianson WT, Collins JE, Benfield DA, Harris L, Gorcyca DE, Chladek DW, Morrison RB, Joo HS: Experimental reproduction of swine infertility and respiratory syndrome in pregnant sows. American journal of veterinary research 1992, 53(4):485-488.
- Christianson WT, Choi CS, Collins JE, Molitor TW, Morrison RB, Joo HS: Pathogenesis of porcine reproductive and respiratory syndrome virus infection in mid-gestation sows and fetuses. Canadian journal of veterinary research = Revue canadienne de recherche veterinaire 1993, 57(4):262-268.
- Delrue I, Delputte PL, Nauwynck H: Assessing the functionality of viral entry-associated domains of porcine reproductive and respiratory syndrome virus during inactivation procedures, a potential tool to optimize inactivated vaccines. Veterinary Research 2009, 62
- Diaz I, Darwich L, Pappaterra G, Pujols J, Mateu E: Different European-type vaccines against poricine reproductive and respiratory syndrome virus have different immunological properties and confer different protection to pigs. Virology 2006, 351, 249-259.
- Lager KM, Mengeling WL: Pathogenesis of in utero infection in porcine fetuses with porcine reproductive and respiratory syndrome virus. Canadian journal of veterinary research = Revue canadienne de recherche veterinaire 1995, 59(3):187-192.
- Mardassi H, Mounir S, Dea S: Molecular analysis of the ORFs 3 to 7 of procine reproductive and respiratory syndrome virus, Quebec reference strain. Arch Virol 1995, 140(8):1405-1418.
- Morozov I, Mang XJ, P aul PS: Sequence analysis of open reading frames (ORFs) 2 to 4 of a US isolate of porcine reproductive and respiratory syndrome virus. Arch Virol 1995, 140(7): 1313-1319.
- Meng XJ: Heterogeneity of porcine reproductive and respiratory syndrome virus: implications for current vaccine efficacy and future vaccine development. Veterinary Microbiology, 2000, 74: 309-329.
- Mengeling WL, Lager KM, Vorwald AC: Temporal characterization of transplacental infection of porcine fetuses with porcine reproductive and respiratory syndrome virus. American journal of veterinary research 1994, 55(10):1391-1398.
- Neumann EJ, Kliebenstein JB, Johnson CD, Mabry JW, Bush EJ, Seitzinger AH, Green AL, Zimmerman JJ: Assessment of the economic impact of porcine reproductive and respiratory syndrome on swine production in the United States. Journal of the American Veterinary Medical Association 2005, 227(3):385-392.
- Ropp SL, Wees CEM, Fanf Y, Nelson EA, Rossow KD, Bien M, Arndt B, Preszler S, Steen P, Christopher-Hennings J, Collins JE, Benfield DA, Faaberg KS: Characterization of emerging European-like porcine reproductive and respiratory syndrome virus isolates in the united states. Journal of Virology 2004, 78(7):3684-3703.
- Rossow KD, Bautista EM, Goyal SM, Molitor TW, Murtaugh MP, Morrison RB, Benfield DA, Collins JE: Experimental porcine reproductive and respiratory syndrome virus infection in one-, four-, and 10-week-old pigs. J Vet Diagn Invest 1994, 6(1):3-12.
- Snijder EJ, Meulenberg JJ: The molecular biology of arteriviruses. The Journal of general virology 1998, 79(Pt 5):961-979.
- Terpstra C, Wensvoort G, Pol JM: Experimental reproduction of porcine epidemic abortion and respiratory syndrome (mystery swine disease) by infection with Lelystad virus: Koch's postulates fulfilled. The Veterinary quarterly 1991, 13(3):131-136.
- Zhang Z, Schwartz S, Wagner L, Miller W: A greedy algorithm for aligning DNA sequences. J Comput Biol 2000; 7(1-2):203-14.

## Claims

1. A vaccine against PRRSV, said vaccine comprising an inactivated or attenuated whole type 1 PRRSV strain wherein the genome of said type 1 PRRSV strain comprises SEQ ID N°1, SEQ ID N°12, or SEQ ID N°13, or a sequence having at least 95% sequence identity with SEQ ID N° 1; and wherein said vaccine is capable of inducing an antibody response against said PRRSV strain.

2. The vaccine against PRRSV according to claim 1, said vaccine comprising a type 1 strain comprising SEQ ID N°1.

3. The vaccine against PRRSV according to claims 1 or 2, said vaccine comprising strain 07V-063 as deposited with the BCCM™ with accession number LMBP 6660.

4. The vaccine against PRRSV according to claim 1, said vaccine comprising strain 08V-204 as deposited with the BCCM™ with accession number LMBP 6662.

5. The vaccine against PRRSV according to claim 1, said vaccine comprising strain 08V-194 as deposited with the BCCM™ with accession number LMBP 6661.

6. The vaccine against PRRSV according to any one of claims 1-5, wherein the PRRSV strain was grown in Marc cells or PK-15 cells.

7. The vaccine against PRRSV according to any one of claims 1-6, wherein the PRRSV strain is inactivated by formaldehyde treatment, glutaraldehyde treatment, AT-2 treatment, pH and/or temperature denaturation, gamma irradiation, UV irradiation or binary ethyleneimine (BEI) treatment.

8. The vaccine against PRRSV according to any one of claims 1-7, further comprising a pharmacologically acceptable carrier or diluent.

9. The vaccine according to claim 8, suitable for oral, intranasal, subcutaneous or intramuscular administration.

10. A whole type 1 PRRSV strain wherein the genome of said type 1 PRRSV strain comprises SEQ ID N°1, SEQ ID N°12, SEQ ID N°13, or sequence having at least 95% sequence identity with SEQ ID N° 1; and wherein said strain is capable of inducing an antibody response against said PRRSV strain.

11. A whole type 1 PRRSV strain according to claim 10 for use as a medicament.

12. A whole type 1 PRRSV strain according to claim 10, for use in prevention or treatment of PRRSV infection in pigs.

## Patentansprüche

1. Ein Impfstoff gegen PRRSV, wobei der besagte Impfstoff einen inaktivierten oder abgeschwächten Typ 1 PRRSV-Ganzerreger-Stamm umfasst, in dem das Genom des besagten Typ 1 PRRSV Erregerstamms SEQ ID Nr. 1, SEQ ID Nr. 12, oder SEQ ID Nr. 13 oder eine Sequenz umfasst, die mindestens 95 % Sequenz-Identität mit SEQ ID Nr. 1 aufweist und bei der der besagte Impfstoff in der Lage ist, eine Antikörperreaktion gegen den besagten PRRSV-Stamm zu induzieren.

2. Der Impfstoff gegen PRRSV nach Anspruch 1, wobei der besagte Impfstoff einen Erregerstamm vom Typ 1 umfasst, der SEQ ID Nr. 1 umfasst.

3. Der Impfstoff gegen PRRSV nach Anspruch 1 oder 2, wobei der besagte Impfstoff den Stamm 07V-063 umfasst, wie beim BCCM™ mit der Zugangsnummer LMBP 6660 angemeldet.

4. Der Impfstoff gegen PRRSV nach Anspruch 1, wobei der besagte Impfstoff den Stamm 08V-204 umfasst, wie beim BCCM™ mit der Zugangsnummer LMBP 6662 angemeldet.

5. Der Impfstoff gegen PRRSV nach Anspruch 1, wobei der besagte Impfstoff den Stamm 08V-194 umfasst, wie beim BCCM™ mit der Zugangsnummer LMBP 6661 angemeldet.

6. Der Impfstoff gegen PRRSV nach einem der Ansprüche 1-5, wobei der PRRSV-Stamm in Marc-Zellen oder PK-15-Zellen gezüchtet wurde.

7. Der Impfstoff gegen PRRSV nach einem der Ansprüche 1-6, wobei der PRRSV-Stamm durch Formaldehyd-Behandlung, Glutaraldehyd-Behandlung, AT-2-Behandlung, pH- und/oder Temperaturdenaturierung, Gamma-Bestrahlung, UV-Bestrahlung oder binäre Ethylenimin-(BEI)-Behandlung inaktiviert wird.

8. Der Impfstoff gegen PRRSV nach einem der Ansprüche 1-7, der weiter einen pharmakologisch akzeptablen Träger oder ein Verdünnungsmittel umfasst.

9. Der Impfstoff nach Anspruch 8, der für orale, intranasale, subkutane oder intramuskuläre Verabreichung geeignet ist.

10. Ein Typ 1 PRRSV-Ganzerreger-Stamm, in dem das Genom des besagten Typ 1 PRRSV-Stamms SEQ ID Nr. 1, SEQ ID Nr. 12, SEQ ID Nr. 13, oder Sequenzen umfasst, die mindestens 95 % Sequenz-Identität mit SEQ ID Nr. 1 aufweisen; und in dem der besagte Stamm in der Lage ist, eine Antikörper-Reaktion gegen den besagten PRRSV-Stamm zu induzieren.

11. Ein Typ 1 PRRSV-Ganzerreger-Stamm nach Anspruch 10 zur Verwendung als Medikament.

12. Ein Typ 1 PRRSV Ganzerreger-Stamm nach Anspruch 10 zur Verwendung bei der Vorbeugung oder Behandlung von PRRSV-Infektion bei Schweinen.

## Revendications

1. Vaccin contre le virus du SDRP, ledit vaccin comprenant une souche entière inactivée ou atténuée du virus du SDRP de type 1, dans lequel le génome de ladite souche du virus du SDRP de type 1 comprend la SEQ ID N°1, la SEQ ID N°12 ou la SEQ ID N°13, ou une séquence ayant au moins 95 % de l'identité de séquence de la SEQ ID N°1 ; et dans lequel ledit vaccin est capable d'induire une réponse des anticorps contre le virus du SDRP.

2. Le vaccin contre le virus du SDRP selon la revendication 1, lequel vaccin comprenant une souche de type 1 comprenant la SEQ ID N°1.

3. Le vaccin contre le virus du SDRP selon les revendications 1 ou 2, ledit vaccin comprenant la souche 07V-063 telle que déposée auprès du BCCM™ sous le numéro d'accès LMBP 6660.

4. Le vaccin contre le virus du SDRP selon la revendication 1, ledit vaccin comprenant la souche 08V-204 telle que déposée auprès du BCCM™ sous le numéro d'accès LMBP 6662.

5. Le vaccin contre le virus du SDRP selon la revendication 1, ledit vaccin comprenant la souche 08V-194 telle que déposée auprès du BCCMTM sous le numéro d'accès LMBP 6661.

6. Le vaccin contre le virus du SDRP selon l'une quelconque des revendications 1 à 5, dans lequel la souche du virus du SDRP a été cultivée dans des cellules Marc ou des cellules PK-15.

7. Le vaccin contre le virus du SDRP selon l'une quelconque des revendications 1 à 6, dans lequel la souche du virus du SDRP est inactivée par traitement au formaldéhyde, traitement au glutaraldéhyde, traitement à l'AT-2, dénaturation par le pH et/ou par la chaleur, irradiation aux rayons gamma, irradiation aux UV ou traitement par l'éthylèneimine binaire (BEI).

8. Le vaccin contre le virus du SDRP selon l'une quelconque des revendications 1 à 7, comprenant en plus un vecteur ou un diluant pharmacologiquement acceptable.

9. Le vaccin selon la revendication 8, approprié pour une administration par voie orale, intranasale, sous-cutanée ou intramusculaire.

10. Une souche entière du virus du SDRP de type 1 dans laquelle le génome de ladite souche du virus du SDRP de type 1 comprend la SEQ ID N°1, la SEQ ID N°12, la SEQ ID N°13 ou une séquence ayant au moins 95 % de l'identité de séquence de la SEQ ID N°1 ; et dans lequel ladite souche est capable d'induire une réponse d'anticorps contre ladite souche du virus du SDRP.

11. Une souche entière du virus SDRP de type 1 selon la revendication 10, destinée à être utilisée en tant que médicament.

12. Une souche entière du virus SDRP de type 1 selon la revendication 10, destinée à être utilisée dans la prévention ou le traitement de l'infection par le virus SDRP chez les porcs.
